# EUROPEAN PATENT APPLICATION

(11) **EP 4 653 018 A1**
(43) Date of publication of application: **26.11.2025**
(21) Application number: 24744323.7
(22) Date of filing: 17.01.2024
(51) Int. Cl.: A61K 47/68, C07K 16/00, A61K 39/00, A61P 35/00

(54) **ANTIBODY DRUG CONJUGATE AS WELL AS PREPARATION METHOD THEREFOR AND USE THEREOF**

(30) Priority: 18.01.2023 CN 202310060263; 23.10.2023 CN 202311377099; 03.01.2024 CN 202410008387
(71) Applicant: Medilink Therapeutics (Suzhou) Co., Ltd., Suzhou, Jiangsu 215000 (CN)
(72) Inventor: SONG, Shuai, Suzhou, Jiangsu 215000 (CN); CAI, Jiaqiang, Suzhou, Jiangsu 215000 (CN); XUE, Tongtong, Suzhou, Jiangsu 215000 (CN); XIAO, Liang, Suzhou, Jiangsu 215000 (CN)
(74) Representative: dompatent
(86) International application number: PCT/CN2024/072859
(87) International publication number: WO 2024/153149

(57) **Abstract**

The present disclosure relates to an antibody drug conjugate containing two or more bioactive molecules having different action mechanisms, the antibody drug conjugate being shown as formula (I), and relates to a preparation method therefor and the use thereof in prevention and/or treatment of diseases related to abnormal cell activities, including but not limited to the use in prevention and/or treatment of tumor diseases.

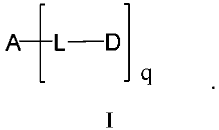

## Description

The present application claims priority to Chinese Patent Application No. 2023100602630 filed on January 18, 2023, Chinese Patent Application No. 2023113770992 filed on October 23, 2023, and Chinese Patent Application No. 2024100083879 filed on January 3, 2024, which are incorporated herein by reference in their entirety.

### TECHNICAL FIELD

The present disclosure belongs to the field of pharmaceutical technology and relates to an antibody-drug conjugate comprising two or more bioactive molecules with different mechanisms of action, a preparation method therefor, and use thereof in the prevention and/or treatment of diseases associated with abnormal cell activity, including but not limited to use thereof in the prevention and/or treatment of tumor diseases.

### BACKGROUND

Antibody-drug conjugates (ADCs) are a class of biomacromolecular drugs formed by attaching bioactive molecules (drugs) to antibodies via chemical linkers. ADCs combine the targeting effects of antibodies with the activity of bioactive molecules, functioning as "biological missiles" with promising efficacy and safety advantages.

In recent years, ADC drug development has progressed rapidly, with 15 drugs already approved for sale. Structurally, 13 of the ADCs use small-molecule chemical toxins as drugs (the bioactive molecules attached). From a mechanism-of-action perspective, 8 of the 13 ADCs use tubulin inhibitors as drugs, 3 use DNA damaging/binding agents as drugs, and 2 use topoisomerase inhibitors as drugs. The approved ADC drugs have demonstrated strong therapeutic efficacy against different indications.

However, all the currently approved ADC molecules use single bioactive molecules as drugs. Although they exhibit certain therapeutic efficacy, they still have limitations. For example, the tubulin inhibitor MMAE exerts its antitumor effect through the following mechanism of action: it inhibits tubulin polymerization or promotes tubulin polymerization to interfere with the mitosis of cells-it disrupts the mitosis of cells and arrests the cell cycle at the M phase, thereby causing rapidly dividing tumor cells to undergo apoptosis. Therefore, from a mechanism-of-action perspective, ADCs using MMAE as a drug have relatively weak effects on tumor cells that are in the resting phase. In addition, prolonged use of MMAE-based ADCs predisposes patients to develop drug resistance, primarily due to three factors: 1. drug efflux, changes in the microtubular structure, and abnormal apoptotic signaling pathways; 2. transport by ATP-binding cassette drug transporter proteins, P-glycoprotein, multidrug resistance protein 2 (M^{RP}2), etc.; and 3. lack of extracellular matrix protein transforming growth factor β1 (TGF-β1), overexpression of β-III tubulin subunits, and elevations in MCL1 protein expression levels.

From a biological mechanism perspective, simultaneously attaching bioactive molecules that target two or more targets to the same antibody to obtain a multi-target ADC molecule may result in a synergistic effect, thereby further improving the antitumor effect of the drug. For example, if the tubulin inhibitor MMAE (or a derivative thereof) and the topoisomerase inhibitor Dxd (or another camptothecin derivative) are conjugated to the same antibody to obtain a multi-targetADC molecule, the ADC simultaneously releases two bioactive molecules upon reaching tumor cells. As a result, the mitosis of the cells is disrupted, and the replication and transcription of DNA are inhibited. The two may have a synergistic effect. Therefore, the development of multi-target ADC molecules is necessary and of great significance.

At present, there have been patents that disclose double-toxin ADC molecules, and most of them use branched linker technology (in which a linker is split into two or even more linkers) to conjugate identical bioactive molecules. However, this only increases the drug antibody ratio (DAR) and cannot achieve synergy mechanistically. In a few patents (WO2022022649), two bioactive molecules with different mechanisms of action are conjugated in a ratio of 1:1. However, due to differences in their mechanisms of action, bioactivity, and pharmacokinetic profiles, the two bioactive molecules differ in both minimum effective and toxic doses. Where they are conjugated in a simple ratio of 1:1 to an antibody to form an ADC, it is difficult to ensure that the molecule is both effective and safe (for example, one toxin has reached the toxic dose before the other one reaches the effective dose; or when one toxin reaches the effective dose, the other one has far exceeded the maximum tolerated dose), and the druggability is relatively low. Further research and development is needed to determine how to control the ratio of two different bioactive molecules in an ADC to ensure that the ADC exhibits optimal therapeutic efficacy against tumor cells while being safe.

Known double-toxin ADCs all bind to tumor cell surface antigens through the antibodies in the ADCs. Then they enter endosomes through endocytosis and are trafficked from the endosomes to lysosomes, and the bioactive molecules (toxins or payloads) are dissociated from the ADCs by the action of enzymes, enter the cytoplasm, and then kill the tumor cells. In the whole process, the tumor killing efficiency is closely related to the expression levels of tumor cell surface antigens, the endocytosis efficiency, the functions of endosomes and lysosomes, the dissociation efficiency of the bioactive molecules, and the efficiency of entering the cytoplasm from lysosomes. Changes in any link can cause resistance to the ADCs, leading to the loss of therapeutic efficacy. Therefore, a double-toxin ADC that can remain stable in the blood circulation system in the body and can be extracellularly cleaved in the tumor microenvironment, releasing the toxins to kill tumors, without the need for endocytosis and dependence on the expression levels of tumor cell surface antigens will be able to overcome a number of mechanisms of resistance to conventional ADCs and thus will be of great significance in clinical treatment.

### SUMMARY

In order to resolve the above problems, the present invention provides an antibody-drug conjugate comprising two or more bioactive molecules with different mechanisms of action. An ADC with a well-defined administration dose is used as a starting material, and a proper number of toxin-linkers (drug linkers) for other targets are conjugated to the ADC via active sites in the molecule to form a new antibody-drug conjugate. In this way, the ratio of the two bioactive molecules with different mechanisms of action conjugated to the antibody can be flexibly regulated under the guidance of efficacy and safety data, and the druggability of the obtained new ADC is greatly increased.

For this purpose, in a first aspect of the present disclosure, the present disclosure provides an antibody-drug conjugate represented by formula (I), 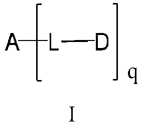
or a stereoisomer of the antibody-drug conjugate, a prodrug thereof, a pharmaceutically acceptable salt thereof, or a pharmaceutically acceptable solvate thereof,
wherein:
   A is a known ADC;
   D is a bioactive molecular fragment that targets a different target compared to a bioactive molecule in A;
   L is a linker that covalently binds to A and D;
   q is selected from any integer between 1 and 20.

In some embodiments, L is attached to A via a sulfur (S) atom, and L is attached to a sulfhydryl group contained in A to form -S-; preferably, L is attached to a single sulfhydryl group of a cysteine (Cys) residue contained in A to form -S-.

In some embodiments, L is attached to A via a nitrogen (N) atom, and L is attached to a lysine (Lys) residue contained in A to form -N-.

In some embodiments, provided is an antibody-drug conjugate represented by formula (II), 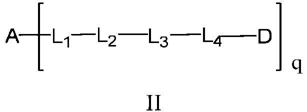
or a stereoisomer of the antibody-drug conjugate, a prodrug thereof, a pharmaceutically acceptable salt thereof, or a pharmaceutically acceptable solvate thereof,
wherein:
   A is a known ADC;
   D is a bioactive molecular fragment that targets a different target compared to a bioactive molecule in A;
   L₁ is a linker unit that covalently binds to A and L₂;
   L₂ is a connector unit that covalently binds to L₁ and L₃;
   L₃ is an amino acid residue or a short peptide consisting of 2-10 amino acid residues; the amino acid residues are selected from natural amino acid residues, non-natural amino acid residues, and amino acid residues represented by AA¹ or stereoisomers thereof;
   the amino acid residues represented by AA¹ have a structure as shown below: 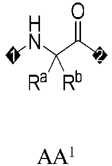
   wherein:
      R^{a} and R^{b} are each independently selected from hydrogen and 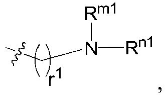 and R^{a} and R^{b} are not simultaneously hydrogen;
      or R^{a} and R^{b}, together with the carbon atom to which they are attached, form a 4-10 membered heterocyclic ring or 4-10 membered carbocyclic ring, wherein the 4-10 membered heterocyclic ring or 4-10 membered carbocyclic ring is optionally substituted with one or more R⁰;
      r¹ is selected from any integer between 0 and 20;
      R^{m1} and Rⁿ¹ are each independently selected from hydrogen, C1-6 alkyl, and C3-6 cycloalkyl;
      or R^{m1} and Rⁿ¹, together with the nitrogen atom to which they are attached, form a 4-10 membered heterocyclic ring, wherein the 4-10 membered heterocyclic ring is optionally substituted with one or more R^{0'};
      R⁰ and R^{0'} are each independently selected from C1-6 alkyl, C3-6 cycloalkyl, -NR^{m2}Rⁿ², and 4-10 membered heterocyclyl, wherein the 4-10 membered heterocyclyl is optionally substituted with C1-6 alkyl;
      R^{m2} and Rⁿ² are each independently selected from hydrogen and C1-6 alkyl;
      L₄ is a direct bond or spacer unit that covalently binds to L₃ and D;
      q is selected from any integer between 1 and 20.

In some embodiments, when being an amino acid residue represented by AA¹, L₃ may be attached to groups at both ends (e.g., L₂ and L₄) in any attachment direction.

In some embodiments, q is selected from any integer between 1 and 12.

In some embodiments, q is selected from any integer between 1 and 10.

In some embodiments, q is selected from 1, 2, 3, 4, 5, 6, 7, 8, 9, and 10.

In some embodiments, q is selected from any integer between 2 and 8.

In some embodiments, q is selected from 2, 4, 6, and 8.

In some embodiments, the linkers of the antibody-drug conjugate of the present invention enable the antibody-drug conjugate to remain stable in the blood circulation system in the body and to be cleaved in the tumor microenvironment, releasing the toxins to kill tumor cells.

In some embodiments, A is selected from an ADC that targets one or more of the following targets: B7H3, CD20, CD19, CD30, GPNMB, Her2, Trop-2, EGFR, GD-2, CD79b, FRα, NAPI2B, BCMA, etc.

In some embodiments, A comprises an antibody or an antigen-binding fragment thereof, Tb.

In some embodiments, the antibody or the antigen-binding fragment thereof and a monoclonal antibody or an antigen-binding fragment thereof comprise: a Fab, a Fab', a F(ab')2, an Fd, an Fv (e.g., scFv), a dAb, a complementarity determining region fragment, a non-human antibody, a humanized antibody, a chimeric antibody, a fully human antibody, a probody, a monoclonal antibody, a bispecific antibody, or a multispecific antibody.

In some embodiments, Tb is an antibody or an antigen-binding fragment thereof with or without endocytic activity.

In some embodiments, Tb is an antibody or an antigen-binding fragment thereof with endocytic activity.

In some embodiments, Tb is an antibody or an antigen-binding fragment thereof with activity of binding to a free antigen in a tumor tissue and/or a tumor cell surface antigen.

In some embodiments, Tb is an antibody or an antigen-binding fragment thereof with activity of being endocytosed by a tumor cell and with activity of binding to a free antigen in a tumor tissue or a tumor cell surface antigen.

In some embodiments, Tb is an antibody or an antigen-binding fragment thereof with activity of being endocytosed by a tumor cell and with activity of binding to a free antigen in a tumor tissue and a tumor cell surface antigen.

In some embodiments, Tb is an antibody or an antigen-binding fragment thereof with activity of being endocytosed by a tumor cell and without activity of binding to a free antigen in a tumor tissue.

In some embodiments, Tb is an antibody or an antigen-binding fragment thereof with activity of being endocytosed by a tumor cell and without activity of binding to a tumor cell surface antigen.

In some embodiments, Tb is an antibody or an antigen-binding fragment thereof without endocytic activity or with weak endocytic activity.

In some embodiments, Tb is an antibody or an antigen-binding fragment thereof without activity of binding to a free antigen in a tumor tissue and/or a tumor cell surface antigen.

In some embodiments, Tb is an antibody or an antigen-binding fragment thereof without activity of being endocytosed by a tumor cell or with weak endocytic activity and with activity of binding to a free antigen in a tumor tissue or a tumor cell surface antigen.

In some embodiments, Tb is an antibody or an antigen-binding fragment thereof without activity of being endocytosed by a tumor cell or with weak endocytic activity and with activity of binding to a free antigen in a tumor tissue and a tumor cell surface antigen.

In some embodiments, Tb is an antibody or an antigen-binding fragment thereof without activity of being endocytosed by a tumor cell or with weak endocytic activity and without activity of binding to a tumor cell surface antigen.

In some embodiments, Tb is an antibody or an antigen-binding fragment thereof without activity of being endocytosed by a tumor cell or with weak endocytic activity and without activity of binding to a free antigen in a tumor tissue.

In some embodiments, Tb is an antibody or an antigen-binding fragment thereof without activity of being endocytosed by a tumor cell and having no corresponding antigen in the human body.

In some embodiments, Tb is an antibody having no binding to a tumor cell-associated antigen.

In some embodiments, the antibody or the antigen-binding fragment thereof is selected from an IgG isotype antibody.

In some embodiments, the antibody or the antigen-binding fragment thereof is selected from isotype IgG1, isotype IgG2, isotype IgG3, and isotype IgG4.

In some embodiments, the antibody or the antigen-binding fragment thereof is an anti-chicken lysozyme human IgG1 isotype antibody.

In some embodiments, Tb is an antibody having binding to a tumor cell-associated antigen.

In some embodiments, Tb is an antibody or an antigen-binding fragment thereof with activity of binding to a free antigen in a tumor tissue or a tumor cell surface antigen.

In some embodiments, Tb is an antibody or an antigen-binding fragment thereof with activity of binding to a free antigen in a tumor tissue and a tumor cell surface antigen.

In some embodiments, Tb is an antibody or an antigen-binding fragment thereof with B7H3-2Ig and/or B7H-4Ig.

In some embodiments, Tb is an antibody or an antigen-binding fragment thereof with higher binding activity to B7H3-4Ig than to B7H3-2Ig.

In some embodiments of the present disclosure, Tb is a ligand or targeting moiety that binds to a target.

In some embodiments, the target of Tb is selected from a target with higher expression in tumor cells than in normal cells.

In some embodiments, the target of Tb is selected from a target that is highly expressed in tumor cells and lowly expressed in normal cells.

In some embodiments, the target of Tb is selected from: B7H3, CD20, CD19, CD30, GPNMB, Her2, Trop-2, EGFR, GD-2, CD79b, FRα, NAPI2B, BCMA, etc.

In some embodiments, Tb is an antibody or an antigen-binding fragment thereof.

In some embodiments, Tb is a non-human antibody, a humanized antibody, a chimeric antibody, or a fully human antibody.

In some embodiments, Tb is a monoclonal antibody, a bispecific antibody, or a multispecific antibody.

In some embodiments, Tb is a monoclonal antibody or an antigen-binding fragment thereof.

In some embodiments, Tb is an anti-B7H3 antibody or an antigen-binding fragment thereof, an anti-Trop-2 antibody or an antigen-binding fragment thereof, an anti-Her 2 antibody or an antigen-binding fragment thereof, an anti-FRα antibody or an antigen-binding fragment thereof, or an anti-NAPI2B antibody or an antigen-binding fragment thereof.

In some embodiments, Tb is an anti-B7H3 monoclonal antibody or an antigen-binding fragment thereof.

In some embodiments, Tb is an anti-B7H3 antibody or an antigen-binding fragment thereof, such as enoblituzumab, mirzotamab, omburtamab, the 1D1, 1D1-01, 2E3, or 2E3-02 antibody described in WO2022170971, or an antigen-binding fragment thereof.

In some embodiments, Tb is an anti-Trop-2 antibody or an antigen-binding fragment thereof, such as datopotamab, sacituzumab, or an antigen-binding fragment thereof.

In some embodiments, Tb is an anti-Trop-2 monoclonal antibody or an antigen-binding fragment thereof.

In some embodiments, Tb is an anti-Her 2 antibody or an antigen-binding fragment thereof, such as anbenitamab, coprelotamab, disitamab, gancotamab, margetuximab, pertuzumab, timigutuzumab, zanidatamab, trastuzumab, pertuzumab, or an antigen-binding fragment thereof.

In some embodiments, Tb is an anti-Her 2 monoclonal antibody or an antigen-binding fragment thereof, such as trastuzumab, pertuzumab, or an antigen-binding fragment thereof.

In some embodiments, A is selected from a known ADC with a tubulin inhibitor as a bioactive molecule.

In some embodiments, A is selected from a known ADC with an auristatin compound as a bioactive molecule, a known ADC with a maytansinoid compound as a bioactive molecule, a known ADC with a tubulysin compound as a bioactive molecule, and a known ADC with an eribulin compound as a bioactive molecule.

In some embodiments, A is selected from a known ADC with MMAE and a derivative thereof as bioactive molecules, a known ADC with MMAF and a derivative thereof as bioactive molecules, and a known ADC with a maytansinoid compound (e.g., DM1, DM4, etc.) as a bioactive molecule.

In some embodiments, A is selected from ARX788, ARX517, FS-1502, AGS62P1, A166, ADC2122, mirvetuximab soravtansine, and trastuzumab emtansine.

In some embodiments, A is selected from ARX788, A166, and ADC2122.

In some embodiments, A is selected from:

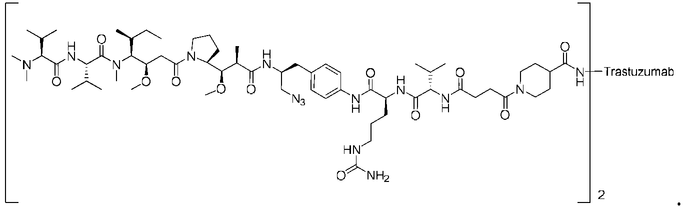

In some embodiments, D is a bioactive molecular fragment that targets a different target compared to a bioactive molecule in A, wherein the bioactive molecule is selected from, but is not limited to, glycopeptide antibiotics; glucocorticoid drugs; calcineurin inhibitors; DNA topoisomerase inhibitors, such as topoisomerase I inhibitors (e.g., camptothecin bioactive molecules) and topoisomerase II inhibitors; drugs that interfere with DNA synthesis; drugs that act on structural proteins; tumor signaling pathway inhibitors, such as kinase inhibitors; drugs that act on RNA; proteasome inhibitors; protease inhibitors; inhibitors of epigenetically related targets; tumor angiogenesis inhibitors; cyclin inhibitors; protein degradation agents, such as PROTACs and molecular glues; nucleic acid drugs; immunomodulators; steroidal saponins; and other active substances that inhibit tumor cell growth and promote tumor cell apoptosis or necrosis.

In some embodiments, D is a bioactive molecular fragment that targets a different target compared to a bioactive molecule in A, wherein the bioactive molecule is selected from DNA topoisomerase inhibitors, such as topoisomerase I inhibitors (e.g., camptothecin bioactive molecules) and topoisomerase II inhibitors; drugs that interfere with DNA synthesis; drugs that act on structural proteins; tumor signaling pathway inhibitors, such as kinase inhibitors; drugs that act on RNA; cyclin inhibitors; protein degradation agents, such as PROTACs and molecular glues; nucleic acid drugs; immunomodulators; and other active substances that inhibit tumor cell growth and promote tumor cell apoptosis or necrosis.

In some embodiments, D is a bioactive molecular fragment that targets a different target compared to a bioactive molecule in A, wherein the bioactive molecule is selected from topoisomerase I inhibitors, such as camptothecin bioactive molecules; drugs that interfere with DNA synthesis; drugs that act on RNA; protein degradation agents, such as PROTACs and molecular glues; and other active substances that inhibit tumor cell growth and promote tumor cell apoptosis or necrosis.

In some embodiments, D is a camptothecin bioactive molecular fragment, a tubulin inhibitor bioactive molecular fragment, or an eribulin bioactive molecular fragment.

In some embodiments, D is a camptothecin bioactive molecular fragment represented by formula (III) that is attached to L₄ via an oxygen atom, a sulfur atom, or a nitrogen atom contained therein, 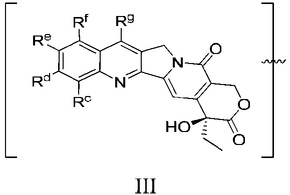 wherein:
R^{c}, R^{d}, R^{e}, R^{f}, and R^{g} are each independently selected from hydrogen, halogen, -OH, -NH₂, optionally substituted C1-6 alkyl, optionally substituted C1-6 alkoxy, and 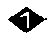 -X-W-H, wherein position 1 is attached to a parent ring;
or R^{d} and R^{e}, together with the carbon atoms to which they are attached, form a 5-7 membered carbocyclic ring or 5-7 membered heterocyclic ring, wherein the heterocyclic ring comprises one or more O, S, N, carbonyl, sulfoxide groups, or sulfonyl, or any combination thereof;
or R^{e} and R^{f}, together with the carbon atoms to which they are attached, form a 5-7 membered carbocyclic ring or 5-7 membered heterocyclic ring, wherein the heterocyclic ring comprises one or more O, S, N, carbonyl, sulfoxide groups, or sulfonyl, or any combination thereof;
or R^{f} and R^{g}, together with the carbon atoms to which they are attached, form a 5-7 membered carbocyclic ring or 5-7 membered heterocyclic ring, wherein the heterocyclic ring comprises one or more O, S, N, carbonyl, sulfoxide groups, or sulfonyl, or any combination thereof;
the carbocyclic ring and heterocyclic ring may be substituted with one or more deuterium, halogens, C1-6 alkyl, or 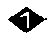 -X-W-H, wherein position 1 is attached to the carbocyclic ring or heterocyclic ring;
X is selected from a direct bond, optionally substituted -(CH₂)ₙ₁-, -O-(CH₂)ₙ₁-, -N(R₁)-(CH₂)ₙ₁-, -S-(CH₂)ₙ₁-, - C(O)-(CH₂)ₙ₁, -SO₂-(CH₂)ₙ₁-, 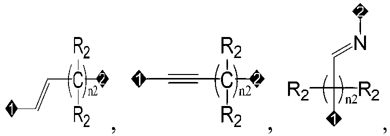 C3-6 cycloalkyl, C6-10 aryl, 5-10 membered heteroaryl, and 4-10 membered heterocyclyl, wherein position 1 is attached to a parent ring, a carbocyclic ring, or a heterocyclic ring, and position 2 is attached to W or H; the substituent is selected from one or more C1-4 alkyl and C3-6 cycloalkyl, or more than one C1-4 alkyl, together with the carbon atom to which they are simultaneously attached, forms C3-6 cycloalkyl;
R₁ and R₂, at each occurrence, are each independently selected from hydrogen, optionally substituted C1-4 alkyl, optionally substituted C1-4 alkoxy, and optionally substituted C3-6 cycloalkyl;
n1 and n2 are each independently selected from any integer between 0 and 6;
W is absent or present, and when W is present, W is selected from -O-, -S-, -NR₃-, 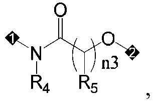 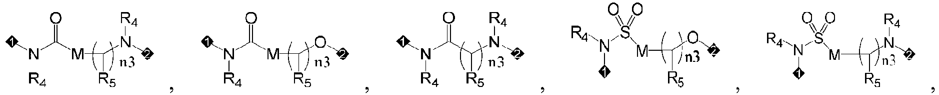 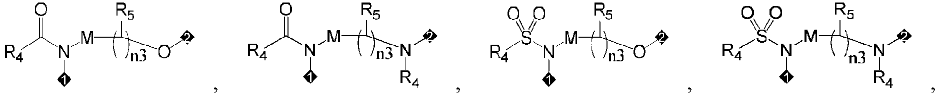 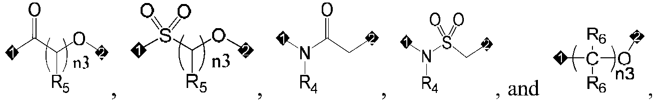 wherein position 1 is attached to X, and position 2 is attached to H;
each M is independently selected from a direct bond, -CR₇R₈-, a 3-7 membered carbocyclic ring, and a 3-7 membered heterocyclic ring;
R₃, R₄, R₅, R₆, R₇, and R₈, at each occurrence, are each independently selected from hydrogen, optionally substituted C1-4 alkyl, optionally substituted C1-4 alkenyl, optionally substituted C1-4 alkynyl, optionally substituted C1-4 alkoxy, and optionally substituted C3-6 cycloalkyl;
n3 is selected from any integer between 0 and 6.

In some embodiments, D is selected from the following structures:

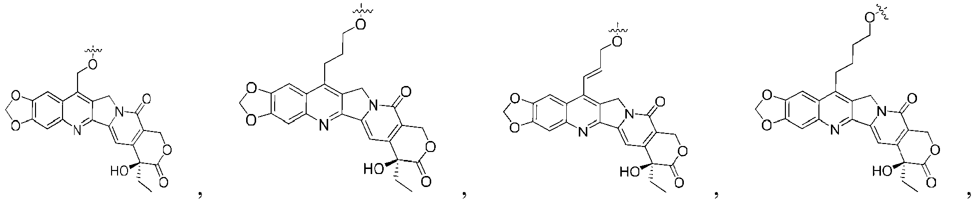

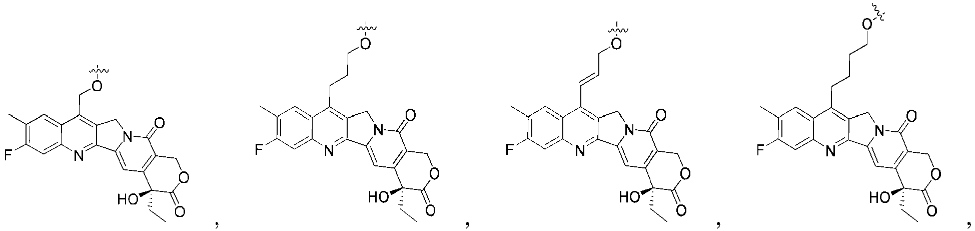

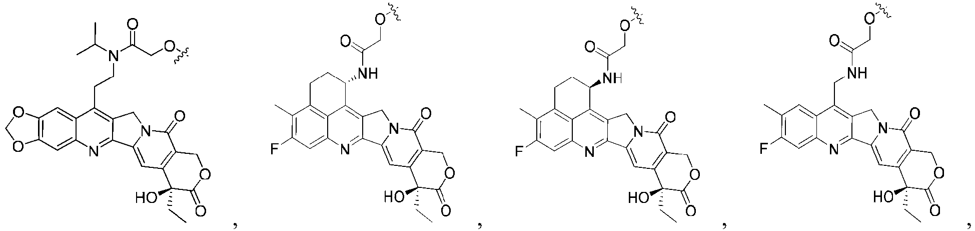

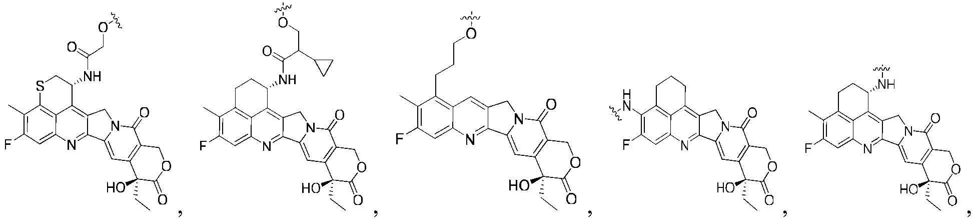

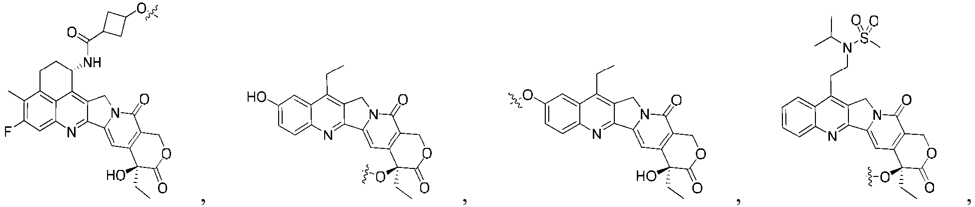

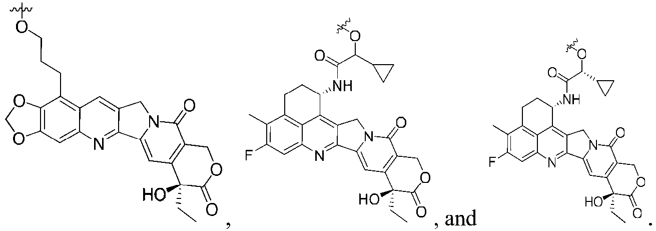

In some embodiments, A is selected from a known ADC with camptothecin as a bioactive molecule, and D is selected from a tubulin inhibitor bioactive molecular fragment; for example, D is MMAE and a derivative thereof, or MMAF and a derivative thereof.

In some embodiments, A is selected from: trastuzumab deruxtecan, SHR-A1811, and

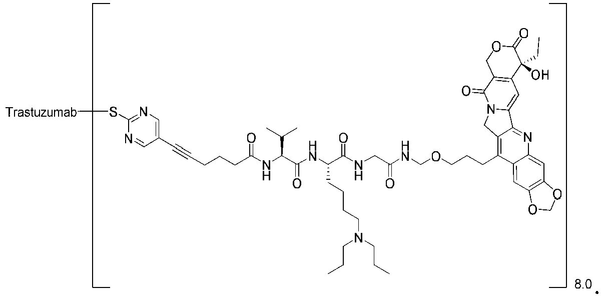

In some embodiments, D is selected from: MMAE and MMAF.

**In** some embodiments, D is selected from the following structures:

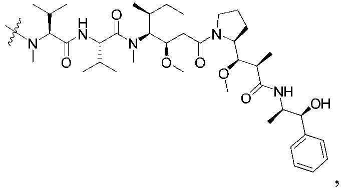

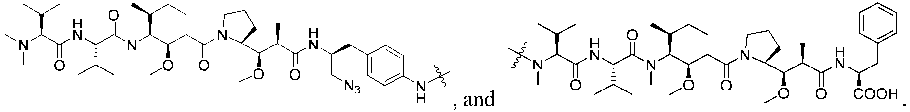

In some embodiments, A is selected from a known ADC with a camptothecin as a bioactive molecule, and D is selected from an eribulin bioactive molecular fragment.

In some embodiments, D is selected from: eribulin.

In some embodiments, D is selected from the following structures:

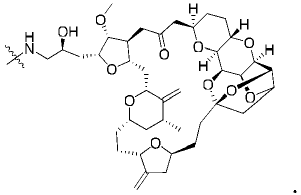

In some embodiments, L₁ is selected from heteroaryl, partially saturated heteroaryl, heteroarylbiaryl, heteroarylbiheteroaryl, heterocyclyl, oxoheterocyclyl, maleimide, cycloalkenyl, alkylamido, cycloalkylcarbonyl, heterocycloalkylcarbonyl, alkenylamido, 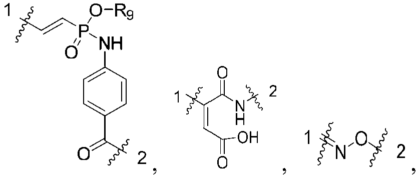 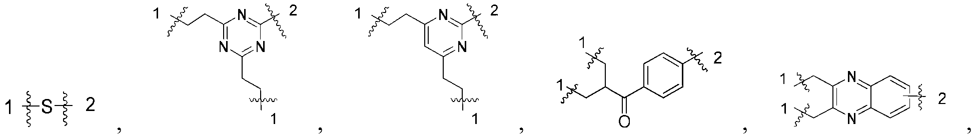 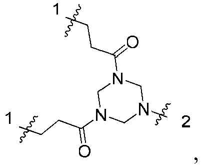 and also included is to use techniques and structures such as enzymatic catalysis (e.g., Sortase, transglutaminase, formylglycine-generating enzyme, isoprenoid transferase, glycosyltransferase, etc.), sulfonyl fluoride, isothiocyanate, etc. to attach Tb to L₂ in a suitable manner, wherein R₉ is selected from C1-6 alkyl, -(CH₂CH₂O)ₚH, and -(CH₂CH₂O)ₚ-C1-6 alkyl, wherein p is selected from any integer between 0 and 30; position 1 is attached to A via a sulfur (S) atom, and position 2 is attached to L₂; or L₁ is selected from 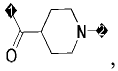 wherein position 1 is attached to A via a nitrogen (N) atom, and position 2 is attached to L₂.

In some embodiments, L₁ is selected from 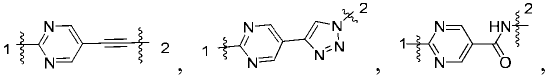 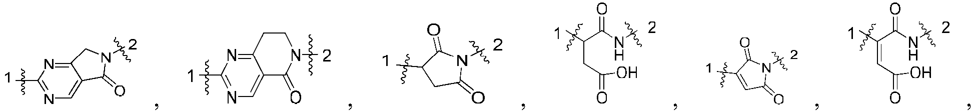 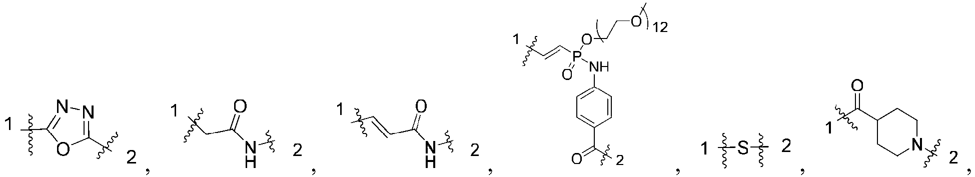 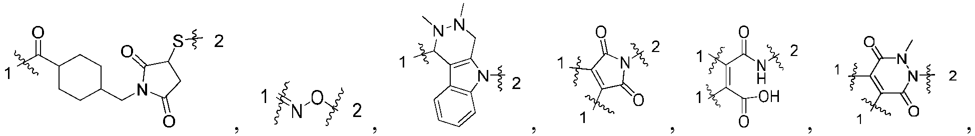 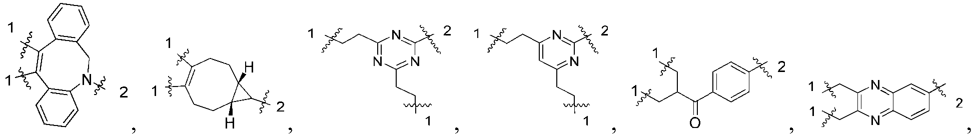 and 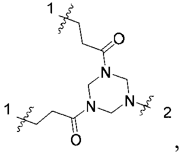 wherein position 1 is attached to A via a sulfur (S) atom, and position 2 is attached to L₂; or L₁ is selected from 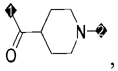 wherein position 1 is attached to A via a nitrogen (N) atom, and position 2 is attached to L₂.

In some embodiments, L₁ is selected from 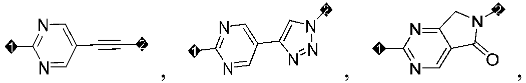 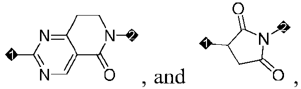 wherein position 1 is attached to A via a sulfur (S) atom, and position 2 is attached to L₂.

In some embodiments, L₁ is selected from 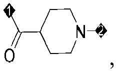 wherein position 1 is attached to A via a nitrogen (N) atom, and position 2 is attached to L₂.

In some embodiments, L₂ is selected from 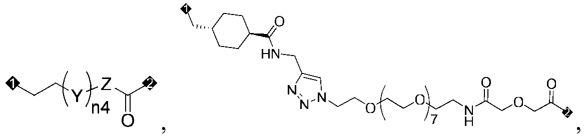 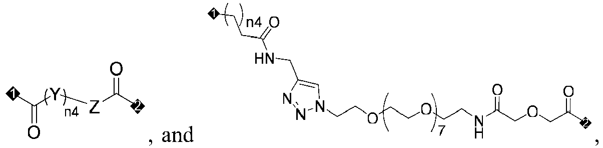 wherein position 1 is attached to L₁, and position 2 is attached to L₃.

In some embodiments, L₂ is selected from 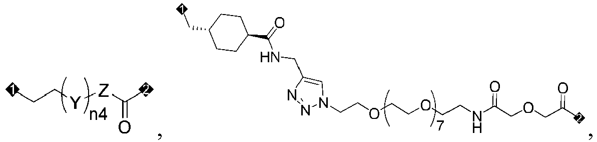 and 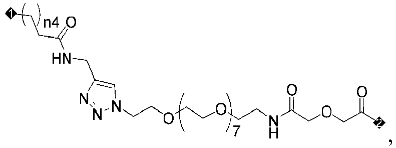 wherein position 1 is attached to L₁, and position 2 is attached to L₃.

In some embodiments, Y is selected from -O-, -CH₂-, -OCH₂CH₂-, 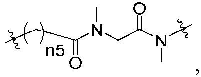 and any combination of more than one of the above groups.

In some embodiments, when selected from 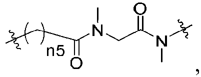 Y may be attached to groups at both ends in any attachment direction.

In some embodiments, Y is -CH₂-.

In some embodiments, n4 and n5 are each independently selected from any integer between 0 and 30.

In some embodiments, Z is selected from -CR^{m}Rⁿ- and -NR^{m}-.

In some embodiments, R^{m} and Rⁿ are each independently selected from hydrogen, C1-6 alkyl, C2-6 alkenyl, C2-6 alkynyl, C3-10 cycloalkyl, and 4-10 membered heterocyclyl;
or R^{m} and Rⁿ, together with the carbon atom to which they are attached, form a 3-6 membered carbocyclic ring or 3-6 membered heterocyclic ring.

In some embodiments, R^{m} and Rⁿ are each independently selected from hydrogen and methyl.

In some embodiments, Z is selected from -CH₂-, -C(CH₃)₂-, -CH₂O-, -N(CH₃)-, and -NH-.

In some embodiments, L₂ is selected from 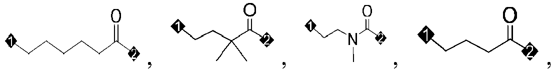 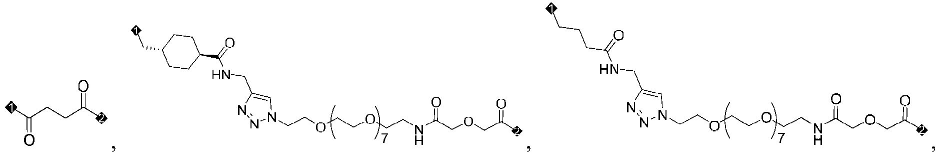 and 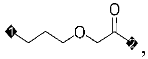 wherein position 1 is attached to L₁, and position 2 is attached to L₃.

In some embodiments, L₂ is selected from 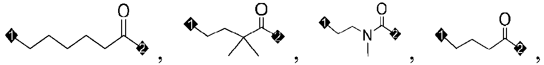 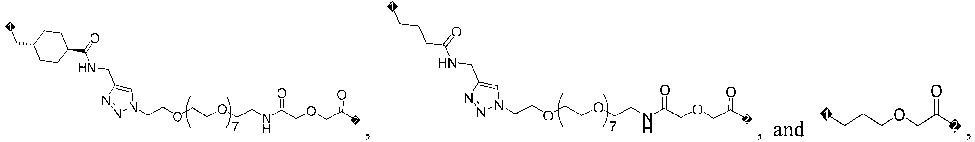 wherein position 1 is attached to L₁, and position 2 is attached to L₃.

In some embodiments, L₁-L₂ is selected from 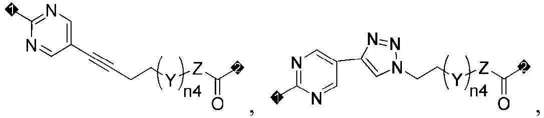 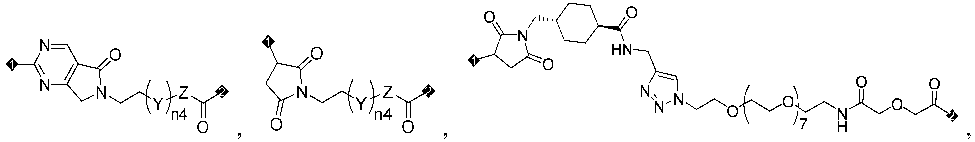 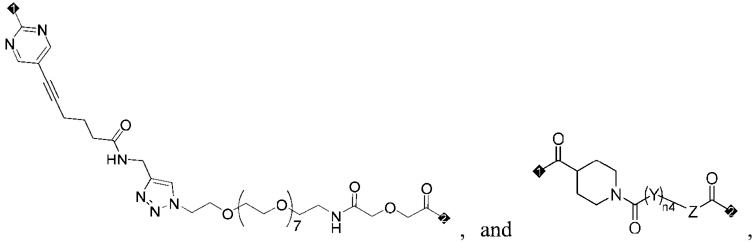 wherein position 1 is attached to A via a sulfur (S) atom or a nitrogen (N) atom, and position 2 is attached to L₃.

In some embodiments, L₁-L₂ is selected from 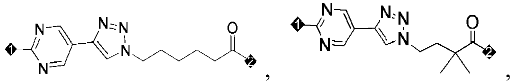 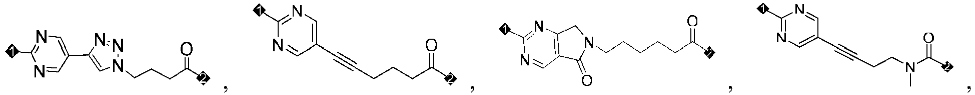 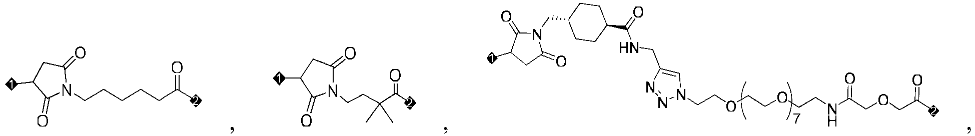 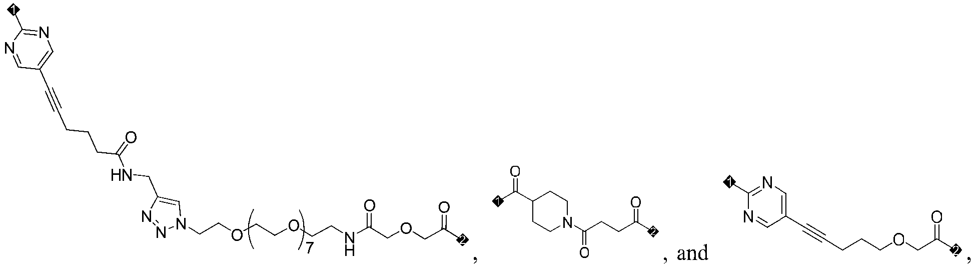 wherein position 1 is attached to A via a sulfur (S) atom or a nitrogen (N) atom, and position 2 is attached to L₃.

In some embodiments, L₁-L₂ is selected from 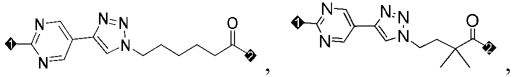 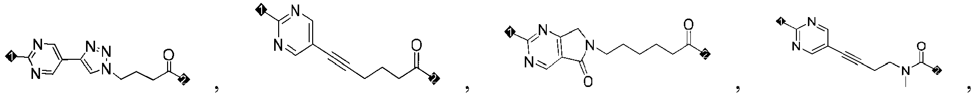 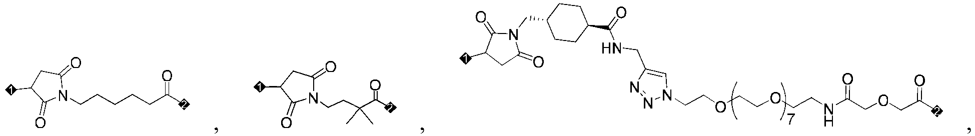 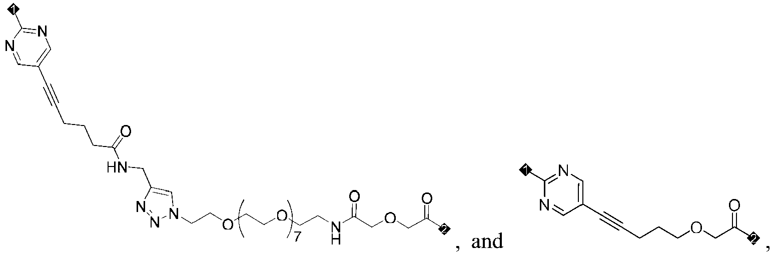 wherein position 1 is attached to A via a sulfur (S) atom, and position 2 is attached to L₃.

In some embodiments, L₁-L₂ is selected from 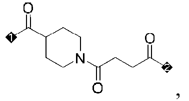 wherein position 1 is attached to A via a nitrogen (N) atom, and position 2 is attached to L₃.

In some embodiments, L₃ is selected from an amino acid residue and a dipeptide, tripeptide, or tetrapeptide consisting of 2-4 amino acid residues, wherein the amino acid residues are selected from natural amino acid residues, non-natural amino acid residues, and amino acid residues represented by AA¹ or stereoisomers thereof. In some embodiments, L₃ is selected from a dipeptide, tripeptide, or tetrapeptide consisting of 2-4 amino acid residues, wherein the amino acid residues are selected from amino acid residues represented by AA¹ or stereoisomers thereof.

In some embodiments, R^{a} and R^{b}, together with the carbon atom to which they are attached, form a 5-6 membered heterocyclic ring or 4-8 membered carbocyclic ring, wherein the 5-6 membered heterocyclic ring or 4-8 membered carbocyclic ring is substituted with R⁰.

In some embodiments, R^{a} and R^{b}, together with the carbon atom to which they are attached, form cyclohexane, a piperidine ring, or a piperazine ring, wherein the cyclohexane, piperidine ring, or piperazine ring is substituted with R⁰.

In some embodiments, R^{a} and R^{b}, together with the carbon atom to which they are attached, form cyclohexane or a piperidine ring, wherein the cyclohexane or piperidine ring is substituted with R⁰.

In some embodiments, R^{a} and R^{b}, together with the carbon atom to which they are attached, form 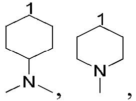 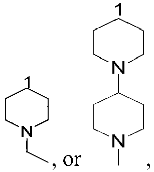 wherein carbon atom No. 1 is the carbon atom to which R^{a} and R^{b} are attached.

In some embodiments, one of R^{a} and R^{b} is hydrogen, and the other is 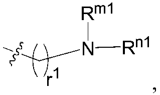 wherein r¹, R^{m1}, and Rⁿ¹ are as defined in any one of the embodiments of the present disclosure.

In some embodiments, r¹ is selected from 0, 1, 2, 3, 4, and 5.

In some embodiments, r¹ is 4.

In some embodiments, R^{m1} and Rⁿ¹ are each independently selected from hydrogen and C1-6 alkyl.

In some embodiments, R^{m1} and Rⁿ¹ are each independently selected from hydrogen, methyl, ethyl, n-propyl, and n-butyl.

In some embodiments, R^{m1} and Rⁿ¹, together with the nitrogen atom to which they are attached, form a 5-6 membered heterocyclic ring optionally substituted with R^{0'}.

In some embodiments, R^{m1} and Rⁿ¹, together with the nitrogen atom to which they are attached, form a piperidine ring or piperazine ring, wherein the piperidine ring or piperazine ring is optionally substituted with R^{0'}.

In some embodiments, R^{m1} and Rⁿ¹, together with the nitrogen atom to which they are attached, form 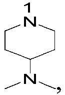 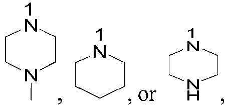 wherein nitrogen atom No. 1 is the nitrogen atom to which R^{m1} and Rⁿ¹ are attached.

In some embodiments, R⁰ and R^{0'} are each independently selected from C1-6 alkyl, -NR^{m2}Rⁿ², and 5-6 membered heterocyclyl, wherein the 5-6 membered heterocyclyl is optionally substituted with C1-6 alkyl.

In some embodiments, R⁰ is selected from C1-6 alkyl and 5-6 membered heterocyclyl, wherein the 5-6 membered heterocyclyl is substituted with C1-6 alkyl and is selected from piperidinyl and piperazinyl.

In some embodiments, R⁰ is selected from methyl, ethyl, and 5-6 membered heterocyclyl, wherein the 5-6 membered heterocyclyl is substituted with methyl and is piperidinyl.

In some embodiments, R⁰ is selected from methyl, ethyl, and

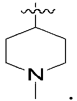

In some embodiments, R^{0'} is selected from C1-6 alkyl and -NR^{m2}Rⁿ².

In some embodiments, R^{0'} is selected from methyl and -NR^{m2}Rⁿ².

In some embodiments, R^{m2} and Rⁿ² are methyl.

In some embodiments, in the structure of the amino acid residues represented by AA¹:
one of R^{a} and R^{b} is hydrogen, the other is 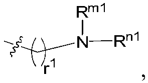 and r¹ is 4;
or R^{a} and R^{b}, together with the carbon atom to which they are attached, form a 5-6 membered heterocyclic ring or 5-7 membered carbocyclic ring, wherein the 5-6 membered heterocyclic ring or 5-7 membered carbocyclic ring is substituted with R⁰;
R^{m1} and Rⁿ¹ are each independently selected from hydrogen and C1-6 alkyl;
or R^{m1} and Rⁿ¹, together with the nitrogen atom to which they are attached, form a 5-6 membered heterocyclic ring optionally substituted with R^{0'};
R⁰ is selected from C1-6 alkyl, -NR^{m2}Rⁿ², and 5-6 membered heterocyclyl, wherein the 5-6 membered heterocyclyl is optionally substituted with C1-6 alkyl;
R^{0'} is selected from C1-6 alkyl and -NR^{m2}Rⁿ²;
R^{m2} and Rⁿ² are each independently selected from hydrogen and C1-6 alkyl.

In some embodiments, the amino acid residues represented by AA¹ are selected from

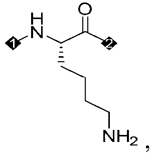

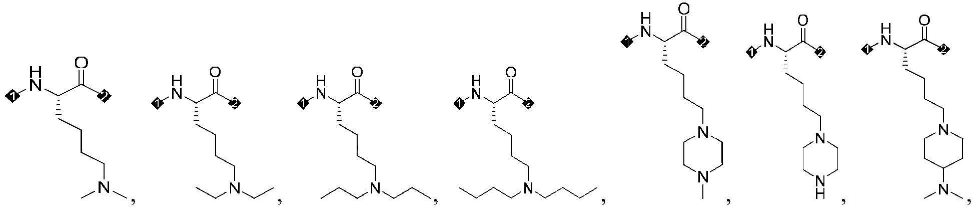

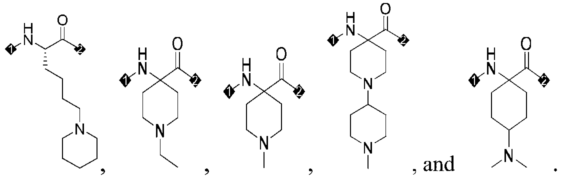

In some embodiments, the amino acid residues represented by AA¹ are selected from

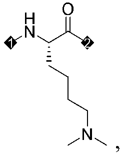

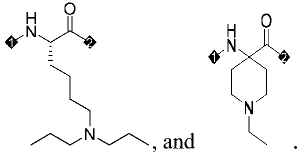

In some embodiments, L₃ is selected from a dipeptide, a tripeptide, and a tetrapeptide.

In some embodiments, L₃ is selected from a dipeptide comprising an amino acid residue represented by AA¹ or a stereoisomer thereof, a tripeptide comprising an amino acid residue represented by AA¹ or a stereoisomer thereof, and a tetrapeptide comprising an amino acid residue represented by AA¹ or a stereoisomer thereof.

In some embodiments, the dipeptide is selected from Val-Ala, Val-Cit, Ala-Ala, Val-AA¹, Ala-AA¹, and Gly-AA¹.

In some embodiments, the dipeptide is selected from Val-AA¹.

In some embodiments, the dipeptide is selected from 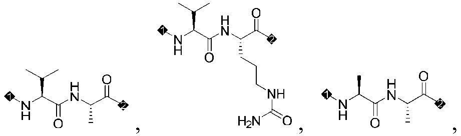 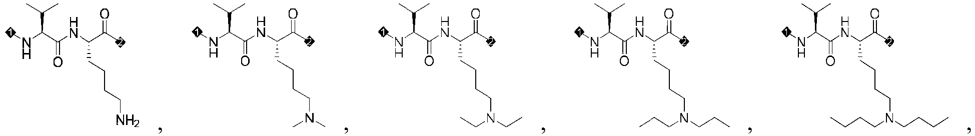 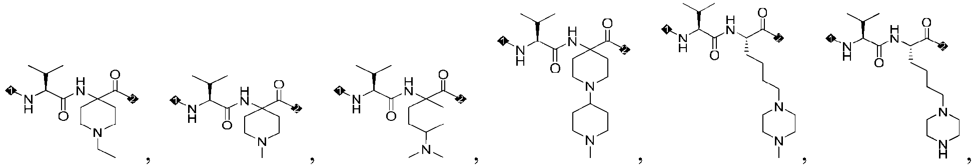 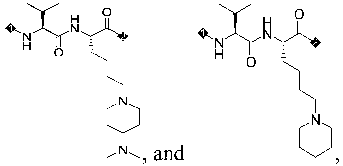 wherein position 1 is attached to L₂, and position 2 is attached to L₄.

In some embodiments, the tripeptide is selected from Val-AA¹-Gly, Ala-AA¹-Gly, Gly-AA¹-Gly, Val-AA¹-Ala, Val-AA¹-Val, Ala-AA¹-Ala, Ala-AA¹-Val, Gly-AA¹-Ala, Gly-AA¹-Val, Ala-Ala-Ala, and Ala-Ala-Asn.

In some embodiments, the tripeptide is selected from 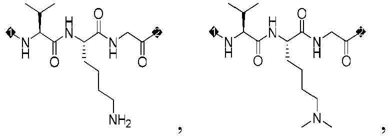 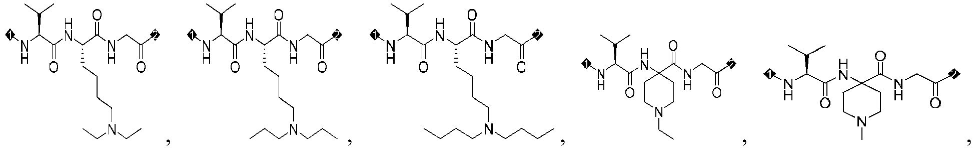 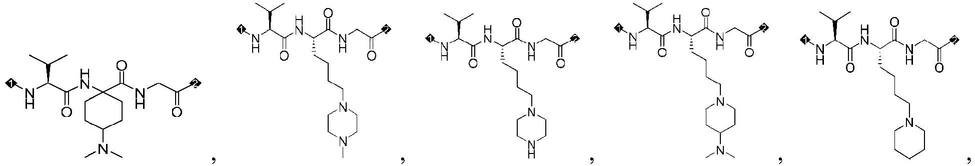 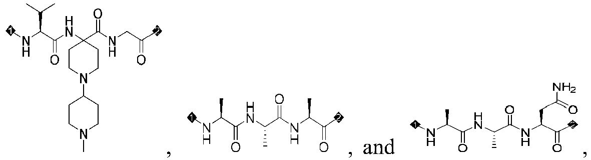 wherein position 1 is attached to L₂, and position 2 is attached to L₄.

In some embodiments, the tetrapeptide is selected from 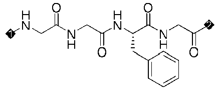 and 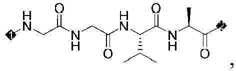 wherein position 1 is attached to L₂, and position 2 is attached to L₄.

In some embodiments, L₄ is selected from a direct bond, 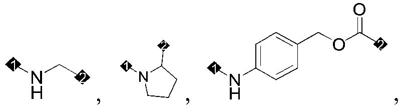 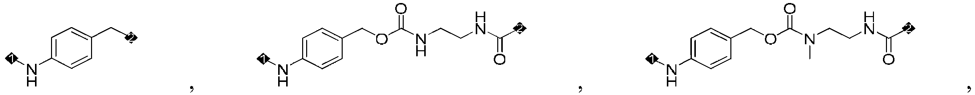 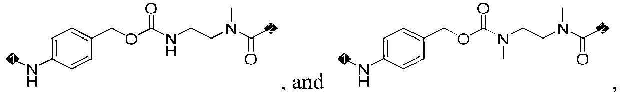 wherein position 1 is attached to L₃, and position 2 is attached to D.

In some embodiments, L₄ is selected from a direct bond, 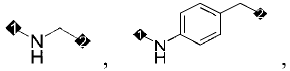 and 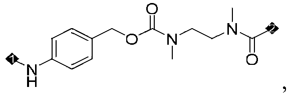 wherein position 1 is attached to L₃, and position 2 is attached to D.

In some embodiments, 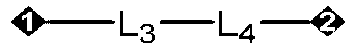 is selected from the following:

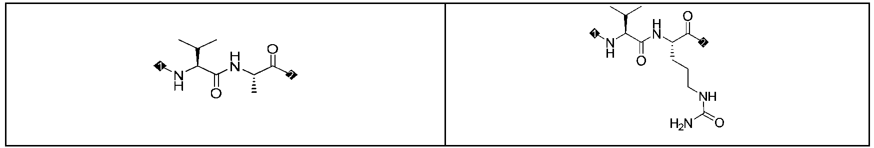

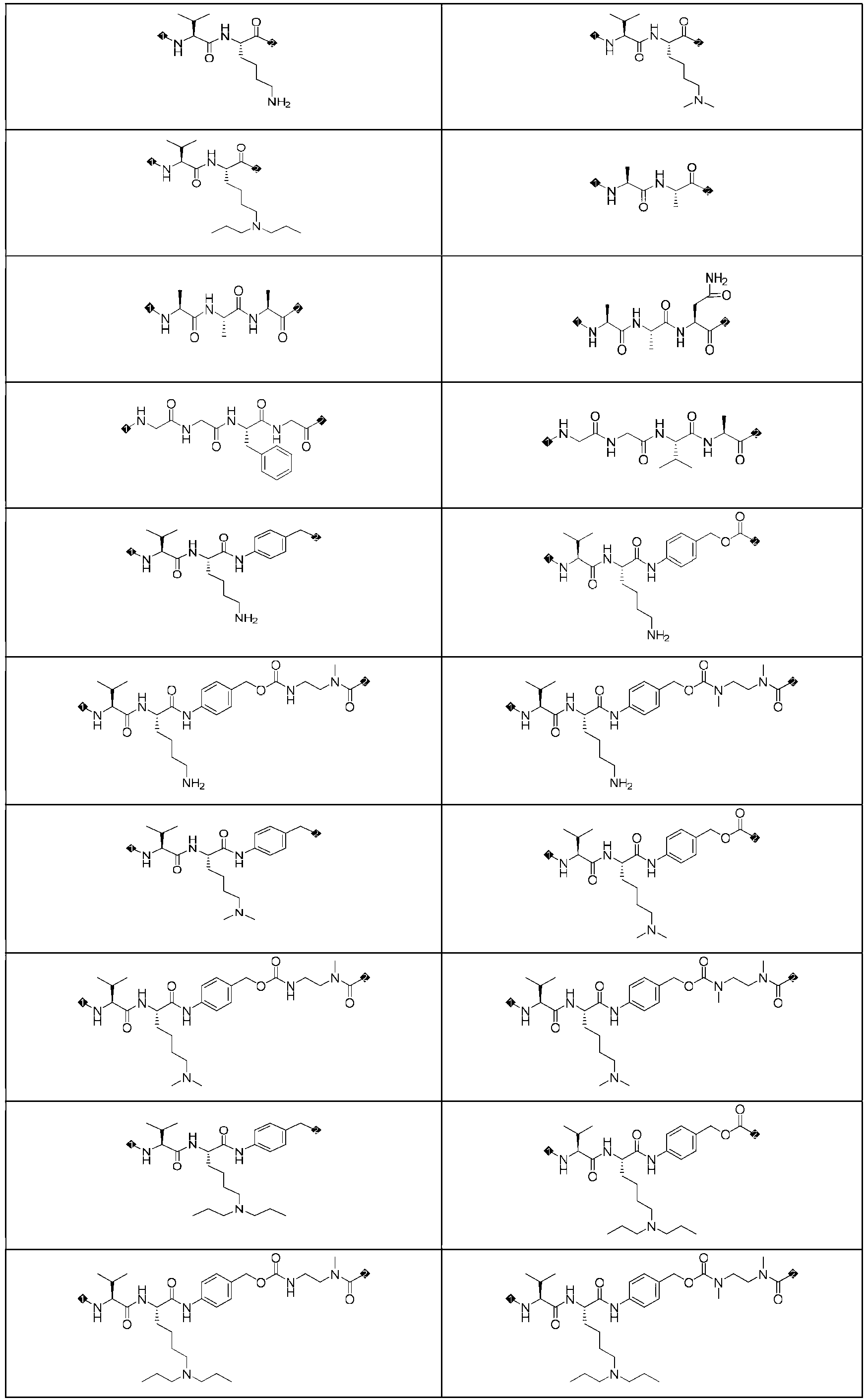

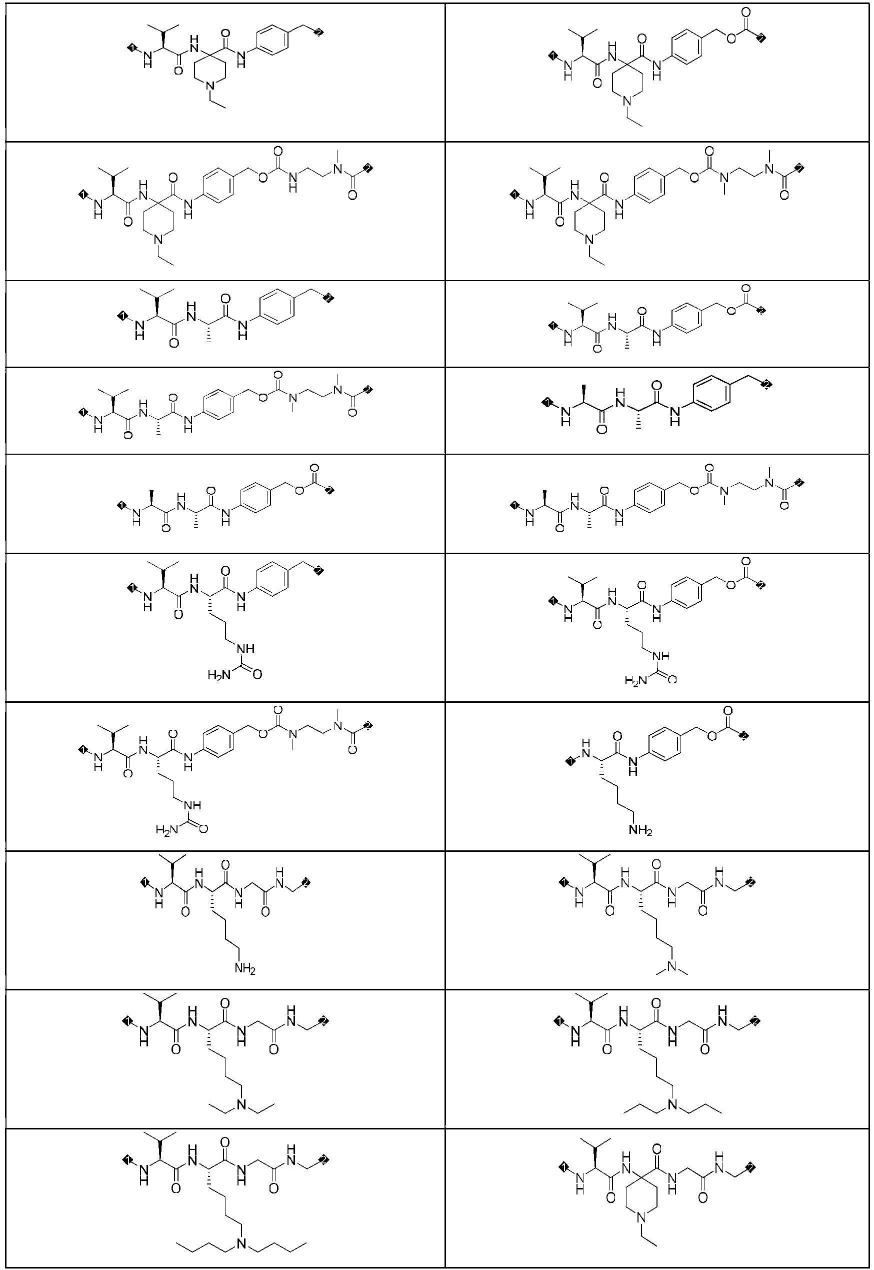

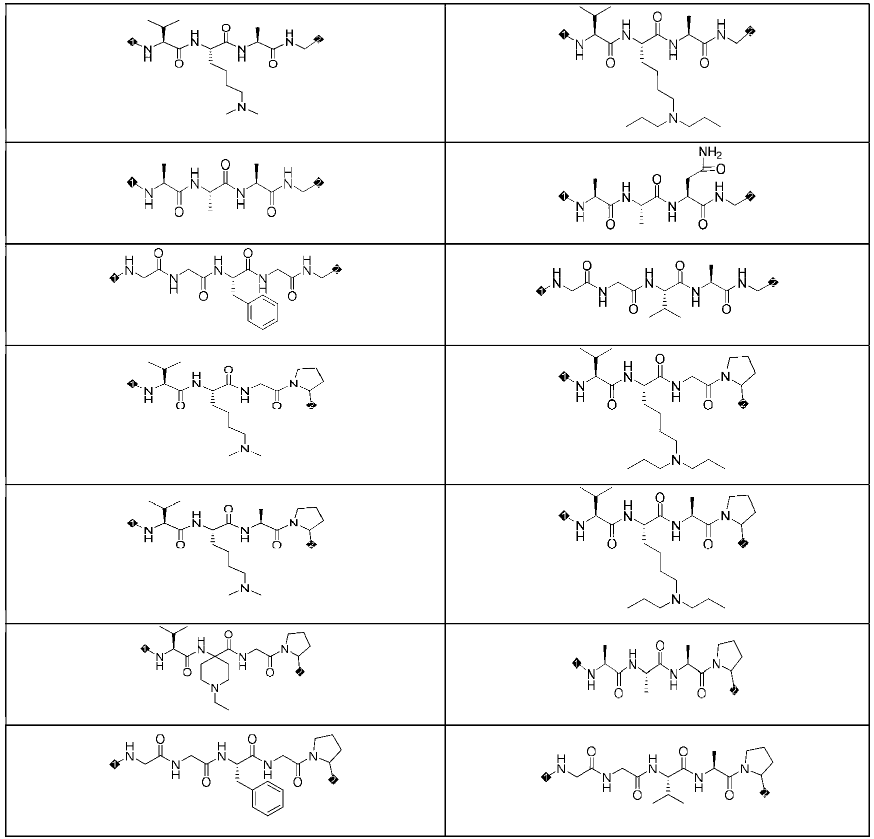

wherein position 1 is attached to L₂, and position 2 is attached to D.

In some embodiments, 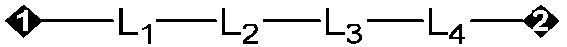 is selected from the following:

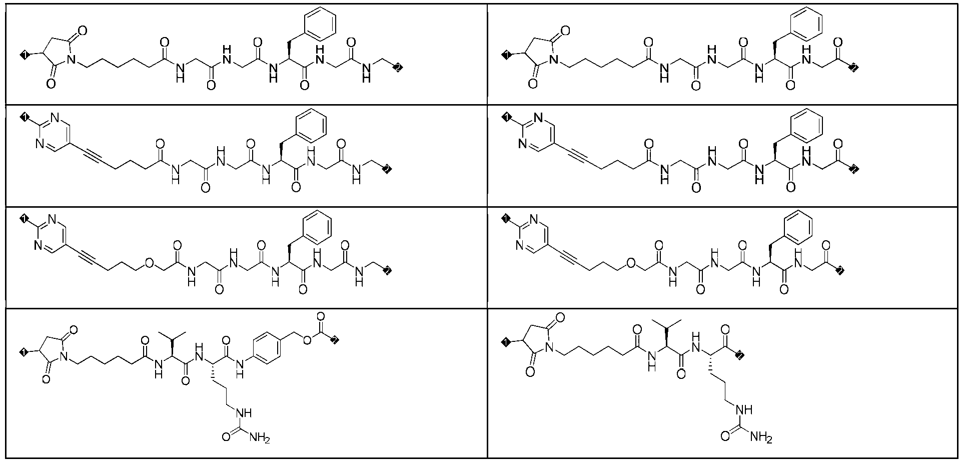

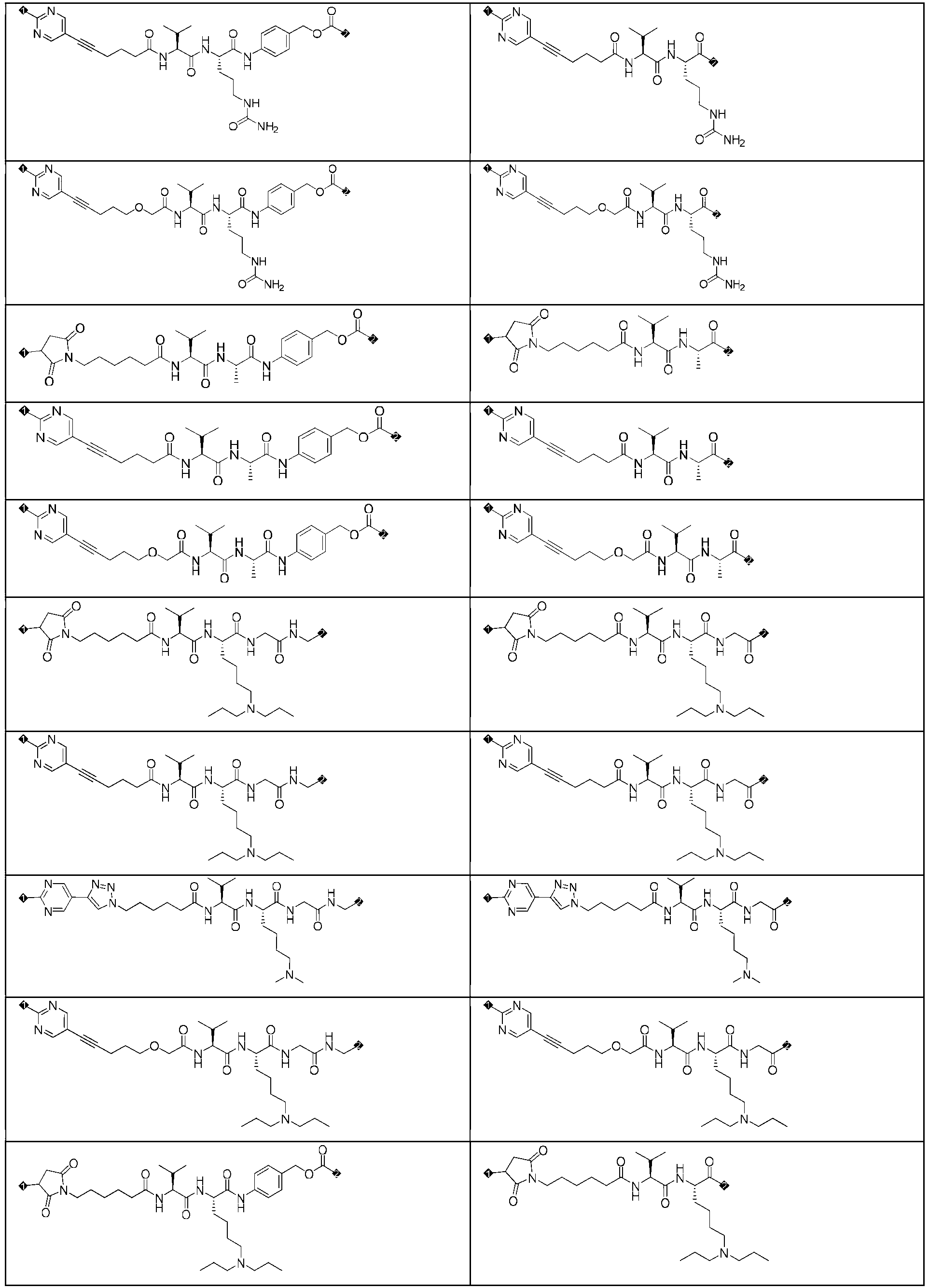

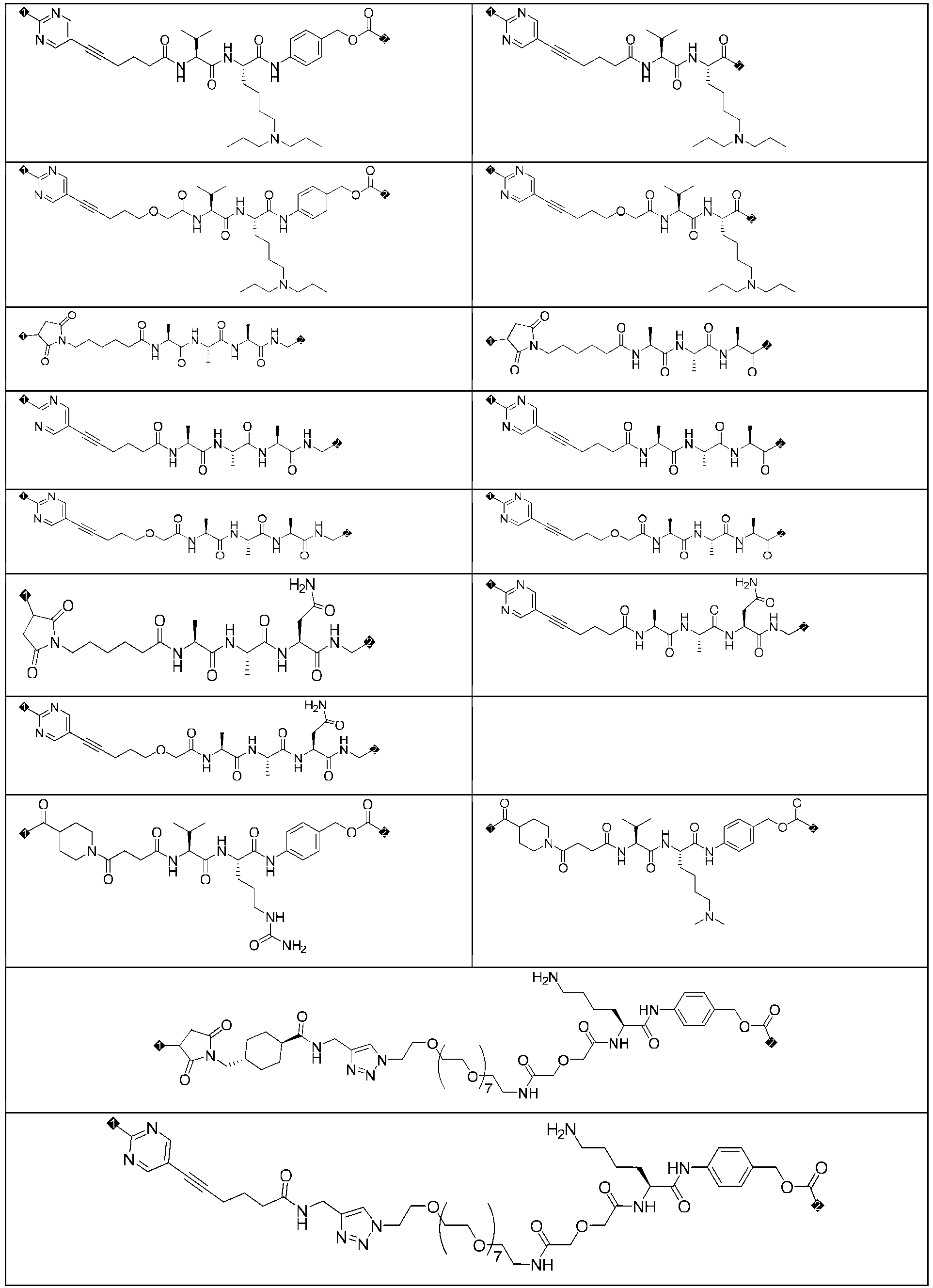

wherein position 1 is attached to A, and position 2 is attached to D.

In some embodiments, the antibody-drug conjugate is selected from the following:

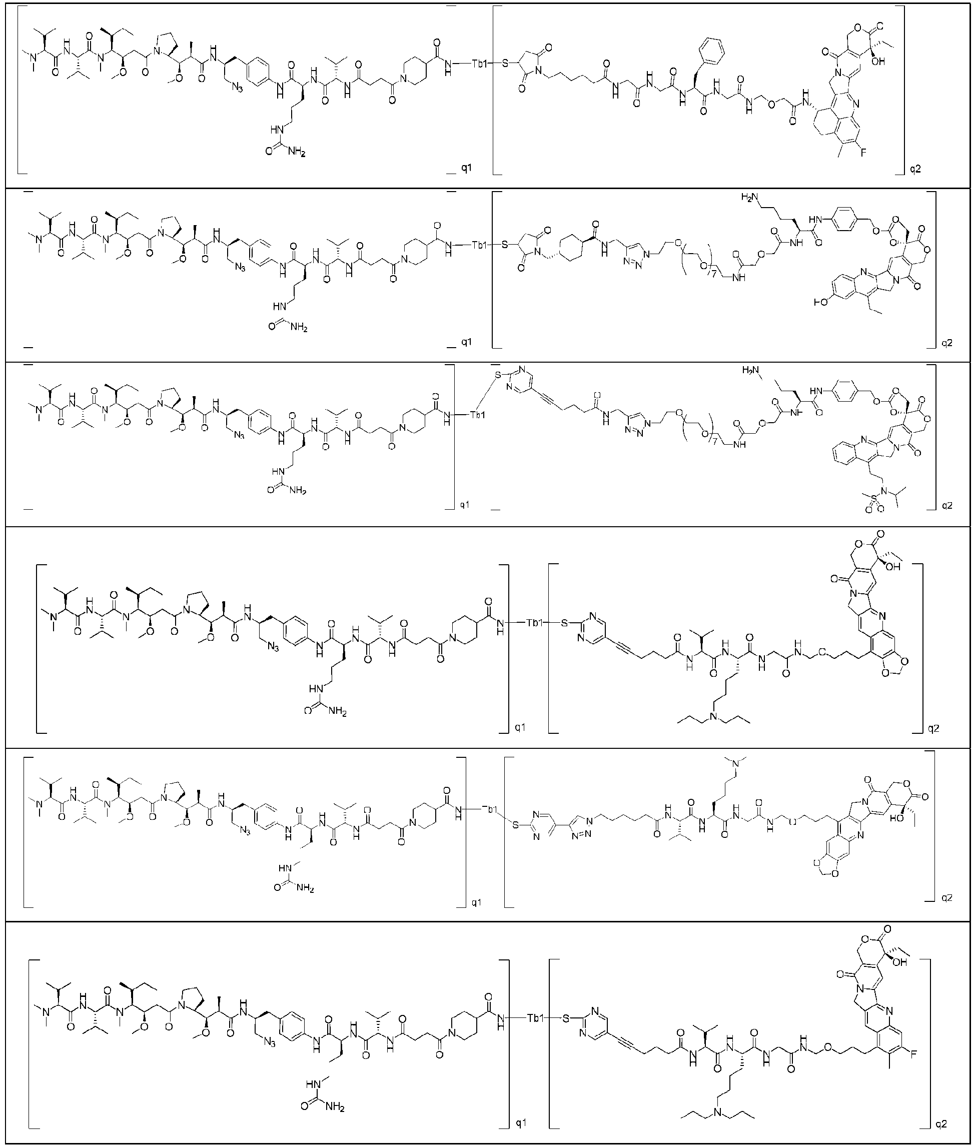

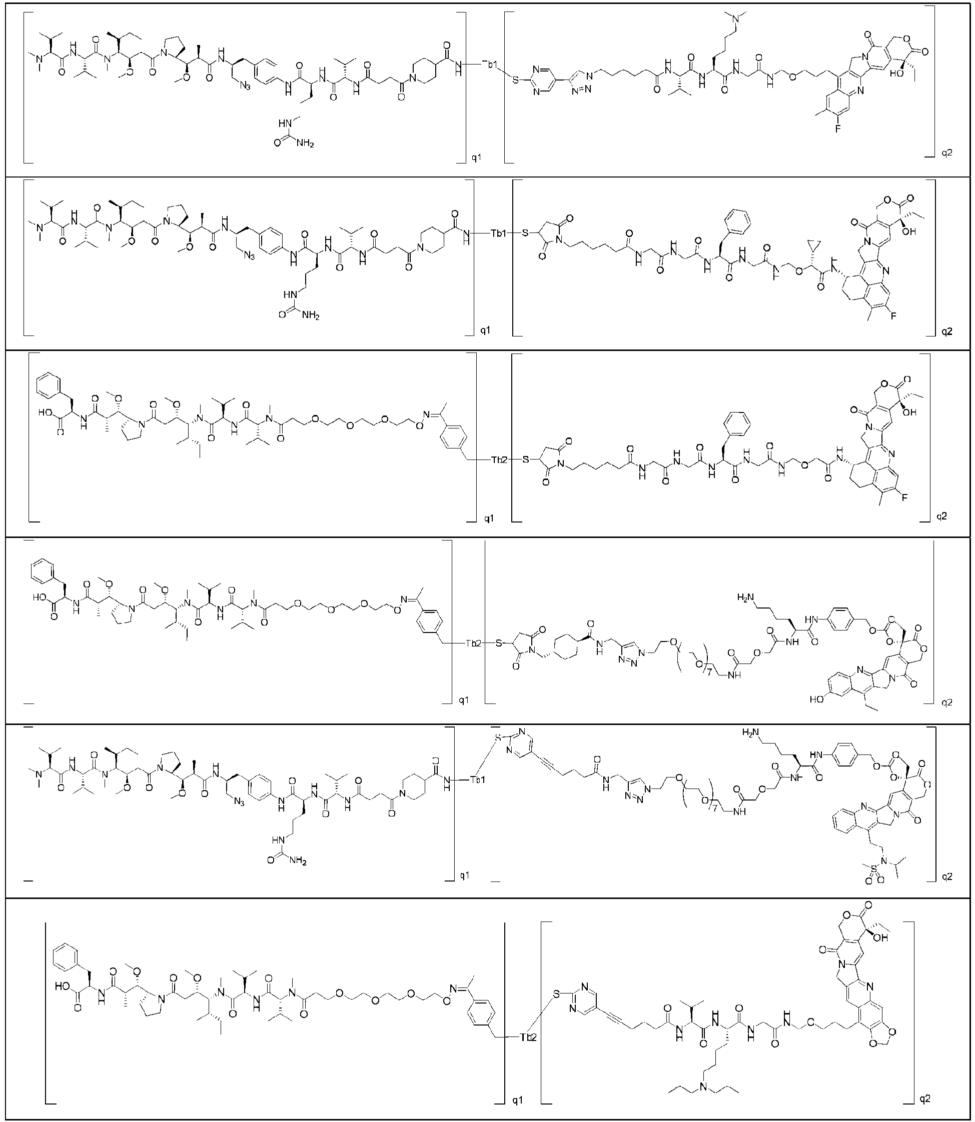

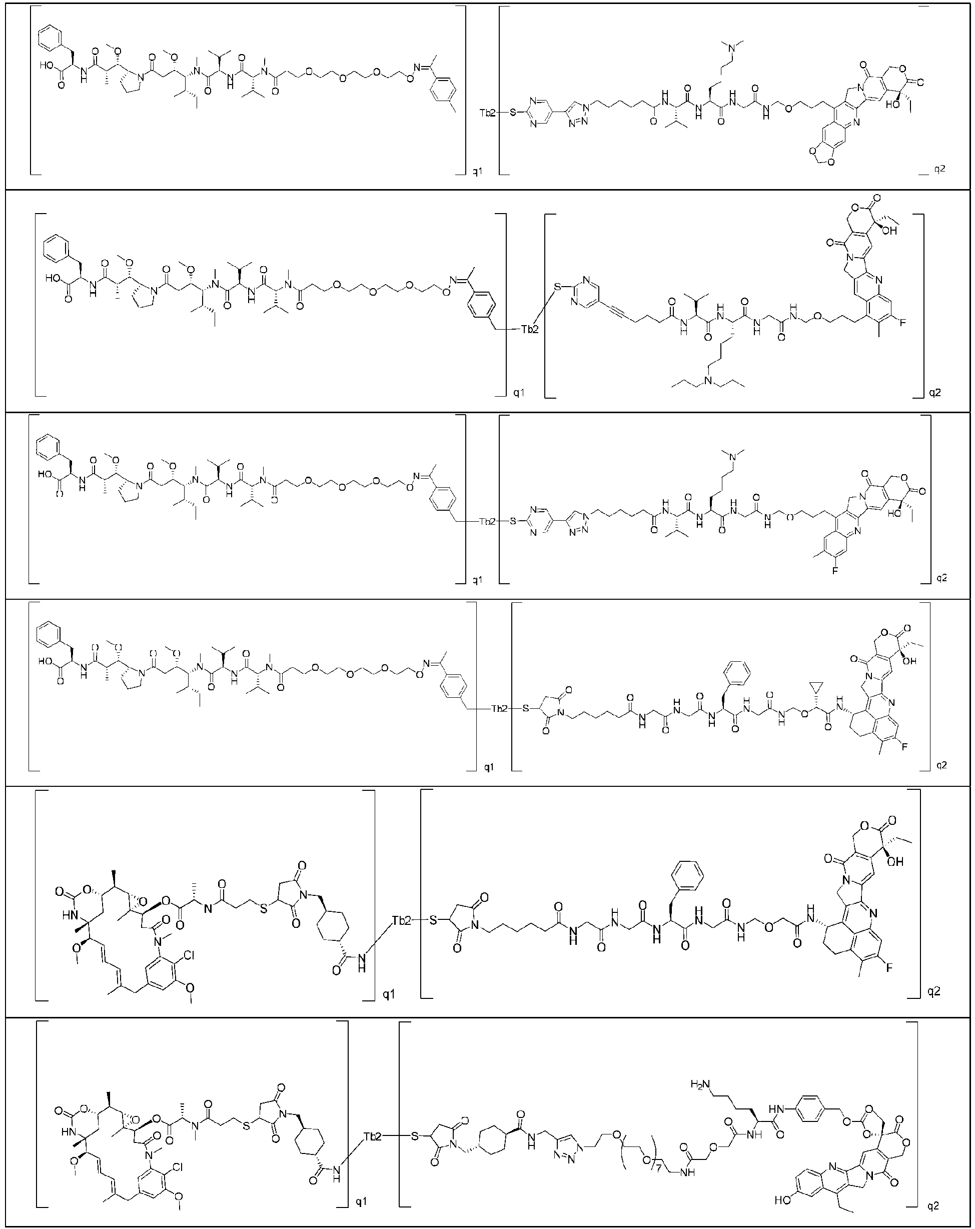

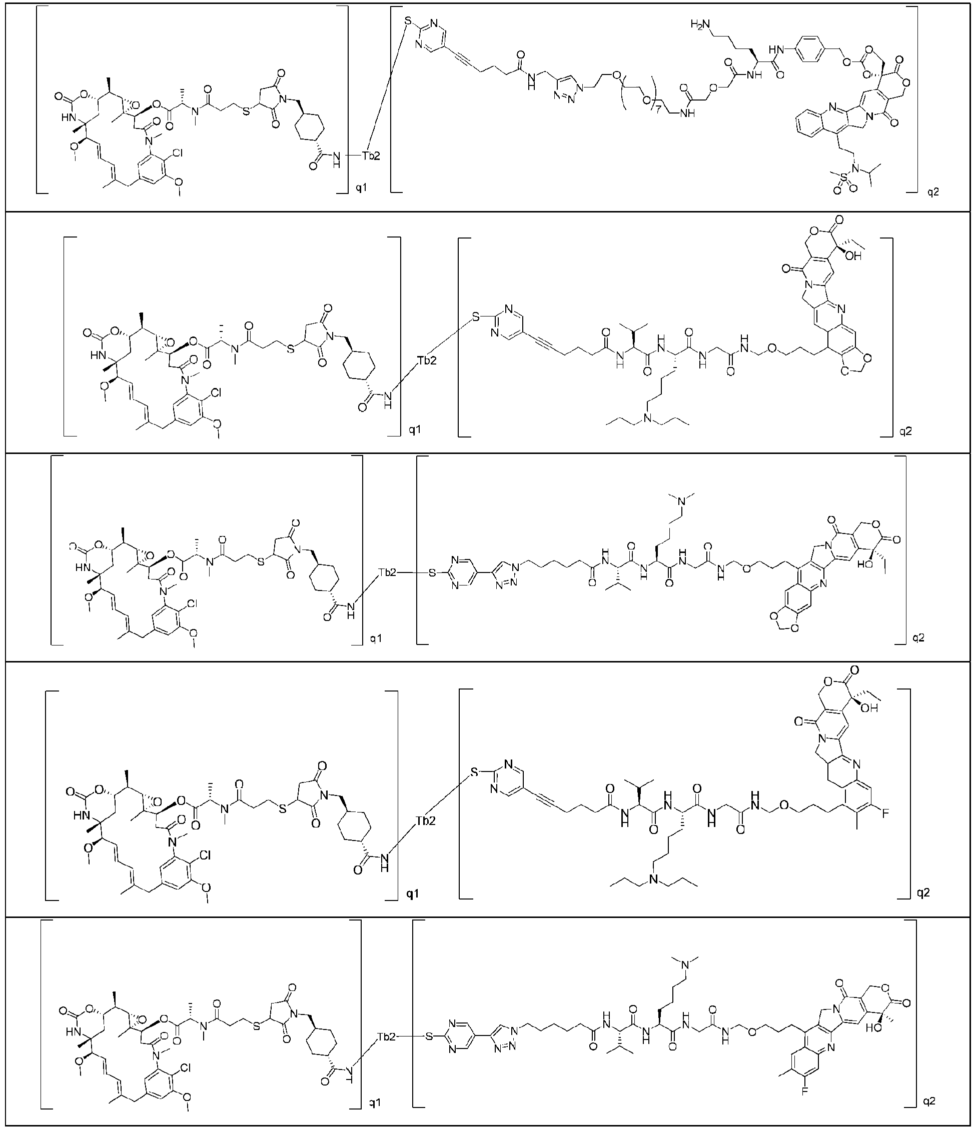

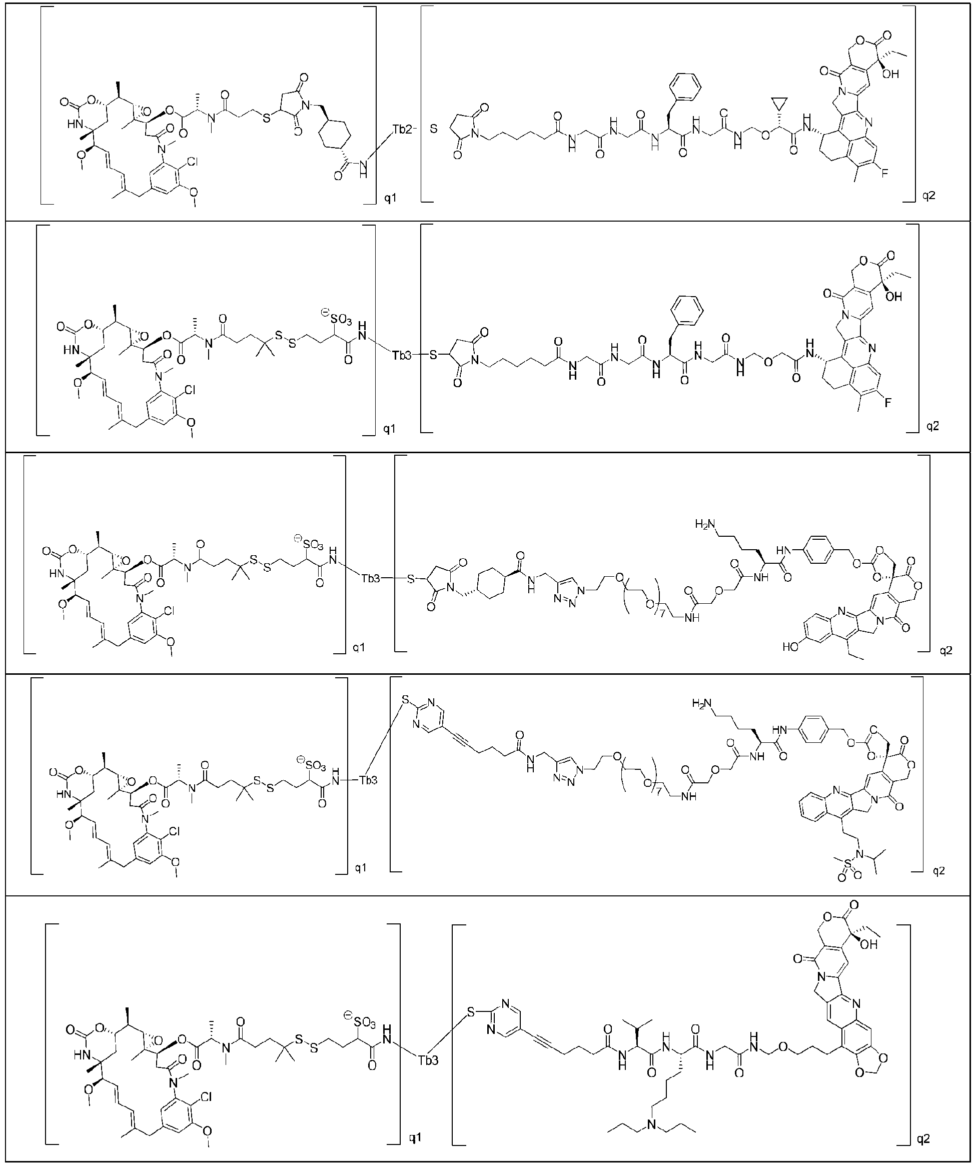

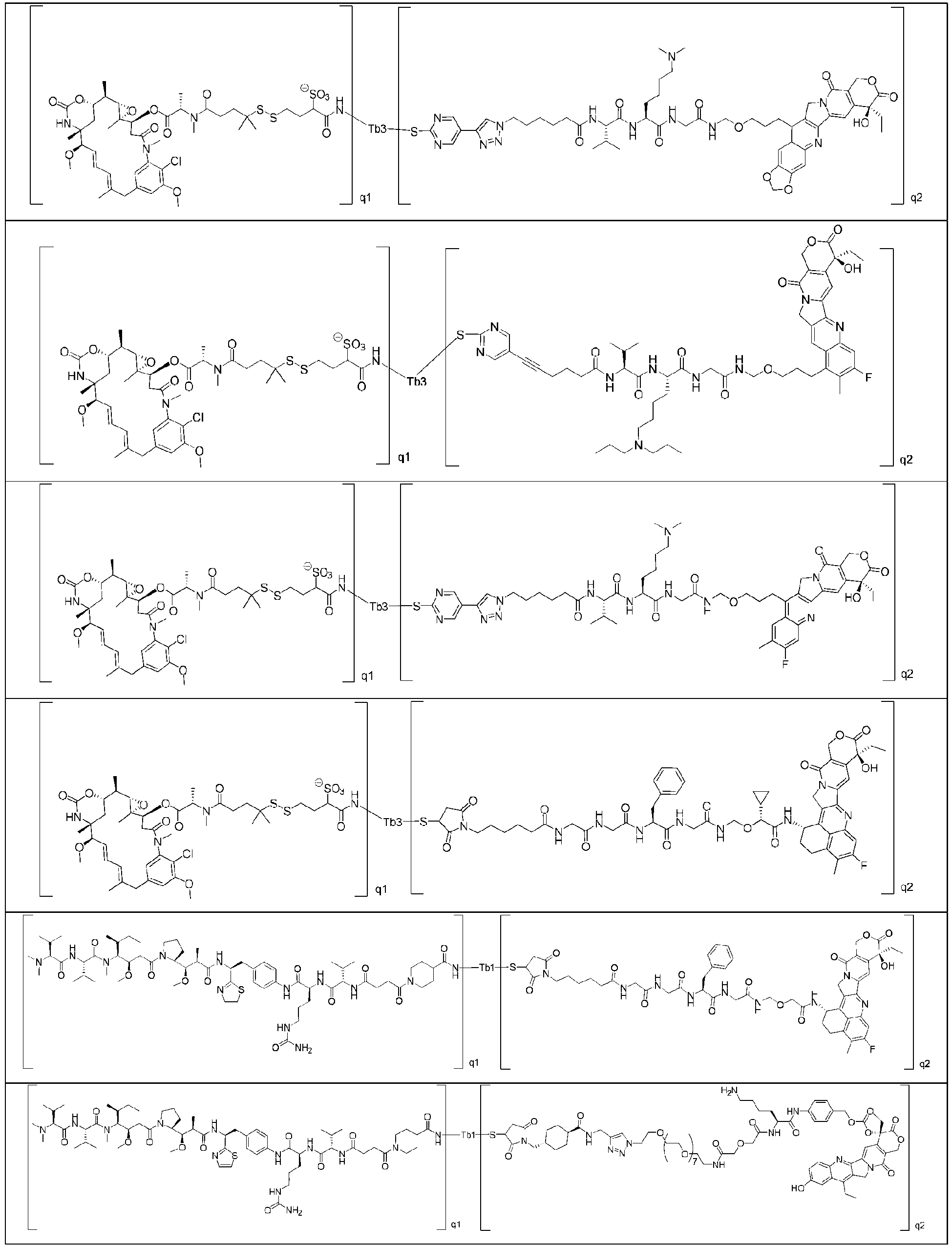

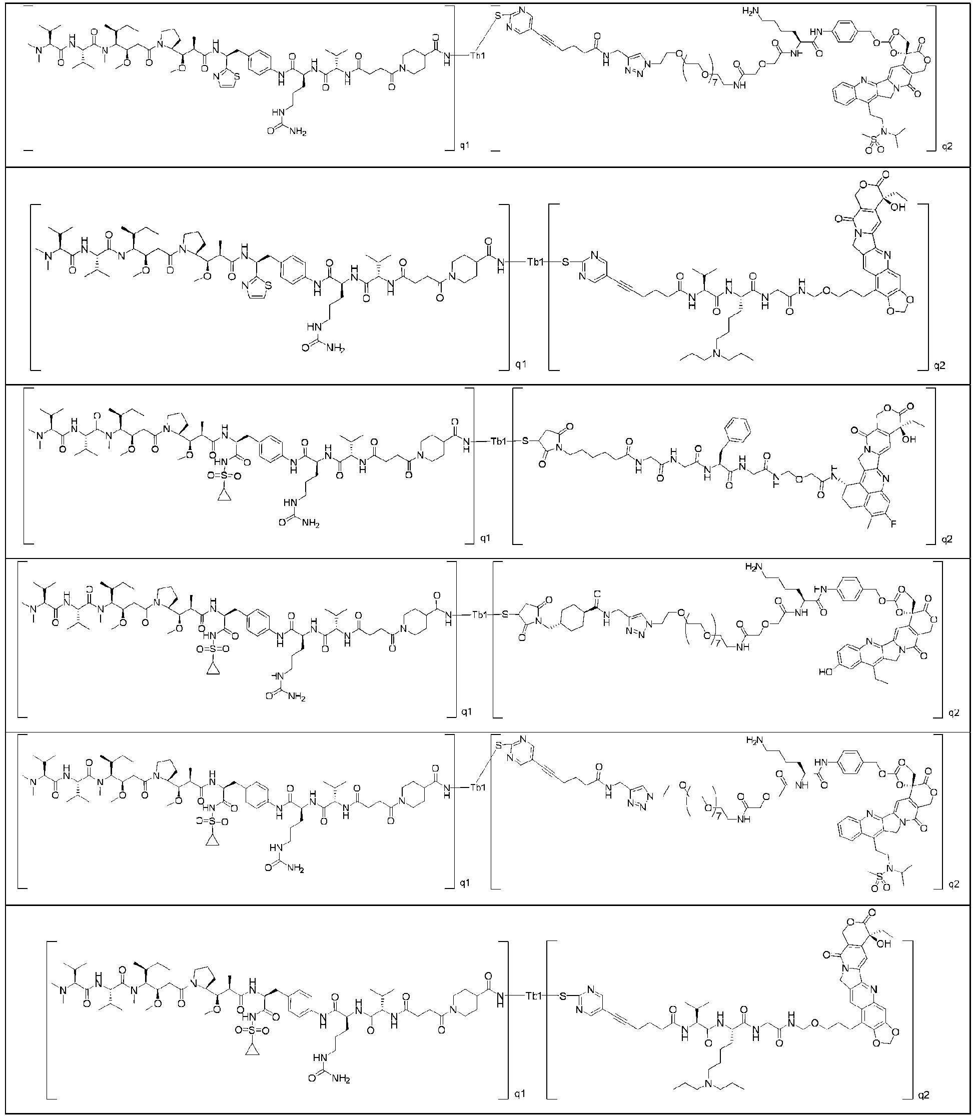

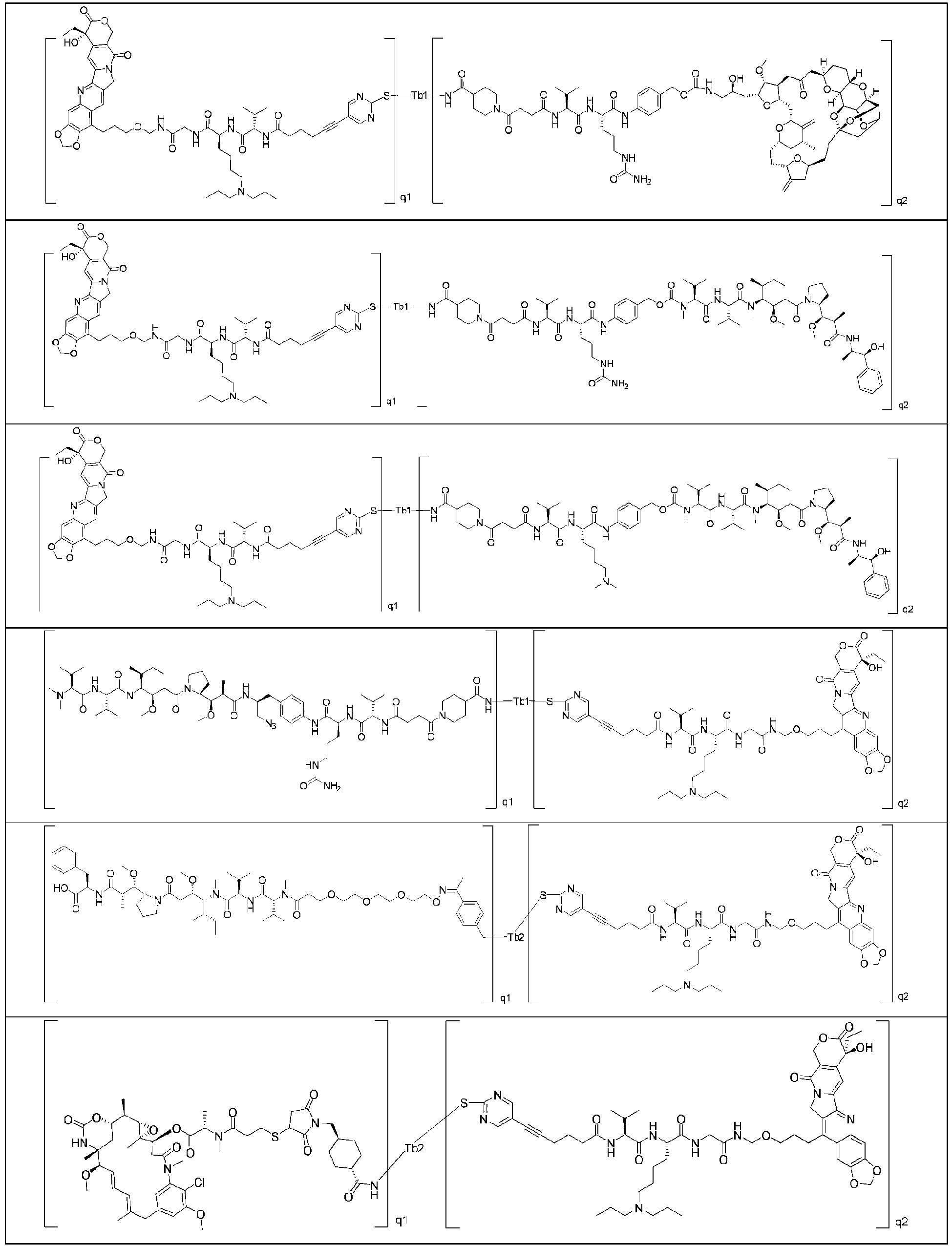

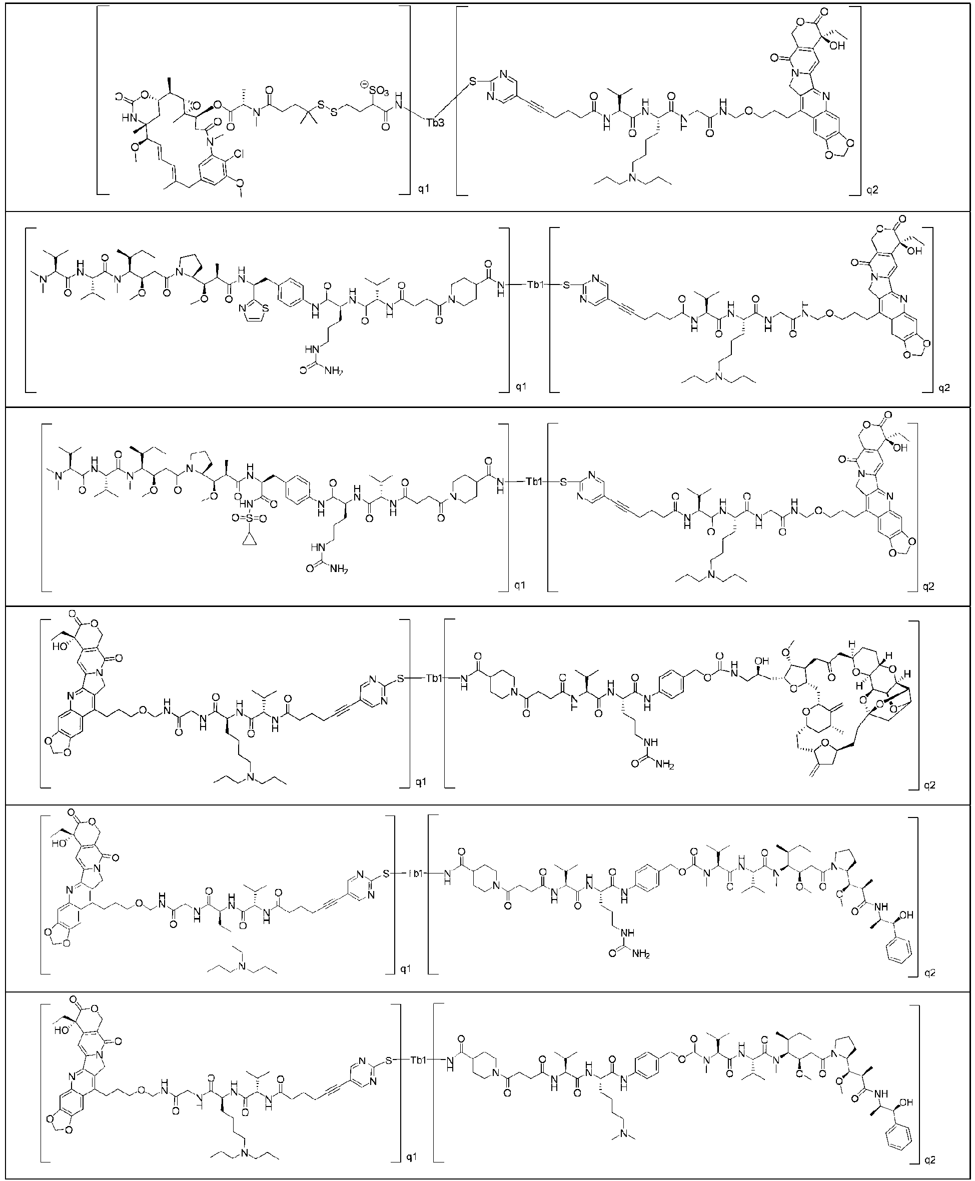

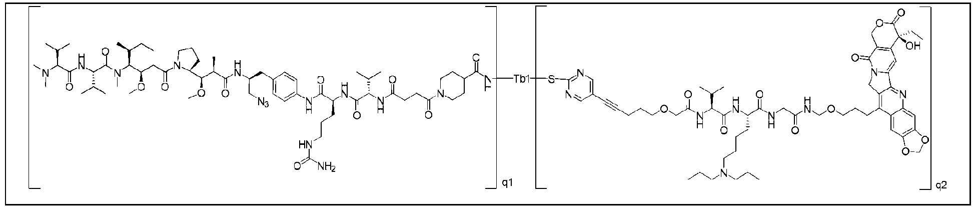

wherein q1 and q2 are each independently selected from any numerical value between 1 and 10;
Tb1, Tb2, and Tb3 are each independently an antibody or an antigen-binding fragment thereof.

In some embodiments, Tb1, Tb2, and Tb3 are each independently selected from the antibody or the antigen-binding fragment thereof described above for Tb.

In some embodiments, Tb1, Tb2, and Tb3 are each independently selected from an antibody or an antigen-binding fragment thereof that targets one or more of the following targets: B7H3, CD20, CD19, CD30, GPNMB, Her2, Trop-2, EGFR, GD-2, CD79b, FRα, NAPI2B, BCMA, etc.

In some embodiments, Tb1, Tb2, and Tb3 are each independently selected from an anti-B7H3 antibody or an antigen-binding fragment thereof, an anti-Trop-2 antibody or an antigen-binding fragment thereof, an anti-Her 2 antibody or an antigen-binding fragment thereof, an anti-FRα antibody or an antigen-binding fragment thereof, and an anti-NAPI2B antibody or an antigen-binding fragment thereof.

In some embodiments, the ratio of q1 to q2 is: 1.0-6.0:4.0-10.0, 1.6-4.0:6.0-9.0, 1.8-3.0:7.0-9.0, 1.9-2.4:7.5-8.5, 1.9-2.2:7.5-8.5, 1.9-2.2:7.8-8.2, 1.9-2.2:7.9-8.1, or 2:8.

In some embodiments, the ratio of q1 to q2 is: 2:4.0-9.5, 2:5.0-9.0, 2:6.0-8.8, 2:7.0-8.6, 2:7.5-8.5, 2:7.6-8.4, 2:7.7-8.3, 2:7.8-8.2, or 2:7.9-8.1.

In some embodiments, the ratio of q1 to q2 is: 1.0-6.0:8, 1.2-5.5:8, 1.4-5.0:8, 1.5-4.5:8, 1.6-4.0:8, 1.7-3.5:8, 1.8-3.0:8, 1.8-2.5:8, 1.8-2.4:8, 1.8-2.3:8, 1.9-2.2:8, 1.1:8, 1.4:8, 1.8:8, 1.9:8, 2.4:8, 2.3:8, 2.5:8, 3.3:8, or 3.7:8.

In some embodiments, the antibody-drug conjugate is selected from the following:

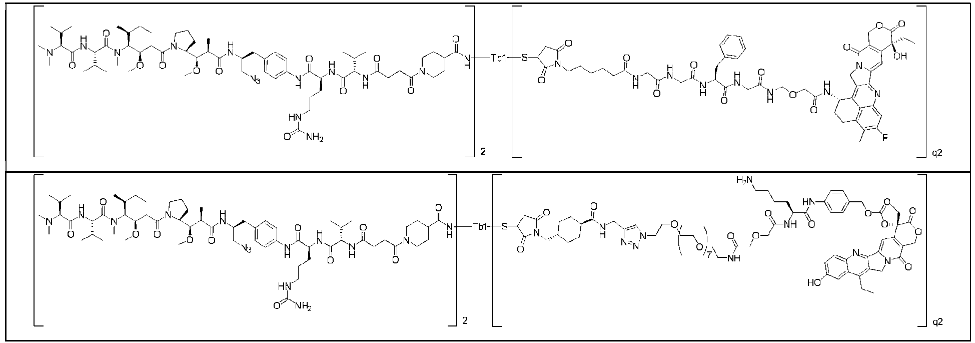

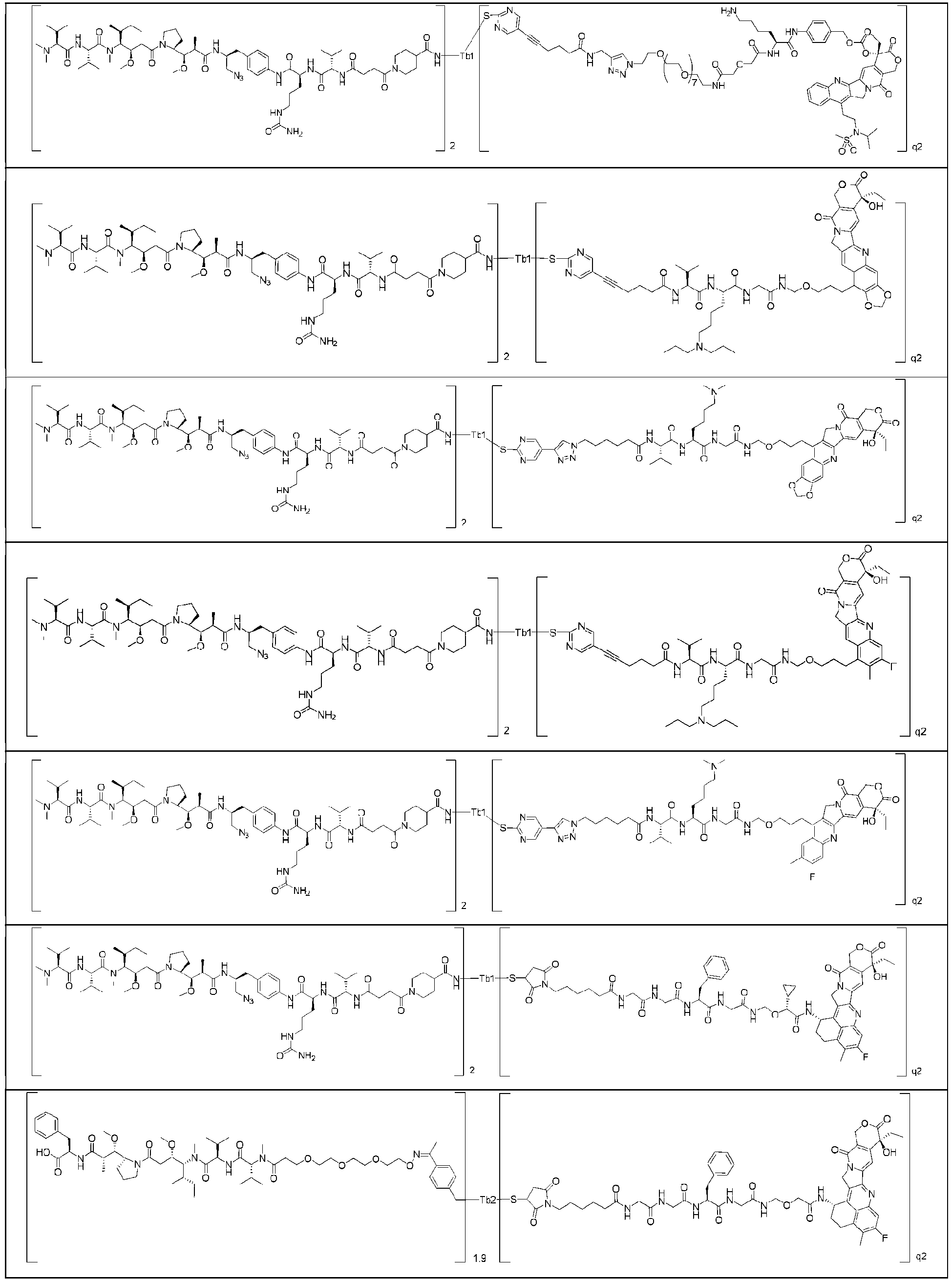

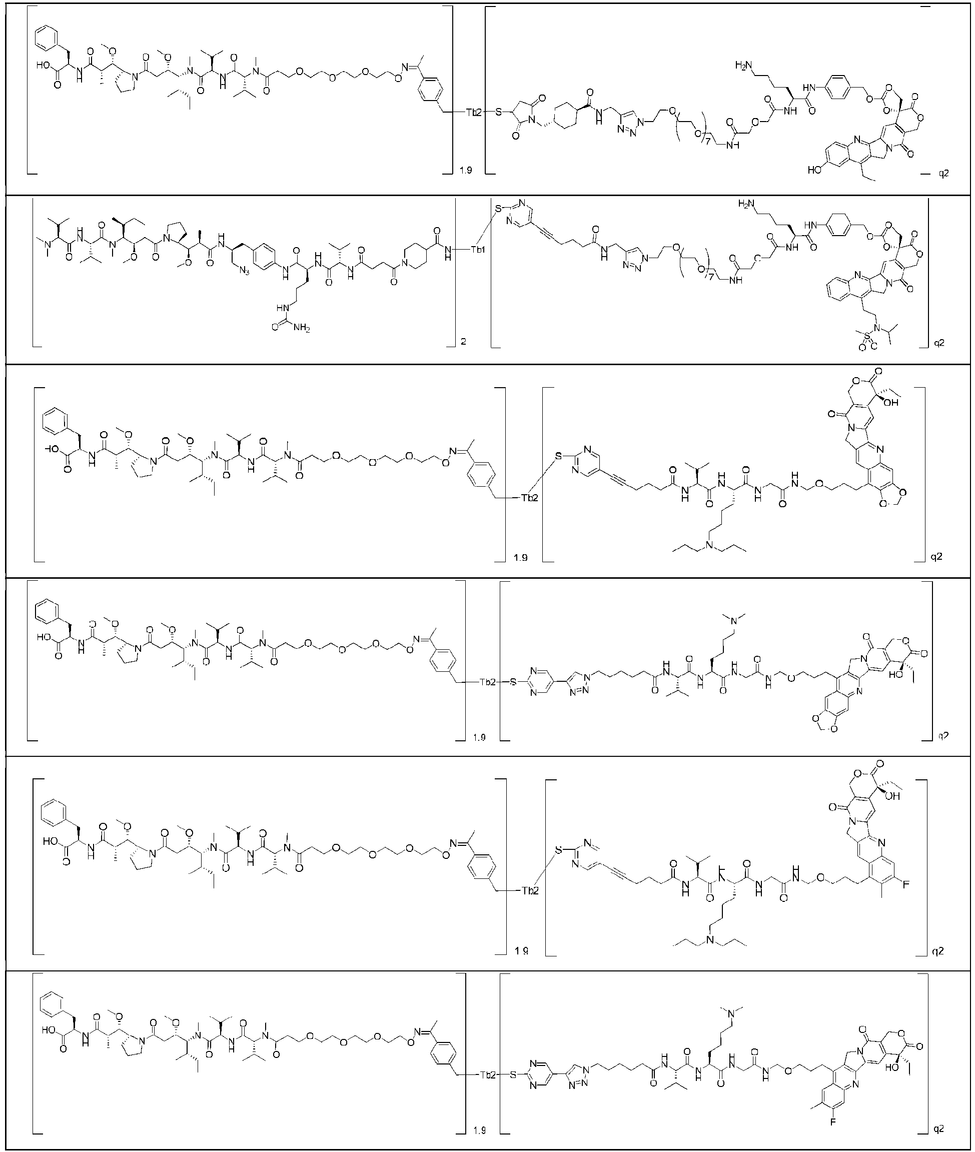

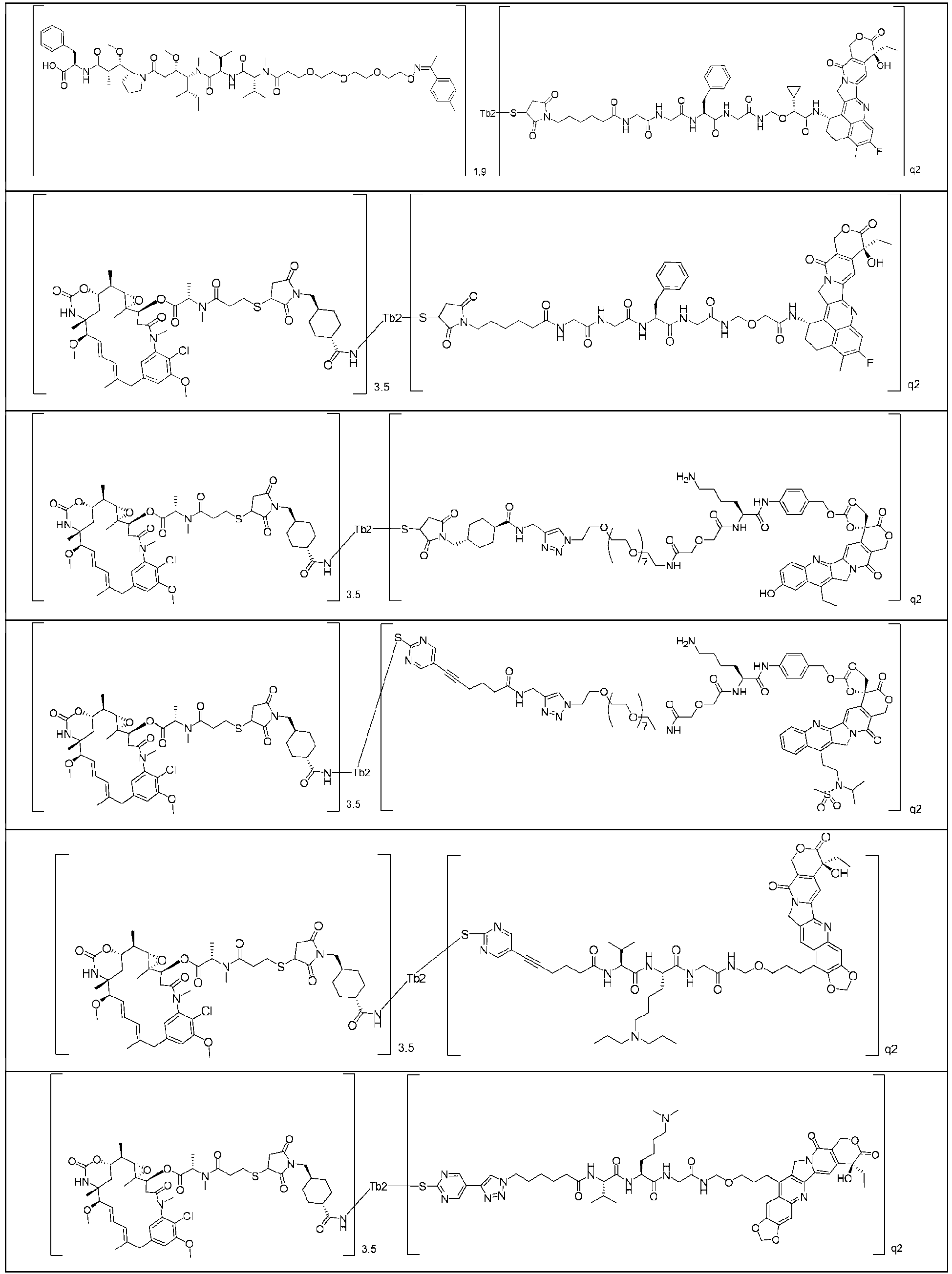

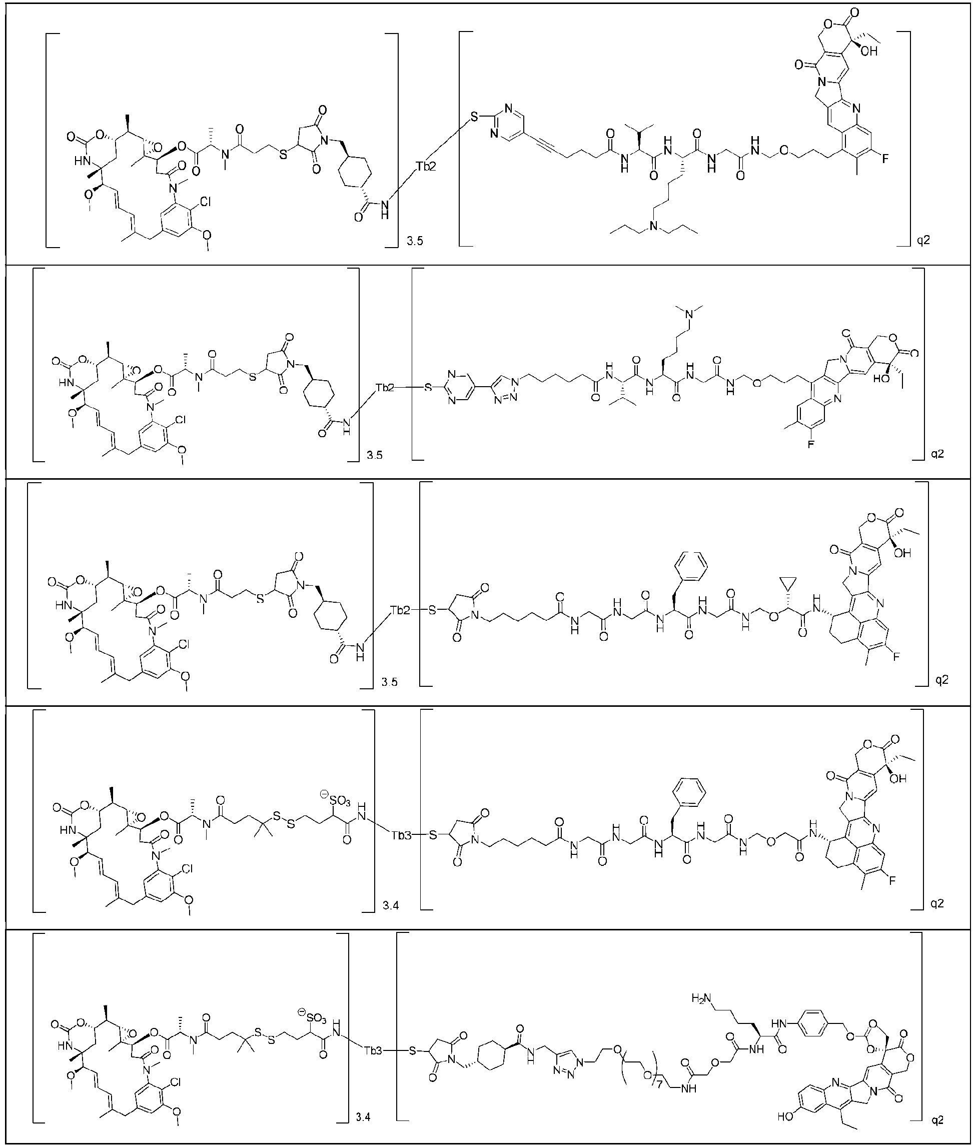

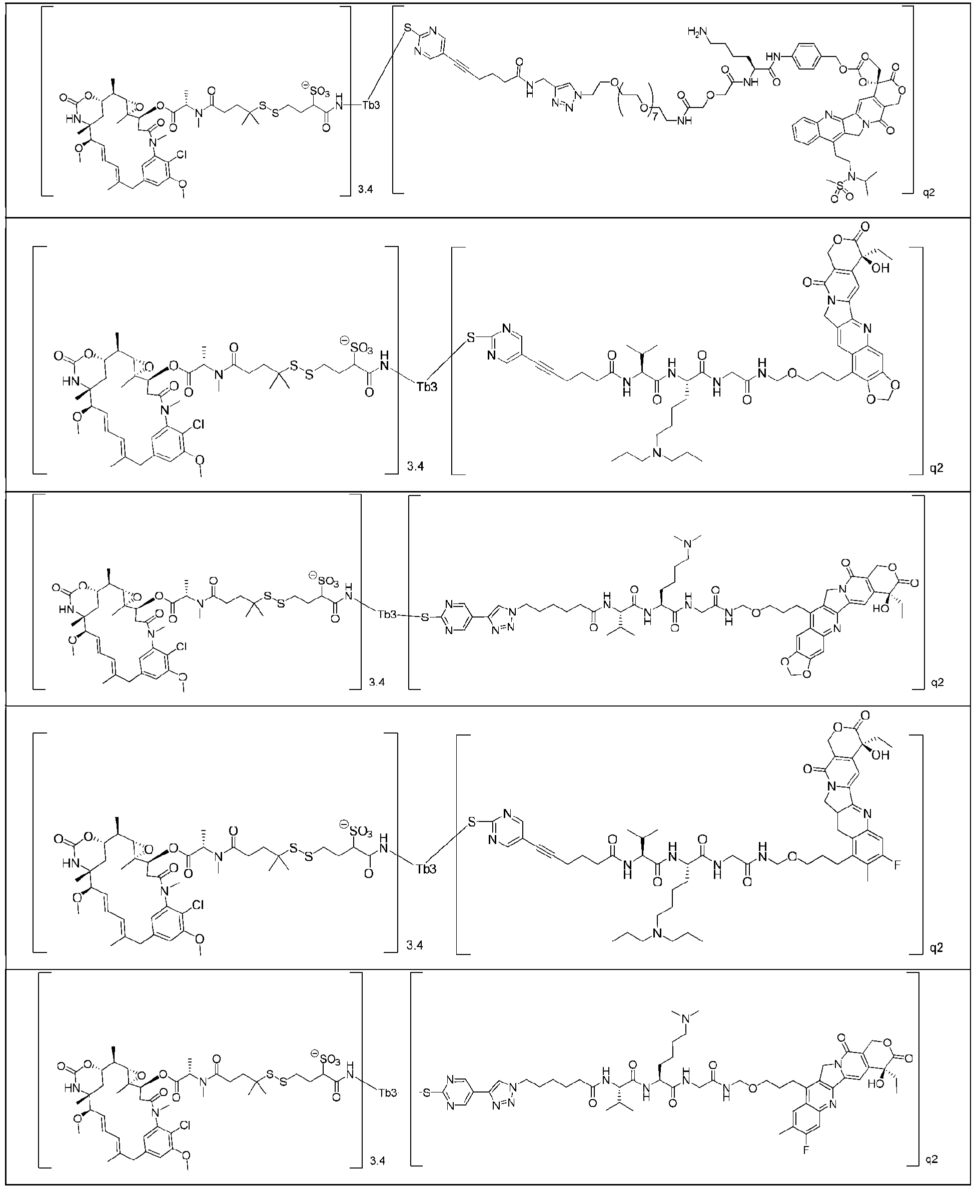

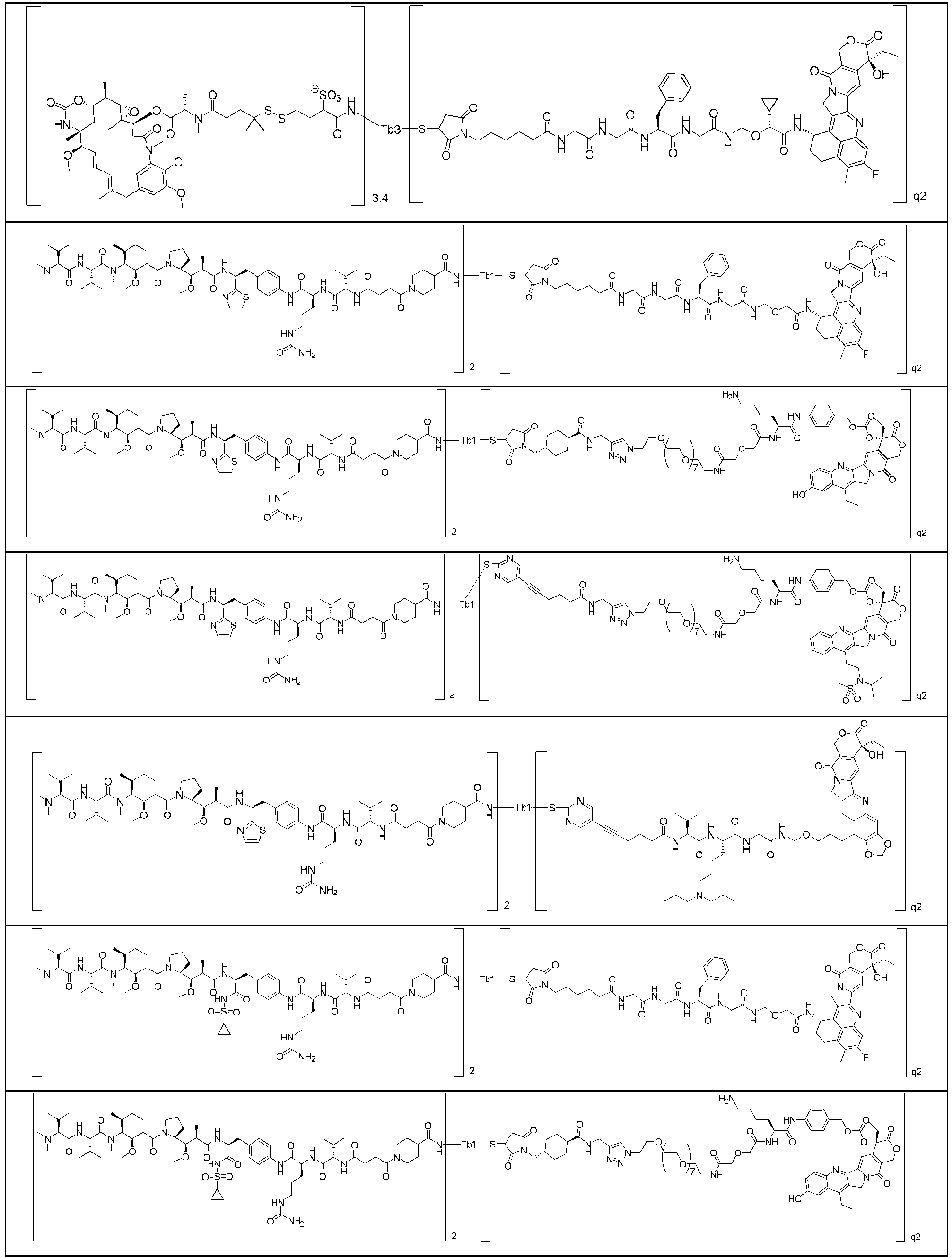

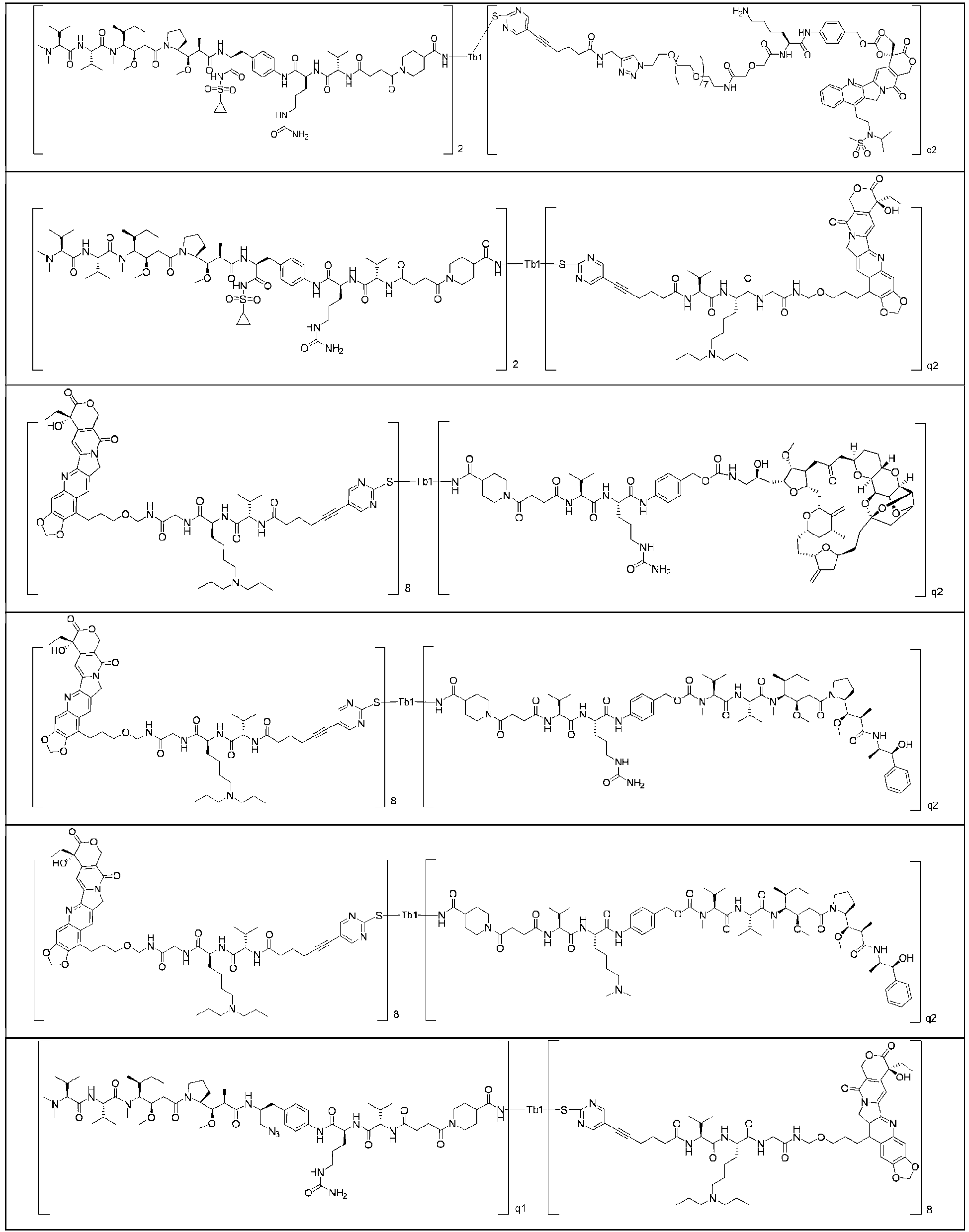

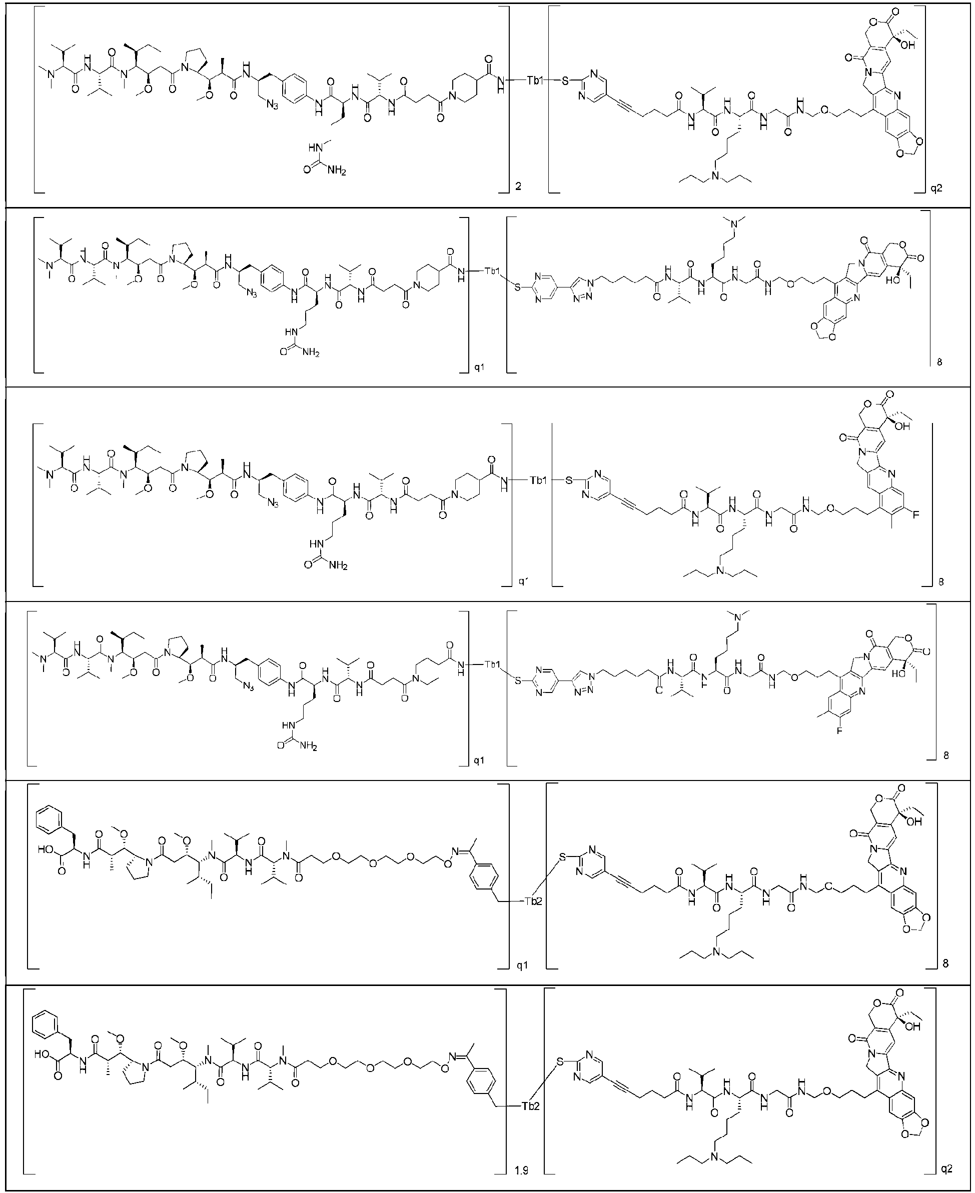

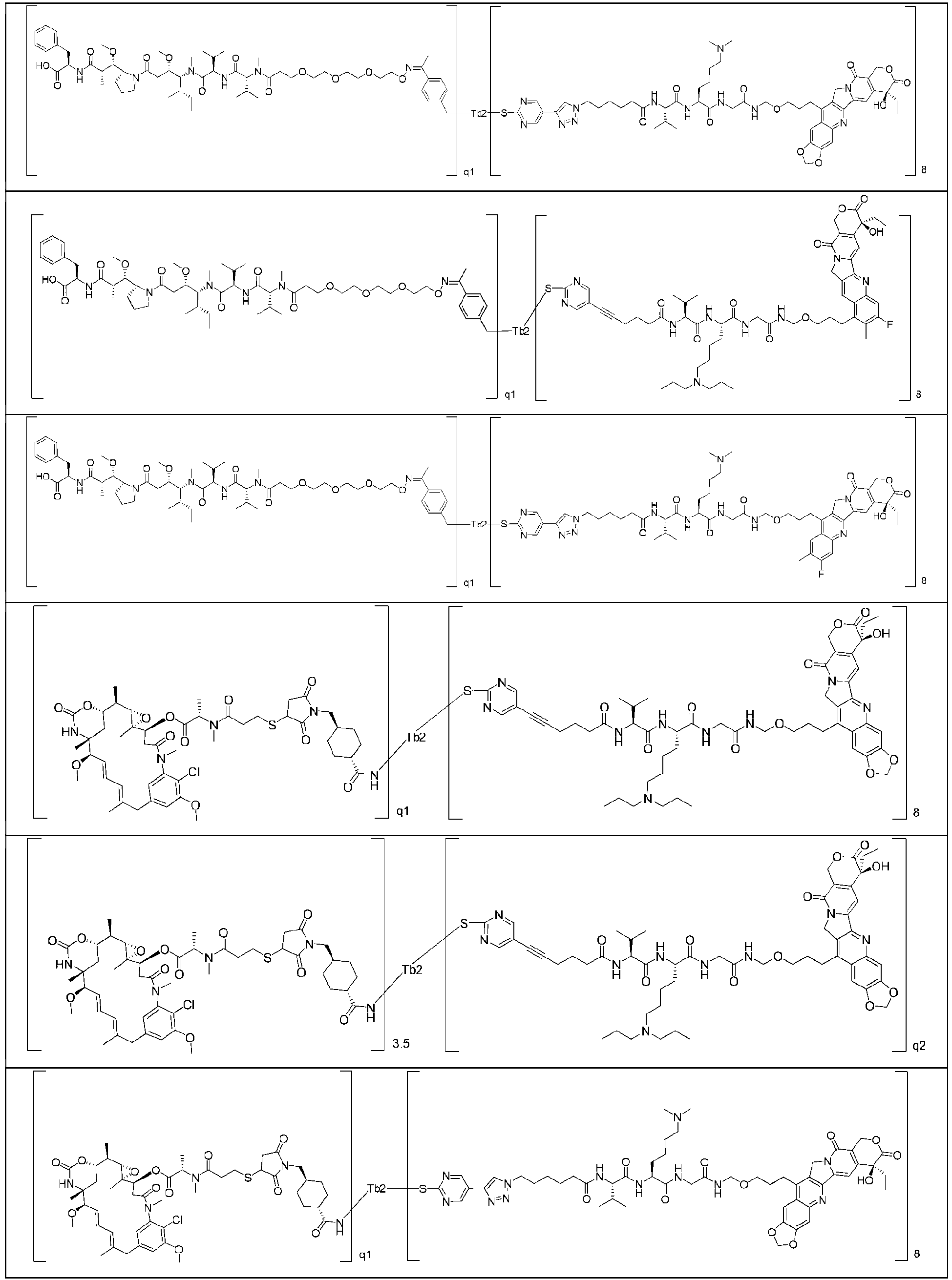

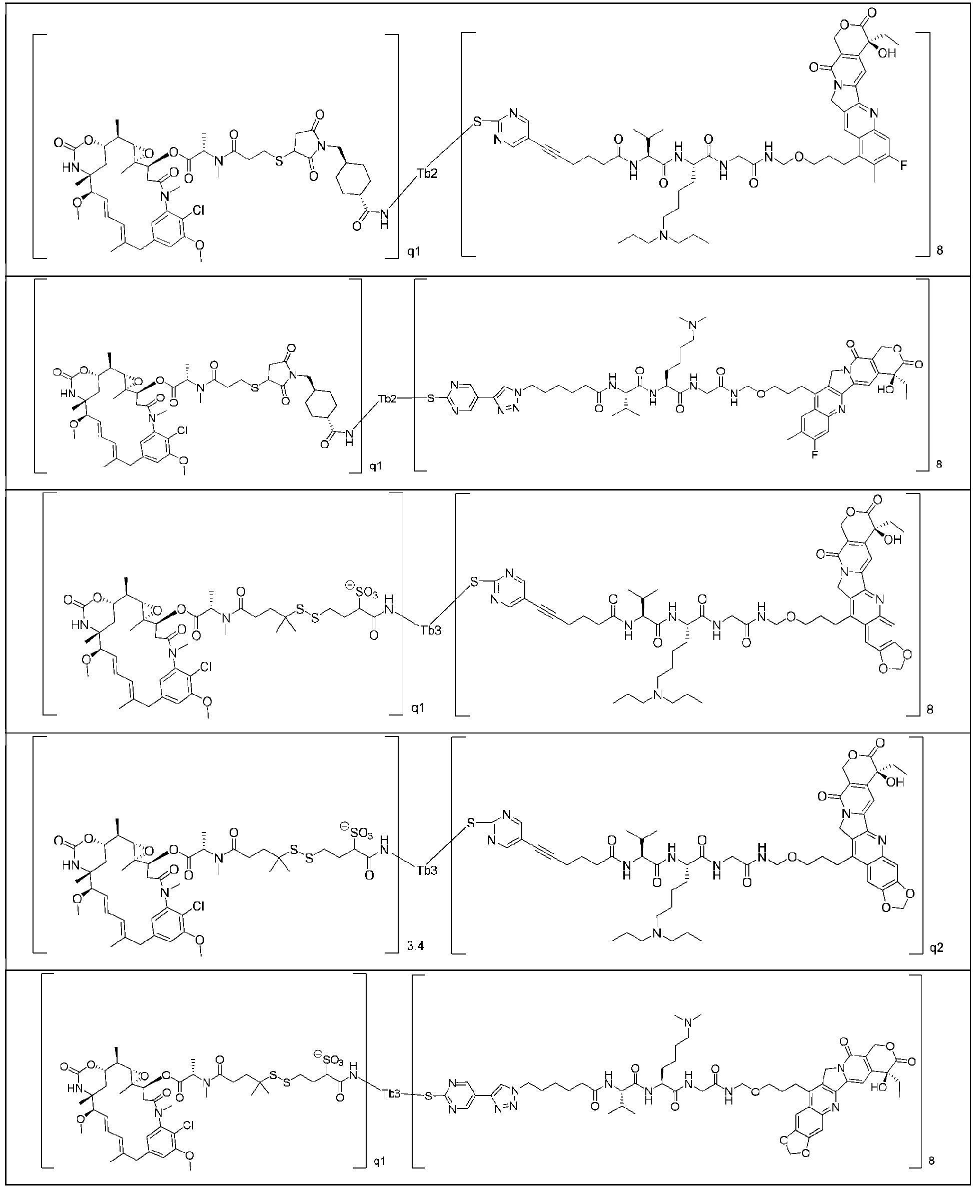

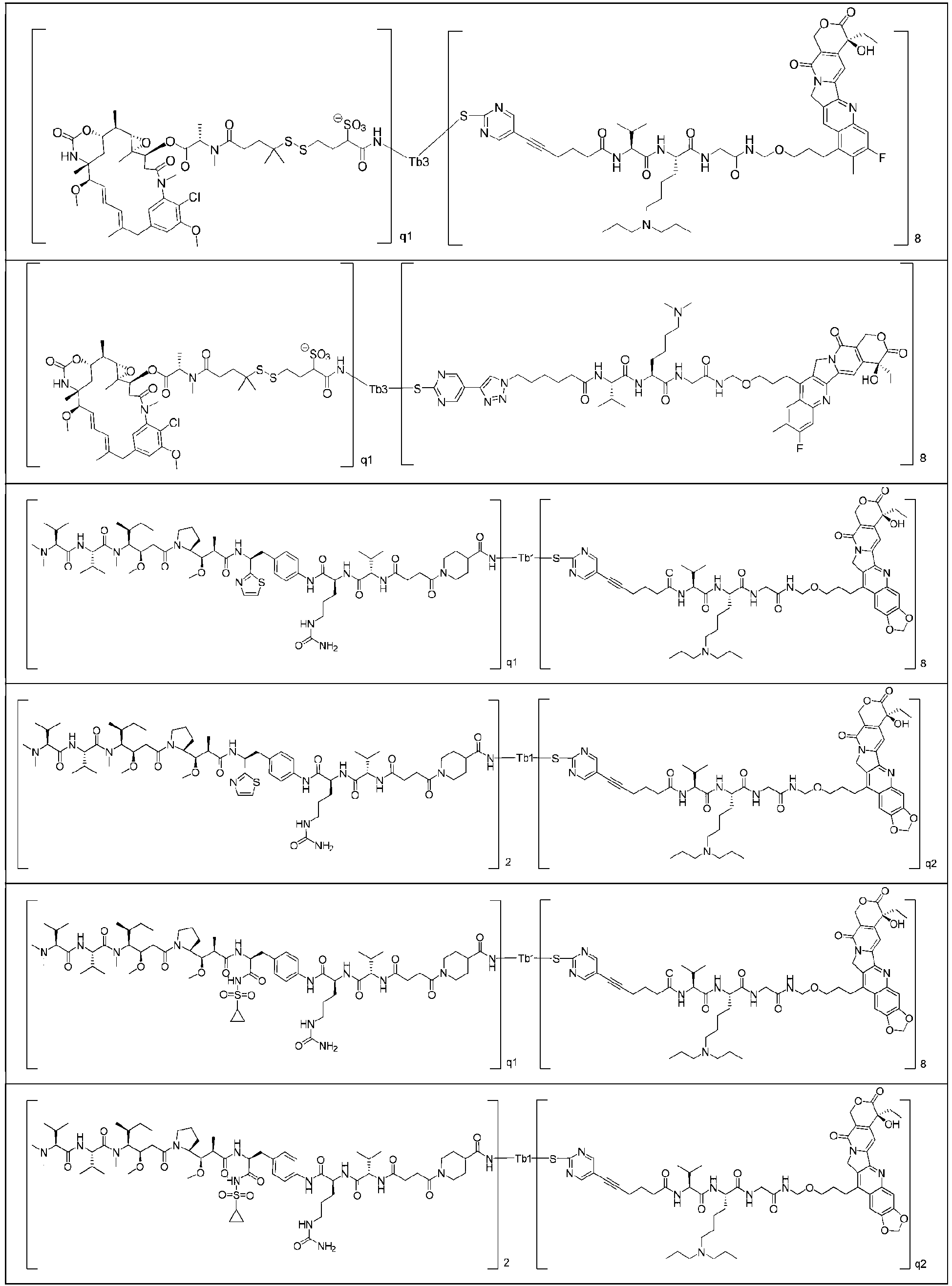

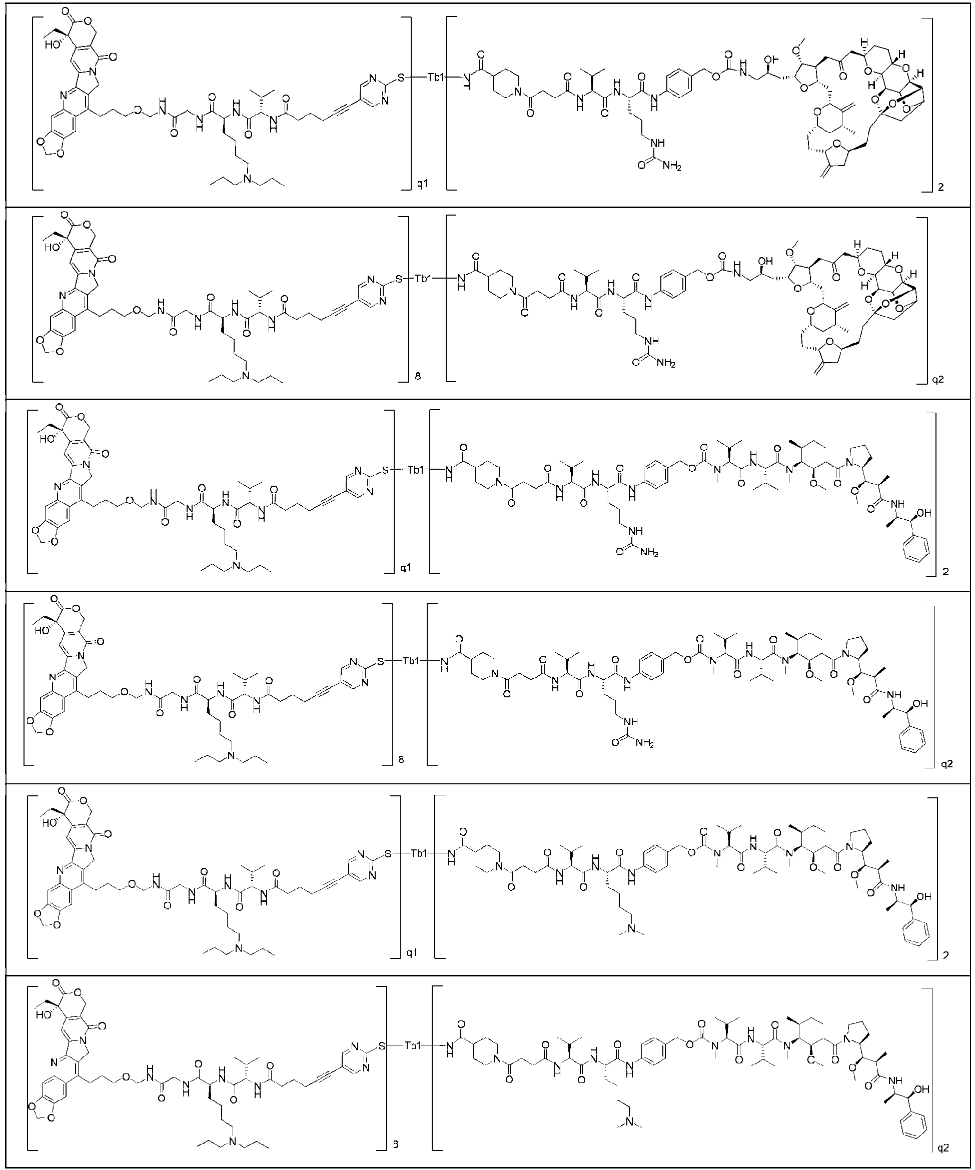

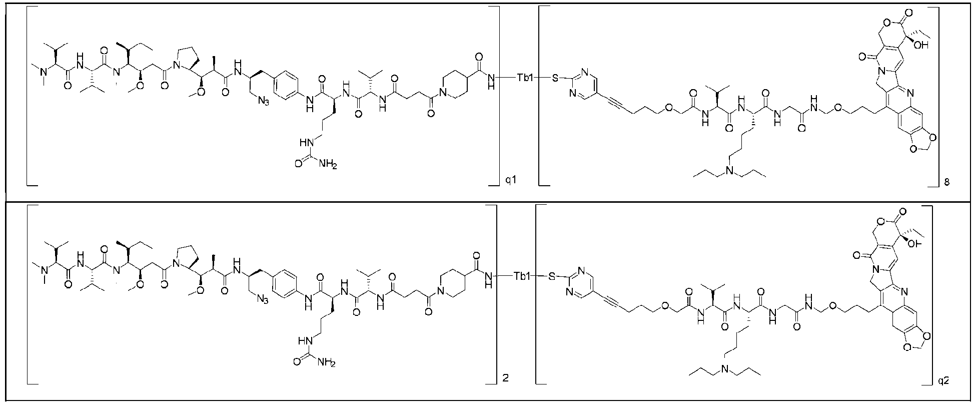

wherein q1 and q2 are each independently selected from any numerical value between 1 and 10;
Tb1, Tb2, and Tb3 are each independently an antibody or an antigen-binding fragment thereof.

In some embodiments, q1 is selected from: 1.0-6.0, 1.2-5.5, 1.4-5, 1.5-4.5, 1.6-4.0, 1.7-3.5, 1.8-3.0, 1.8-2.5, 1.8-2.4, 1.8-2.3, 1.9-2.2, and 2.0; q2 is selected from: 4.0-10, 4.0-9.5, 5.0-9.0, 6.0-8.8, 7.0-8.6, 7.5-8.5, 7.6-8.4, 7.7-8.3, 7.8-8.2, 7.9-8.1, and 8.0.

In some embodiments, Tb1 and Tb2 are anti-Her 2 antibodies or antigen-binding fragments thereof, such as anbenitamab, coprelotamab, disitamab, gancotamab, margetuximab, pertuzumab, timigutuzumab, zanidatamab, trastuzumab, pertuzumab, or antigen-binding fragments thereof.

In some embodiments, Tb1 is trastuzumab, pertuzumab, or an antigen-binding fragment thereof.

In some embodiments, Tb2 is trastuzumab or an antigen-binding fragment thereof.

In some embodiments, Tb3 is an anti-FRα antibody or an antigen-binding fragment thereof.

In some embodiments, Tb3 is mirvetuximab or an antigen-binding fragment thereof.

In some embodiments, the antibody-drug conjugate is selected from the following:

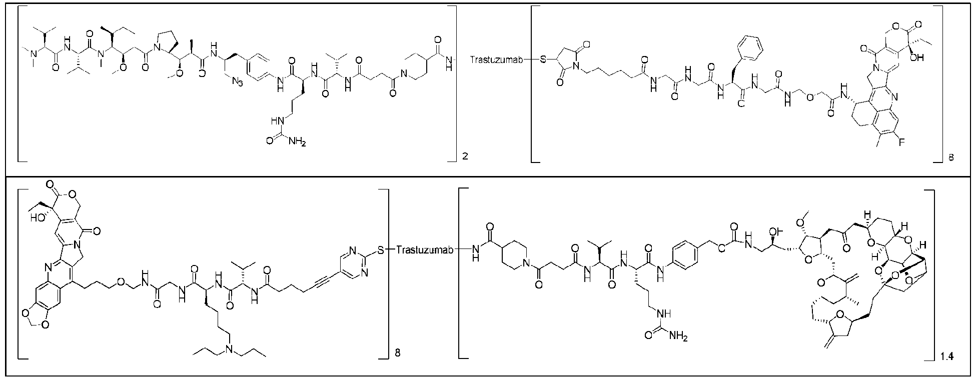

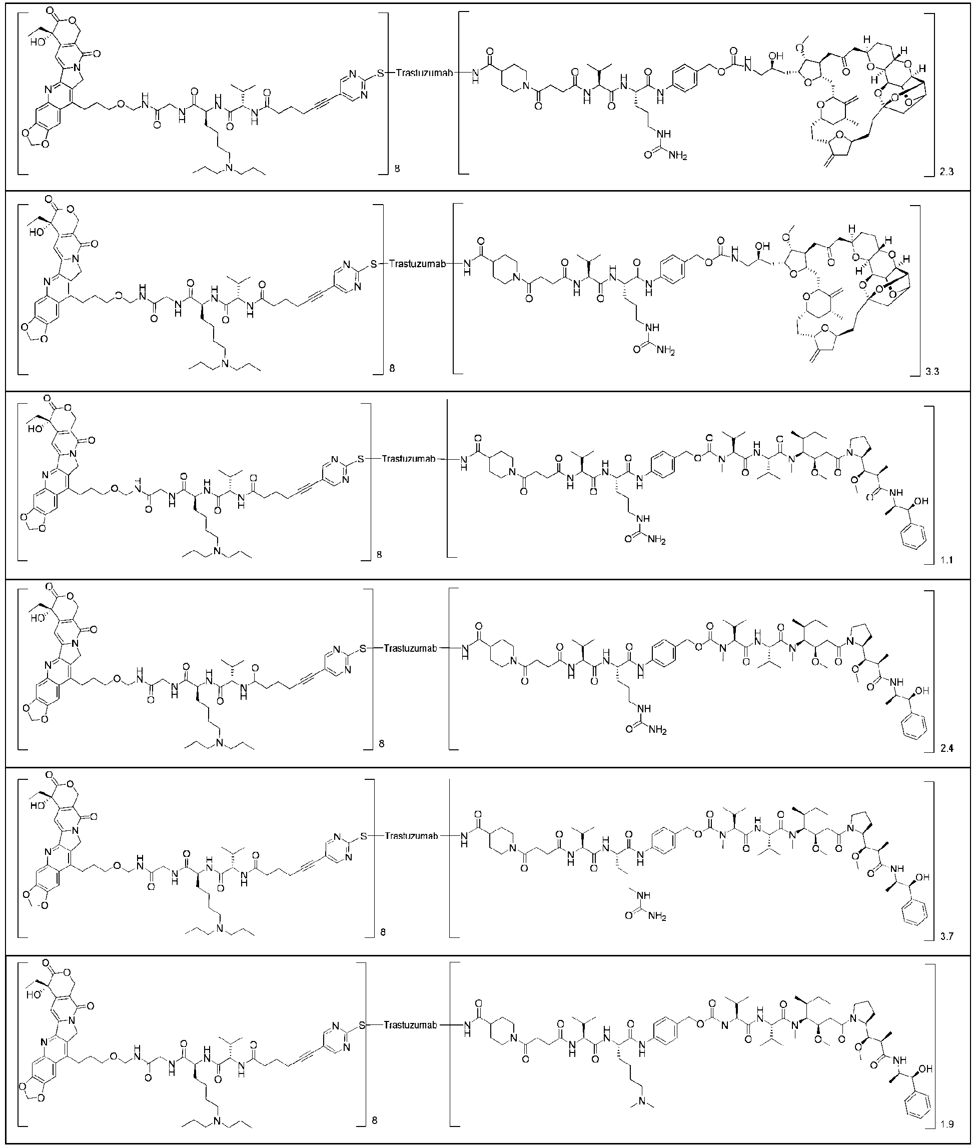

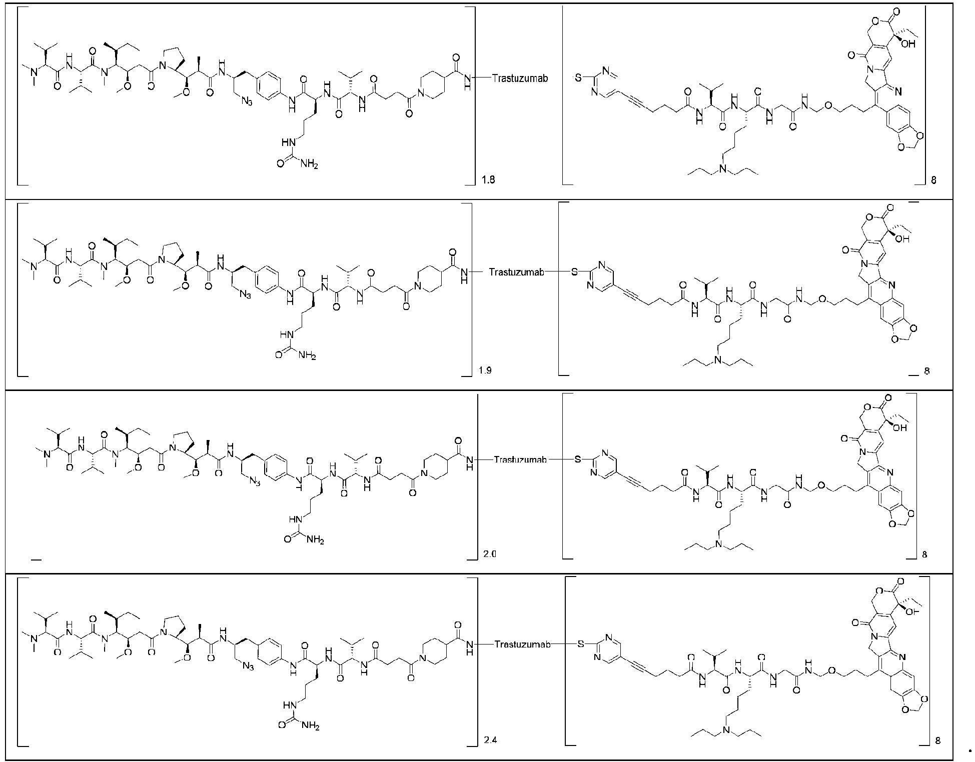

In a second aspect of the present disclosure, the present disclosure provides a toxin-linker represented by formula (IV),

L'-D IV

or a stereoisomer of the toxin-linker, a prodrug thereof, a pharmaceutically acceptable salt thereof, or a pharmaceutically acceptable solvate thereof,
wherein L' is a linker precursor, and D is as defined in any one of the embodiments of the present disclosure. In some embodiments, provided is a toxin-linker represented by formula (V),

   **Lg-L₁-L₂-L₃-L₄-D** V
or a stereoisomer of the toxin-linker, a prodrug thereof, a pharmaceutically acceptable salt thereof, or a pharmaceutically acceptable solvate thereof,
wherein:
   Lg is a leaving group when reacting with an antibody; L₂, L₃, L₄, and D are as defined in any one of the embodiments of the present disclosure.

In some embodiments, Lg is selected from halogen, sulfonyl, tertiary amine salt groups (Me₃N⁺ and Et₃N⁺), diazonium salt groups, -OMs, MeSO₂-, CF₃SO₃-, p-toluenesulfonyl, 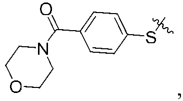 and phenoxy, wherein the phenoxy is substituted with one or more halogens.

In some embodiments, Lg is selected from F, Cl, Br, MeSO₂-, and pentafluorophenoxy; more preferably, Lg is MeSO₂- or pentafluorophenoxy.

In some embodiments, Lg is selected from F, Cl, Br, MeSO₂-, and pentafluorophenoxy; more preferably, Lg is MeSO₂-.

In some embodiments, L₁ and Lg-L₁ comprise the following features:
(1) when L₁ is 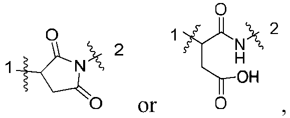 Lg-L₁ is 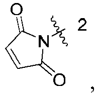 and position 2 is attached to L₂;
(2) when L₁ is 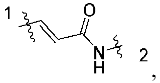 Lg-L₁ is 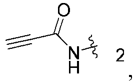 and position 2 is attached to L₂;
(3) when L₁ is 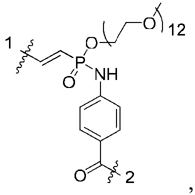 Lg-L₁ is 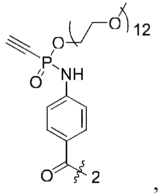 and position 2 is attached to L₂;
(4) when L₁ is 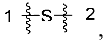 Lg-L₁ is 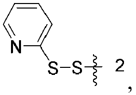 and position 2 is attached to L₂;
(5) when L₁ is 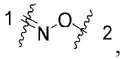 Lg-L₁ is 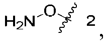 and position 2 is attached to L₂;
(6) when L₁ is 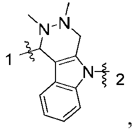 Lg-L₁ is 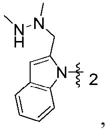 and position 2 is attached to L₂;
(7) when L₁ is 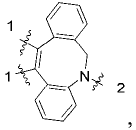 Lg-L₁ is 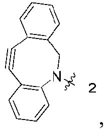 and position 2 is attached to L₂;
(8) when L₁ is 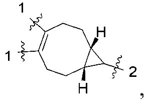 Lg-L₁ is 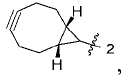 and position 2 is attached to L₂;
(9) when L₁ is 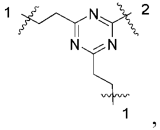 Lg-L₁ is 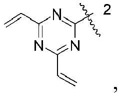 and position 2 is attached to L₂;
(10) when L₁ is 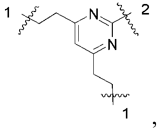 Lg-L₁ is 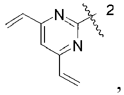 and position 2 is attached to L₂;
(11) when L₁ is 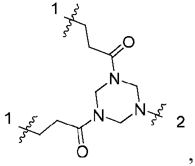 Lg-L₁ is 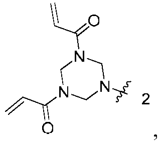 and position 2 is attached to L₂; L₂, L₃, L₄, and D are as defined in any one of the embodiments of the present disclosure.

In some embodiments, when L₁ is as defined in any one of the embodiments of the present disclosure and is not 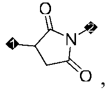 Lg is a leaving group when reacting with an antibody; preferably, Lg is selected from halogen, sulfonyl, tertiary amine salt groups (Me₃N⁺ and Et₃N⁺), diazonium salt groups, -OMs, MeSO₂-, CF₃SO₃-, and p-toluenesulfonyl; more preferably, Lg is selected from F, Cl, Br, and MeSO₂-; particularly preferably, Lg is MeSO₂-; L₂, L₃, L₄, and D are as defined in any one of the embodiments of the present disclosure;
when L₁ is 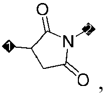 Lg-L₁ is 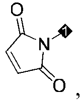 and L₂, L₃, L₄, and D are as defined in any one of the embodiments of the present disclosure.

In some embodiments, the L'-D toxin-linker is selected from the following:

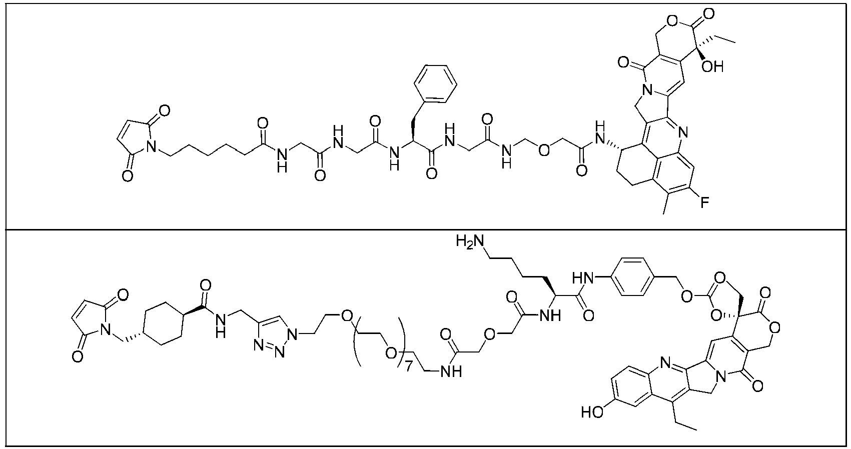

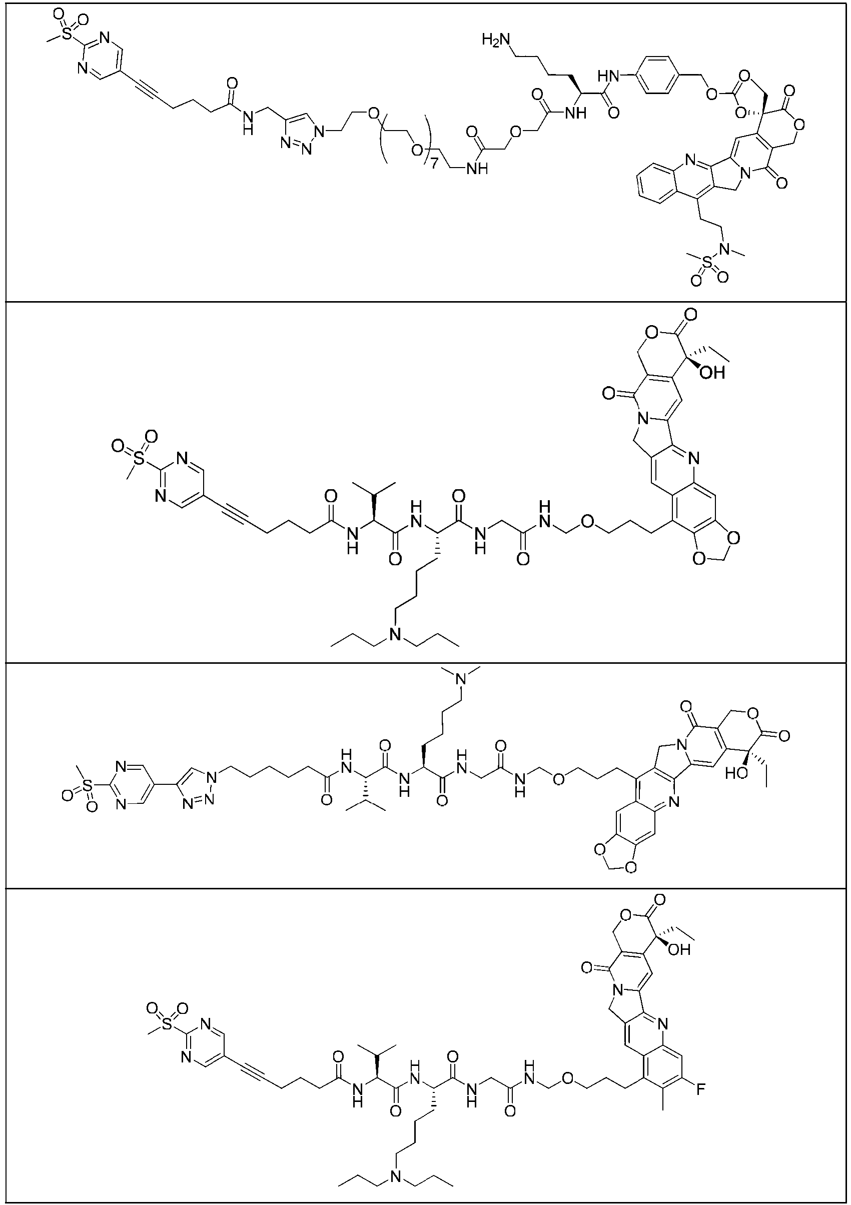

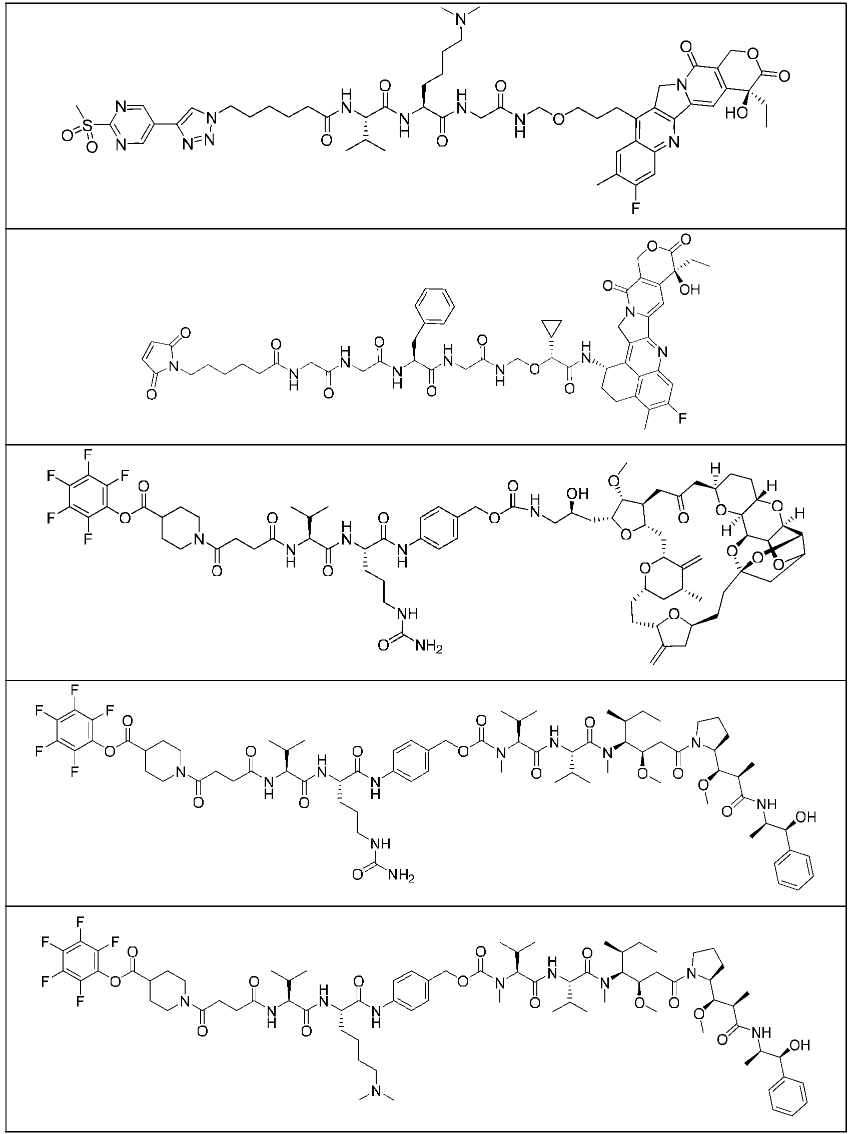

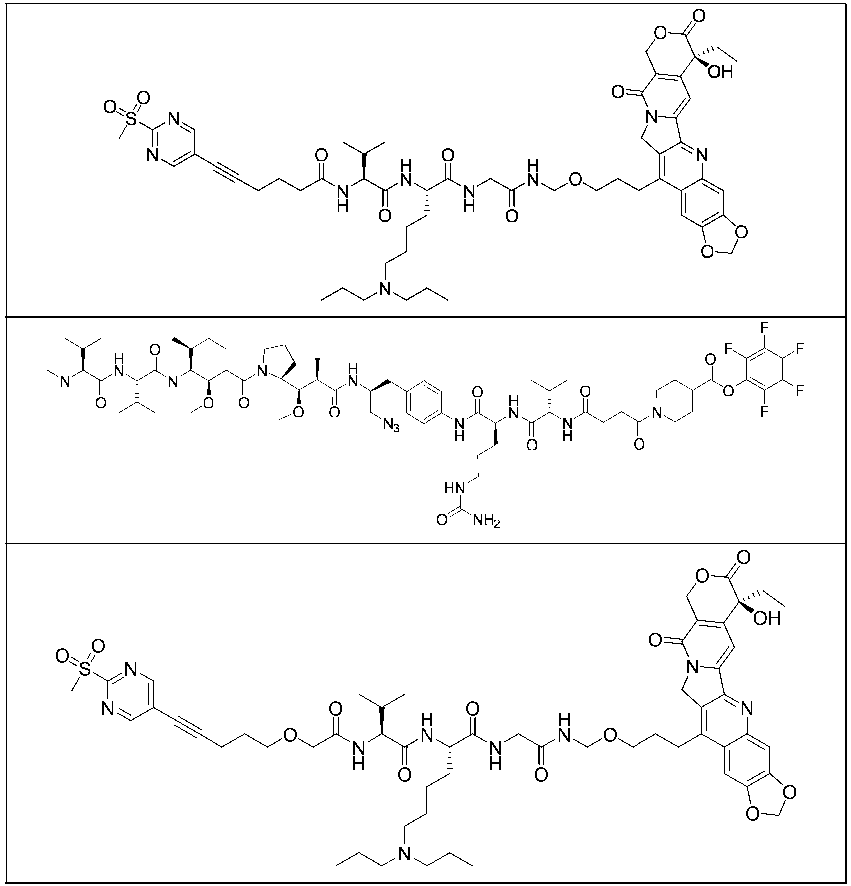

In a third aspect, the present disclosure provides a method for preparing an antibody-drug conjugate represented by formula (I), comprising:
subjecting A to a conjugation reaction with a toxin-linker represented by formula (IV), wherein A, L', and D are as defined in any one of the embodiments of the present disclosure.

Specifically, the method comprises a step of subjecting A to a conjugation reaction with the toxin-linker represented by formula (IV) in a solvent to form a C-S bond.

In some embodiments, a molar ratio of A to the toxin-linker represented by formula (IV) is 1:(1-20), such as 1:2-16, 1:4-16, 1:6-16, 1:8-16, or 1:10-16.

In some embodiments, the conjugation reaction is performed in water and/or an organic solvent.

In some embodiments, the organic solvent is selected from N,N-dimethylformamide, N,N-dimethylacetamide, dimethyl sulfoxide, N-methylpyrrolidone, nitriles (e.g., acetonitrile), alcohols (e.g., methanol and ethanol), and any combination thereof.

In some embodiments, the method further comprises a step of purifying the conjugate product.

In some embodiments, the conjugate product is purified by chromatography.

In some embodiments, the chromatography comprises one or more of ion exchange chromatography, hydrophobic chromatography, reversed-phase chromatography, or affinity chromatography.

The present disclosure provides a population of antibody-drug conjugates, comprising or consisting of the antibody-drug conjugate or the stereoisomer thereof, the prodrug thereof, the pharmaceutically acceptable salt thereof, the tautomer thereof, or the pharmaceutically acceptable solvate thereof described above, wherein the antibody-drug conjugates have one, two, or more q values.

In some embodiments, when one q value of the antibody-drug conjugates dominates (e.g., 80%, 85%, 90%, 95%, 95%, 97%, 98%, or 99%) in the population of antibody-drug conjugates, the q value is close to the average DAR. In some embodiments, when there are only antibody-drug conjugates of one q value in the population of antibody-drug conjugates, the q value is equal to the average DAR.

In some embodiments, when the antibody-drug conjugates in the population of antibody-drug conjugates have two or more q values, the proportion of antibody-drug conjugates of a specific q value among all antibody-drug conjugates in the composition is greater than 50%, 60%, 70%, 80%, 90%, 95%, 96%, 97%, 98%, or 99%.

In some embodiments, the average drug-to-antibody ratio (average DAR) of the population of antibody-drug conjugates is selected from integers and decimals of 1-16 (preferably 1-10).

In some embodiments, the average drug-to-antibody ratio (average DAR) of the population of antibody-drug conjugates is selected from 1-1.5, 1.5-2.5, 2.5-3.5, 3.5-4.5, 4.5-5.5, 5.5-6.5, 6.5-7.5, and 7.5-8.5.

In some embodiments, the average drug-to-antibody ratio (average DAR) of the population of antibody-drug conjugates is selected from 1.5-2.5, 3.5-4.5, 5.5-6.5, and 7.5-8.5.

In some embodiments, the average drug-to-antibody ratio (average DAR) of the population of antibody-drug conjugates is selected from about 2.0, 4.0, 6.0, and 8.0.

In some embodiments, the average drug-to-antibody ratio (average DAR) of the population of antibody-drug conjugates is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, 9, and 9.7.

In some embodiments, the population of antibody-drug conjugates comprises ADCs with a distribution of DARs of 1 to 8, such as 1.5, 2, 4, 6, and 8 (i.e., drug-loaded species of 1.5, 2, 4, 6, and 8). Notably, degradation products may be produced, such that the mixture may also comprise DARs of 1, 3, 5, and 7. In addition, the population of antibody-drug conjugates may also have an average DAR greater than 8. The antibody-drug conjugate is produced by the reduction of an interchain disulfide followed by conjugation. In some embodiments, the antibody-drug conjugate comprises the following two: an antibody-drug conjugate with a DAR of 4 or less (i.e., drug-loaded species of 4 or less) and an antibody-drug conjugate with a DAR of 6 or more (i.e., drug-loaded species of 6 or more).

The present disclosure provides a pharmaceutical composition comprising the aforementioned antibody-drug conjugate or the stereoisomer of the antibody-drug conjugate, the prodrug thereof, the pharmaceutically acceptable salt thereof, or the pharmaceutically acceptable solvate thereof; or the aforementioned population of antibody-drug conjugates and optionally one or more pharmaceutical adjuvants.

The present disclosure provides use of the aforementioned antibody-drug conjugate or the stereoisomer of the antibody-drug conjugate, the prodrug thereof, the pharmaceutically acceptable salt thereof, or the pharmaceutically acceptable solvate thereof; or the aforementioned population of antibody-drug conjugates; or the aforementioned pharmaceutical composition as a medicament (i.e., for therapeutic use).

The present disclosure provides use of the aforementioned antibody-drug conjugate or the stereoisomer of the antibody-drug conjugate, the prodrug thereof, the pharmaceutically acceptable salt thereof, or the pharmaceutically acceptable solvate thereof; or the aforementioned population of antibody-drug conjugates; or the aforementioned pharmaceutical composition in the manufacture of a medicament for treating and/or preventing a disease associated with abnormal cell activity (e.g., a cancer disease); or for treating and/or preventing a disease associated with abnormal cell activity (e.g., a cancer disease).

The present disclosure provides a method for preventing and/or treating a disease associated with abnormal cell activity (e.g., a cancer disease), comprising: administering to an individual in need thereof a prophylactically and/or therapeutically effective amount of the aforementioned antibody-drug conjugate or the stereoisomer of the antibody-drug conjugate, the prodrug thereof, the pharmaceutically acceptable salt thereof, or the pharmaceutically acceptable solvate thereof; or the aforementioned population of antibody-drug conjugates; or the aforementioned pharmaceutical composition.

In some embodiments, the cancer disease is a cancer disease associated with a target of the antibody or the antigen-binding fragment thereof in the aforementioned antibody-drug conjugate.

In some embodiments, the cancer disease is a solid tumor or a hematological tumor.

In some embodiments, the cancer disease is selected from esophageal cancer (e.g., esophageal adenocarcinoma and esophageal squamous cell carcinoma), brain tumor, lung cancer (e.g., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer (colon cancer or rectal cancer), liver cancer, kidney cancer, urothelial cancer, solid tumors, non-Hodgkin lymphoma, central nervous system tumors (e.g., neuroglioma, glioblastoma multiforme, glioma, or sarcoma), prostate cancer, and thyroid cancer.

In the present disclosure, unless otherwise indicated, scientific and technical terms used in the present disclosure have the meanings generally understood by those skilled in the art. In addition, the laboratory procedures of cell culture, molecular genetics, nucleic acid chemistry, and immunology used in the present disclosure are the conventional procedures widely used in the corresponding fields. Moreover, in order to better understand the present disclosure, the definitions and explanations of related terms are provided below.

An example of the term "pharmaceutically acceptable salt" as used herein is an organic acid addition salt formed of an organic acid that forms a pharmaceutically acceptable anion. See "Handbook of Pharmaceutical Salts: Properties, Selection, and Use" by Stahl and Wermuth (Wiley-VCH, 2002) for a review of salts, including but not limited to formate, acetate, propionate, benzoate, maleate, fumarate, succinate, tartrate, citrate, ascorbate, α-ketoglutarate, α-glycerophosphate, alkylsulfonate, or arylsulfonate; preferably, the alkylsulfonate is methylsulfonate or ethylsulfonate; the arylsulfonate is benzene sulfonate or p-toluenesulfonate. Suitable inorganic salts may also be formed, including but not limited to hydrochloride, hydrobromide, hydroiodide, nitrate, bicarbonate and carbonate, sulfate, or phosphate, etc.

Pharmaceutically acceptable salts may be obtained using standard procedures well known in the art, for example, by reacting a sufficient amount of an alkaline compound with a suitable acid that provides a pharmaceutically acceptable anion.

As used herein, the term "stereoisomer" refers to an isomer formed due to at least one asymmetric center. In compounds with one or more (e.g., one, two, three, or four) asymmetric centers, racemic mixtures, single enantiomers, mixtures of diastereomers, and individual diastereomers may be produced. Certain individual molecules may also exist as geometric isomers (cis/trans). Similarly, the compounds of the present invention may exist as mixtures of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present disclosure encompasses all such isomers or mixtures thereof in any proportion (e.g., 60%, 65%, 70%, 75%, 80%, 85%, 90%, 95%, 96%, 97%, 98%, or 99%).

A solid line (-), a solid wedge, or a dashed wedge may be used in the present disclosure to depict carbon-carbon bonds of the compounds of the present invention. Using the solid line to depict a bond that is bonded to an asymmetric carbon atom is intended to indicate that all possible stereoisomers (e.g., particular enantiomers, and racemic mixtures) at the carbon atom are included. Using the solid or dashed wedge to depict a bond that is bonded to an asymmetric carbon atom is intended to indicate that the stereoisomers shown are present. When present in a racemic mixture, the solid and dashed wedges are used to define the relative stereochemistry rather than the absolute stereochemistry. Unless otherwise indicated, the compounds of the present invention are intended to be present in the form of stereoisomers (including cis and trans isomers, optical isomers (e.g., R and S enantiomers), diastereomers, geometric isomers, rotamers, conformers, atropisomers, and mixtures thereof). The compounds of the present invention may exhibit one or more types of isomerism and consist of mixtures thereof (e.g., racemic mixtures and diastereomer pairs).

The present invention also includes all pharmaceutically acceptable isotopic compounds, which are identical to the compounds of the present invention, except that one or more atoms are replaced by an atom that has the same atomic number but whose atomic mass or mass number is different from the atomic mass or mass number predominant in nature. Examples of isotopes suitable for incorporation into the compounds of the present invention include, but are not limited to, isotopes of hydrogen (e.g., ²H and ³H); isotopes of carbon (e.g., ¹¹C, ¹³C, and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S).

The compounds of the present disclosure may be present in the form of solvates (preferably hydrates), and the compounds of the present disclosure comprise a polar solvent as a structural element of the crystal lattices of the compounds, particularly, for example, water, methanol, or ethanol. The amount of the polar solvent, particularly water, may be present in a stoichiometric or non-stoichiometric ratio.

Also included within the scope of the present invention are metabolites of the compounds of the present invention, i.e., substances formed *in vivo* upon administration of the compounds of the present invention. Such products may result from, for example, oxidation, reduction, hydrolysis, amidation, deamidation, esterification, defatting, enzymatic digestion, etc. of the administered compounds. Thus, the present invention includes metabolites of the compounds of the present invention, including compounds made by the process of contacting the compounds of the present invention with a mammal for a time sufficient to produce metabolites thereof. The present disclosure further comprises, within its scope, prodrugs of the compounds of the present invention. Generally, such prodrugs will be functional group derivatives of the compounds which are readily converted into desired therapeutically active compounds *in vivo.* Thus, in these cases, the term "administering", "administered", or "administration" as used in the treatment method of the present disclosure shall include the treatment of various diseases or disorders with prodrug forms of one or more of the claimed compounds, but the prodrug forms are converted into the compounds described above *in vivo* upon administration to an individual. Conventional methods for selecting and preparing suitable prodrug derivatives are described in, for example, "Design of Prodrug", ed. H. Bundgaard, Elsevier, 1985.

In the present disclosure, the pharmaceutical adjuvants refer to excipients and additives used in the production of a pharmaceutical product and in the formulation of a pharmaceutical formula, and refer to substances, other than the active ingredient, which have been rationally evaluated for safety and are contained in a pharmaceutical formulation. In addition to shaping, serving as carriers, and enhancing stability, pharmaceutical adjuvants also have such important functions as solubilization, aiding dissolution, sustaining or controlling release, etc. They are important ingredients that possibly affect the quality, safety, and effectiveness of pharmaceutical products. According to their origins, pharmaceutical adjuvants may be classified into natural products, semi-synthetic products, and total synthetic products. According to their effects and uses, pharmaceutical adjuvants may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesive agents, antioxidants, chelating agents, permeation promoters, pH regulators, buffers, plasticizers, surfactants, foaming agents, defoaming agents, thickeners, inclusion agents, humectants, absorbents, diluents, flocculants, deflocculants, filter aids, release retardants, etc.; according to their routes of administration, pharmaceutical adjuvants may be classified into pharmaceutical adjuvants for oral administration, injection, mucosal administration, transdermal or local administration, nasal or oral inhalation administration, ocular administration, etc. The same pharmaceutical adjuvant may be used in pharmaceutical formulations for different routes of administration, and may have different effects and uses.

The pharmaceutical composition may be prepared into various suitable dosage forms according to the route of administration, for example, tablets, capsules, granules, oral solutions, oral suspensions, oral emulsions, powders, tinctures, syrups, injections, suppositories, ointments, creams, pastes, ophthalmic formulations, pills, implants, aerosols, powder aerosols, sprays, etc. The pharmaceutical composition or suitable dosage form may comprise 0.01 mg to 1000 mg of a compound of the present disclosure or a pharmaceutically acceptable salt thereof or a conjugate thereof, suitably 0.1 mg to 800 mg, preferably 0.5-500 mg, preferably 0.5 to 350 mg, and particularly preferably 1-250 mg.

The pharmaceutical composition may be administered in the form of an injection, including an injectable liquid, a sterile powder for injection, and a concentrated solution for injection. The carriers and solvents that may be used include water, Ringer's solution, and isotonic sodium chloride solution. In addition, sterilized non-volatile oils may also be used as solvents or suspending media, such as monoglycerides or diglycerides. The pharmaceutical composition may be administered in the form of an infusion.

The term "treat", "treated", "treating", or "treatment" as used herein usually refers to acquiring needed pharmacological effects and/or physiological effects. In terms of fully or partially preventing a disease or a symptom thereof, the effect may be prophylactic; and/or in terms of partially or fully stabilizing or curing the disease and/or a side effect caused by the disease, the effect may be therapeutic. As used herein, "treat", "treating", or "treatment" encompasses any treatment to a disease in a patient, including (a) preventing a disease or a symptom in a patient susceptible to the disease or the symptom that has not yet been diagnosed; (b) inhibiting a symptom of a disease, i.e., arresting its progression; or (c) alleviating a symptom of a disease, i.e., causing regression of the disease or the symptom.

In the present disclosure, the term "individual" includes a human or non-human animal. Exemplary human individuals include human individuals with a disease (e.g., a disease described herein), which are referred to as patients, or normal individuals. In the present disclosure, the term "non-human animal" includes all vertebrates, such as non-mammals (e.g., birds, amphibians, and reptiles) and mammals, such as non-human primates, livestock, and/or domesticated animals (e.g., sheep, dogs, cats, cows, pigs, etc.).

In the present disclosure, the term "effective dose" refers to the amount of an antibody-drug conjugate, a toxin-linker, a compound, or a composition that can alleviate one or more symptoms of the disorder to be treated to some extent after administration.

In the present disclosure, the term "antibody-drug conjugate" or "ADC" refers to a substance obtained by attaching a bioactive compound fragment (a bioactive compound or drug molecule) to a moiety of an antibody or an antigen-binding fragment thereof. In some embodiments of the present disclosure, the bioactive compound fragment is attached to the targeting moiety via a linker. The linker can be cleaved in a particular environment (e.g., an intracellular low-pH environment) or under a particular action (e.g., the action of a lysosomal protease), so that the bioactive compound fragment is separated from the targeting moiety or the antibody or the antigen-binding fragment thereof. In some embodiments of the present disclosure, the linker comprises a cleavable or non-cleavable unit, such as a peptide or a disulfide bond. In some embodiments of the present disclosure, the bioactive compound fragment is directly attached to the targeting moiety or the antibody or the antigen-binding fragment thereof via a covalent bond that can be cleaved in a particular environment or under a particular action, so that the bioactive compound fragment is separated from the moiety of the antibody or the antigen-binding fragment thereof.

In the present disclosure, the term "drug" or "medicament" refers to a substance that inhibits or prevents cell functions and/or causes cell death or cell destruction.

In the present disclosure, the term "amino acid" refers to an organic compound containing basic amino and acidic carboxyl, consisting of an amino group (NH₂), carboxyl (COOH), and one side chain. In the present disclosure, the term "short peptide" refers to a peptide consisting of 2-10 amino acids and is also known as an oligopeptide. In the present disclosure, "position 1 is attached to A via a sulfur (S) atom" can be understood by those skilled in the art to mean that position 1 is attached to a sulfhydryl group contained in A after the disulfide bond is opened (for example, the disulfide bond may be opened by reducing the disulfide bond with the reducing agent TCEP, generating the sulfhydryl group -SH); that is, -S- is not an additional extraneous sulfur atom. For example, when position 1 of L₁ is attached to A via a sulfur (S) atom, it is -S- formed by attaching a sulfhydryl group contained in A after the disulfide bond is opened to position 1 of L₁ (e.g., 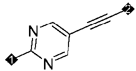 ).

In the present disclosure, "position 1 is attached to A via a nitrogen (N) atom" can be understood by those skilled in the art to mean that position 1 is attached to the amino residue of lysine in A; that is, -N- is not an additional extraneous nitrogen atom. For example, when position 1 of L₁ is attached to A via a nitrogen (N) atom, it is -N-formed by attaching an amino group contained in A to position 1 of L₁ (e.g., 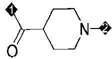 ).

In the present disclosure, the term "disulfhydryl bridging conjugation" can be understood by those skilled in the art to refer to a conjugation mode formed by first opening an interchain disulfide bond in the antibody to form two free cysteine residues and then simultaneously pairing with the two free cysteine residues. Since one antibody has only 4 interchain disulfide bonds, and 8 free cysteine residues are formed after all the interchain disulfide bonds are opened, the average DAR value of an ADC formed by disulfhydryl bridging conjugation is 0-4.

In the present disclosure, the term "linker" refers to a fragment that attaches a bioactive molecular fragment (drug molecule) to an antibody moiety. In this regard, a linker has a functional group that can form a bond with a functional group of an antibody or an antigen-binding fragment thereof before being attached to the antibody or the antigen-binding fragment thereof (i.e., a linker precursor).

In the present disclosure, " 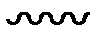 " represents the point of attachment of an attaching moiety.

In the present disclosure, the "known ADC molecule" refers to an "ADC" known in the art that has been approved by a pharmaceutical regulatory authority, and it also includes any ADC with potential therapeutic bioactivity in clinical practice or in research and development and academic research, and ADCs consisting of a commercially available antibody and a known toxin-linker, i.e., ADCs with well-defined administration doses. In the present disclosure, the term "bioactive molecular fragment" refers to a moiety (fragment or group) of an antibody-drug conjugate (or referred to as ADC) well known in the art, which is capable of forming a bioactive drug (e.g., a small molecule cytotoxic drug, which comprises the group that results from the loss of an atom or an atomic group) or a derivative thereof (e.g., a precursor thereof) after cleavage/degradation/enzyme digestion of a linker among tumor tissues or inside tumor cells. To avoid ambiguity, "drug" or "medicament" does not refer only to a "pharmaceutical product" that has been approved by a pharmaceutical regulatory authority, but also includes any compound with potential therapeutic bioactivity in clinical practice or in research and development and academic research.

In the present disclosure, the term "antibody" is interpreted in its broadest sense and includes intact monoclonal antibodies, polyclonal antibodies, and multispecific antibodies (e.g., bispecific antibodies) formed from at least two intact antibodies, so long as they possess the desired bioactivity. In the present disclosure, "antibody" and "immunoglobulin" are used interchangeably.

In the present disclosure, the term "monoclonal antibody" refers to an antibody from a population of substantially homogeneous antibodies, i.e., the individual antibodies constituting the population are identical except for a small number of natural mutations that may be present. Monoclonal antibodies have high specificity for one determinant (epitope) of an antigen, while polyclonal antibodies in contrast thereto comprise different antibodies against different determinants (epitopes). In addition to specificity, another advantage of monoclonal antibodies is that they can be synthesized without contamination by other antibodies. The modifier "monoclonal" used herein indicates that the antibody is characterized as being from a population of substantially homogeneous antibodies, and should not be construed as requiring a particular preparation method.

In some embodiments of the present disclosure, monoclonal antibodies also particularly include chimeric antibodies; that is, a portion of the heavy chain and/or light chain is identical or homologous to a type, a class, or a subclass of antibodies, and the remainder is identical or homologous to another type, another class, or another subclass of antibodies, so long as they possess the desired bioactivity (see, e.g., US 4,816,567; and Morrison et al., 1984, PNAS, 81: 6851-6855). Chimeric antibodies that may be used in the present disclosure include primatized antibodies comprising variable region antigen-binding sequences from non-human primates (e.g., ancient monkeys, chimpanzees, etc.) and human constant region sequences.

The term "antibody fragment" refers to a portion of an antibody, preferably an antigen-binding region or a variable region. Examples of antibody fragments include Fab, Fab', F(ab')₂, Fd, Fv, dAb, complementarity determining region fragments, diabodies, linear antibodies, and single-chain antibody molecules.

In the present disclosure, although in most cases, amino acid substitutions in antibodies are L-amino acid substitutions, they are not limited thereto. In some embodiments, an antibody peptide chain may comprise one or more D-amino acids. Peptides comprising D-amino acids are more stable and less susceptible to degradation in the oral cavity, intestine, or plasma than peptides comprising only L-amino acids.

In the present disclosure, the term "q value" refers to the number of conjugated toxin-linkers on an antibody, and it may be selected from any integer of 1-20; the terms "q1" and "q2" refer to the drug-to-antibody ratio in the antibody-drug conjugate, i.e., the DAR value; for example, q1 and q2 are preferably integers or decimals of 1-10, and examples thereof include, but are not limited to: q1 being selected from: 1.0-6.0, 1.2-5.5, 1.4-5, 1.5-4.5, 1.6-4.0, 1.7-3.5, 1.8-3.0, 1.8-2.5, 1.8-2.4, 1.8-2.3, 1.9-2.2, and 2.0; and q2 being selected from: 4.0-10, 4.0-9.5, 5.0-9.0, 6.0-8.8, 7.0-8.6, 7.5-8.5, 7.6-8.4, 7.7-8.3, 7.8-8.2, 7.9-8.1, and 8.0.

In the present disclosure, unless otherwise expressly indicated, the description "each ... be independently selected from" and "... be each independently selected from" are used interchangeably throughout the present disclosure and should be understood in a broad sense, and it may mean that specific items expressed by the same or different symbols in different groups do not affect each other, or that specific items expressed by the same or different symbols in the same group do not affect each other.

In the present disclosure, the term "substitution" means that one or more (e.g., one, two, three, or four) hydrogen atoms on a specified atom are replaced with a selection from the designated group, with the proviso that the normal valence of the atom specified under the present circumstances is not exceeded and that the substitution results in a stable compound. A combination of substituents and/or variables is permissible only if the combination results in a stable compound.

If a substituent is described as "optionally substituted with...", the substituent may be (1) unsubstituted or (2) substituted. If a carbon of a substituent is described as being optionally substituted with one or more of a list of substituents, then one or more hydrogen atoms on the carbon (to the extent of any hydrogen atoms present) may be replaced individually and/or together with an independently selected optional substituent. If a nitrogen of a substituent is described as being optionally substituted with one or more of a list of substituents, then one or more hydrogen atoms on the nitrogen (to the extent of any hydrogen atoms present) may be each replaced with an independently selected optional substituent.

If a substituent is described as being "independently selected from" one group, each substituent is selected independently of another. Thus, each substituent may be identical to or different from another (other) substituent(s).

As used herein, the term "one or more" refers to one or more than one, such as 2, 3, 4, 5, 6, 7, 8, 9, 10, or 20, under reasonable conditions.

In the structure 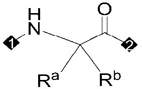 of amino acid residues represented by AA¹, if R^{a} and R^{b}, together with the carbon atom to which they are attached, form a 4-10 membered heterocyclic ring, the 4-10 membered heterocyclic ring is optionally substituted with one or more R⁰, wherein the term "the 4-10 membered heterocyclic ring is optionally substituted with one or more R⁰" means that the 4-10 membered heterocyclic ring may be unsubstituted or substituted with one or more R⁰, and in the more R⁰, the definition of each R⁰ may be identical or different. Other similar definitions may be understood with reference to the aforementioned content. In each part of this specification, the structures of the compounds of the present disclosure comply with the valence bond rules; substituents of the compounds of the present disclosure are disclosed according to group types or ranges. Particularly, the present disclosure includes each independent sub-combination of the members of these group types and ranges. For example, the term "C1-6 alkyl" refers particularly to independently disclosed methyl, ethyl, C3 alkyl, C4 alkyl, C5 alkyl, and C6 alkyl.

In the present disclosure, the term "include", "comprise", "have", "contain", or "involve" and other variant forms thereof herein are inclusive or open-ended and do not exclude other unenumerated elements or method steps. In the present disclosure, the term "C1-6 alkyl" refers to linear or branched alkyl containing 1-6 carbon atoms, including, for example, "C1-3 alkyl" or "C1-4 alkyl", methyl, ethyl, etc., and specific examples include, but are not limited to, methyl, ethyl, n-propyl, isopropyl, n-butyl, isobutyl, sec-butyl, tert-butyl, pentyl, and hexyl.

In the present disclosure, the term "C2-6 alkenyl" refers to linear, branched, or cyclic alkenyl containing at least one double bond and 2-6 carbon atoms, including, for example, "C2-4 alkenyl", etc. Examples thereof include, but are not limited to, ethenyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl, etc.

In the present disclosure, the term "C2-6 alkynyl" refers to linear or branched alkynyl containing at least one triple bond and 2-6 carbon atoms, including, for example, "C2-4 alkynyl", etc. Examples thereof include, but are not limited to, ethynyl, propynyl, 2-butynyl, 2-pentynyl, 3-pentynyl, 4-methyl-2-pentynyl, 2-hexynyl, 3-hexynyl, 5-methyl-2-hexynyl, etc.

In the present disclosure, the term "halogen" includes fluorine, chlorine, bromine, and iodine.

In the present disclosure, the term "C1-6 alkoxy" refers to alkyl as defined above which is attached to the parent molecular moiety via an oxygen atom, including, for example, "C1-4 alkoxy". Specific examples include, but are not limited to, methoxy, ethoxy, propoxy, isopropoxy, n-propoxy, isopropoxy, n-butoxy, isobutoxy, tert-butoxy, pentoxy, hexoxy, etc.

In the present disclosure, the term "4-10 membered heterocyclic ring" refers to a saturated or partially saturated ring containing 4-10 ring atoms (at least one of which is a heteroatom, such as a nitrogen atom, an oxygen atom, or a sulfur atom). The term "5-7 membered heterocyclic ring" refers to a saturated or partially saturated ring containing 5-7 ring atoms (at least one of which is a heteroatom, such as a nitrogen atom, an oxygen atom, or a sulfur atom). The term "3-7 membered heterocyclic ring" refers to a saturated or partially saturated ring containing 3-7 ring atoms (at least one of which is a heteroatom, such as a nitrogen atom, an oxygen atom, or a sulfur atom). The term "3-6 membered heterocyclic ring" refers to a saturated or partially saturated ring containing 3-6 ring atoms (at least one of which is a heteroatom, such as a nitrogen atom, an oxygen atom, or a sulfur atom). Optionally, a ring atom (e.g., a carbon atom, a nitrogen atom, or a sulfur atom) in the cyclic structure may be substituted with oxo, including but not limited to pyrrolidine, dihydropyrrolidine, tetrahydrofuran, piperidine, piperazine, tetrahydropyran, pyrrolidone, and other rings.

In the present disclosure, the term "4-10 membered carbocyclic ring" refers to a ring containing 4-10 ring atoms that are carbon atoms, including monocycles, fused rings, spiro rings, bridged rings, saturated rings, or partially saturated rings. The term "5-7 membered carbocyclic ring" refers to a ring containing 5-7 ring atoms that are carbon atoms. The term "3-7 membered carbocyclic ring" refers to a ring containing 3-7 ring atoms that are carbon atoms. Optionally, a carbon atom in the cyclic structure may be substituted with oxo, including but not limited to cyclopropane, cyclobutane, and other rings.

In the present disclosure, the term "C3-10 cycloalkyl" refers to cyclic alkyl containing 3-10 carbon atoms, including monocycles, fused rings, spiro rings, bridged rings, saturated rings, or partially saturated rings. The term "C3-6 cycloalkyl" refers to cyclic alkyl containing 3-6 carbon atoms, including monocycles, fused rings, spiro rings, bridged rings, saturated rings, or partially saturated rings. Optionally, a carbon atom in the cyclic structure may be substituted with oxo, for example, cyclopropanyl (i.e., cyclopropyl), cyclobutanyl (i.e., cyclobutyl), cyclopentanyl (i.e., cyclopentyl), and cyclohexyl.

In the present disclosure, the term "4-10 membered heterocyclyl" refers to a cyclic group containing 4-10 ring atoms (at least one of which is a heteroatom, such as a carbon atom, a nitrogen atom, or a sulfur atom). The term "5-6 membered heterocyclyl" refers to a cyclic group containing 5-6 ring atoms (at least one of which is a heteroatom, such as a nitrogen atom, an oxygen atom, or a sulfur atom). Optionally, a ring atom (e.g., a carbon atom, a nitrogen atom, or a sulfur atom) in the cyclic structure may be substituted with oxo. "4-8 membered heterocyclyl" includes, for example, "4-8 membered nitrogen-containing heterocyclyl", "4-8 membered oxygen-containing heterocyclyl", "4-7 membered heterocyclyl", "4-7 membered oxygen-containing heterocyclyl", "4-7 membered heterocyclyl", "4-6 membered heterocyclyl", "5-7 membered heterocyclyl", "5-6 membered heterocyclyl", and "5-6 membered nitrogen-containing heterocyclyl", including but not limited to oxocyclobutanyl, pyrrolidinyl, tetrahydrofuranyl, piperidinyl, piperazinyl, tetrahydropyranyl, homopiperazinyl, etc.

In the present disclosure, the term "aryl" refers to an aromatic monocyclic or polycyclic hydrocarbon group, such as C6-10 aryl. Specific examples include, but are not limited to, phenyl, naphthyl, anthryl, phenanthryl, etc. The "C6-10 aryl" refers to aryl containing 6-10 ring atoms. The "C5-10 aryl" refers to aryl containing 5-10 carbon atoms.

In the present disclosure, the term "heteroaryl" refers to an aromatic cyclic group, wherein at least one ring atom is a heteroatom, such as a nitrogen atom, an oxygen atom, or a sulfur atom. Optionally, a ring atom (e.g., a carbon atom, a nitrogen atom, or a sulfur atom) in the cyclic structure may be substituted with oxo. Specific examples include, but are not limited to, 5-10 membered heteroaryl, 5-6 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, etc., such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridinyl, 2-pyridonyl, 4-pyridonyl, pyrimidinyl, 1,4-dioxacyclohexadienyl, 2*H*-1,2-oxazinyl, 4*H*-1,2-oxazinyl, 6*H*-1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azepinyl, 1,3-diazepinyl, azocinyl, etc.

When a bond of a substituent is shown to pass through a bond connecting two atoms in a ring, the substituent may be bonded to any one of the ring-forming atoms in the substitutable ring.

In the present disclosure, the term "linker unit" refers to a component of an antibody-drug conjugate or a toxin-linker or a linker that functions to attach an antibody or an antigen-binding fragment thereof that binds to a target to the remainder of the antibody-drug conjugate. The linker unit is capable of attaching unit A to L₂, and specific examples include, but are not limited to (wherein position 1 is attached to the antibody or the antigen-binding fragment thereof, and position 2 is attached to L₂ or L₃):

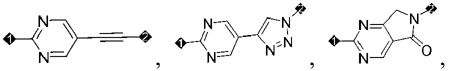

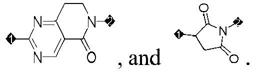

In the present disclosure, the term "connector unit" refers to a component of an antibody-drug conjugate or a toxin-linker or a linker that functions to join a linker unit and an amino acid residue or a short peptide consisting of 2-10 amino acid residues. The connector unit, when present, is capable of attaching L₁ to L₃. Specific examples include, but are not limited to (wherein position 1 is attached to the linker unit, and position 2 is attached to L₃):

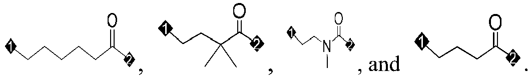

In the present disclosure, the term "spacer unit" refers to a component of an antibody-drug conjugate or a toxin-linker or a linker that functions to join a bioactive molecular fragment and an amino acid residue or a short peptide consisting of 2-10 amino acid residues, or may provide an additional structural component to further facilitate the release of the bioactive molecular fragment from the remainder of the antibody-drug conjugate. The spacer unit, when present, is capable of attaching D to L₃. Specific examples include, but are not limited to (wherein position 1 is attached to L₃, and position 2 is attached to D): 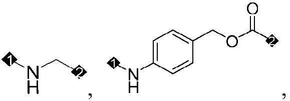 and

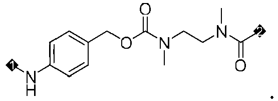

Unless otherwise specified, as used herein, the point of attachment of a substituent may be from any suitable position of the substituent.

The preferred conditions described above may be combined arbitrarily to obtain preferred examples of the present invention without departing from the general knowledge in the art.

The reagents and starting materials used in the present disclosure are commercially available.

### Beneficial Effects of the Invention

The antibody-drug conjugate comprising two or more bioactive molecules with different mechanisms of action described in the present disclosure can be used for treating a variety of hematological tumors and solid tumors and has the following advantages over antibody-drug conjugates with a single mechanism of action:
1. The antibody-drug conjugate having a variety of toxins provided in the present invention can have a synergistic effect on antitumor activity due to the different mechanisms of action of the different toxins, can overcome the problem of high heterogeneity of tumors, and has a good inhibitory effect on tumor cells (including *in vivo* and *in vitro*).
2. The linkers adopted in the present invention enable the ADC of the present invention to have high plasma stability and high chemical stability and to be cleavable in the tumor microenvironment (both inside tumor cells and outside tumor cells). Therefore, the ADC of the present invention can produce a good antitumor effect in tumors with low or no antigen expression, without only relying on the endocytosis of tumor cells.
3. The present invention improves the exposure of the whole ADC molecule in relatively acidic tumor environments by adjusting the physicochemical properties of the linkers and the whole ADC molecule, has a better tumor tissue targeting capacity, i.e., the ability to be enriched in the tumor microenvironment, has a higher therapeutic index, and overcomes the drug resistance problem associated with tumors.
4. The antibody-drug conjugate having a variety of toxins provided in the present invention has an effect of overcoming drug resistance and, in particular, provides a solution to the drug resistance problem arising during treatment with single-toxin ADCs. In both *in vitro* cells and *in vivo* drug resistance model experiments, the double-toxin ADC of the present invention can maintain good activity, for example, double-toxin ADCs comprising MMAE and camptothecin. In contrast, single-toxin ADCs with single toxins such as eribulin, MMAE, MMAF, camptothecin, etc. can only have activity against one of them or have no activity. It can be seen that the double-toxin ADC of the present disclosure can overcome the drug resistance problem associated with single toxins.
5. The antibody-drug conjugate having a variety of toxins provided in the present invention has the characteristic of convenient administration and, in particular, has improved compliance compared to combination treatment with different single-toxin ADCs.

### BRIEF DESCRIPTION OF THE DRAWING

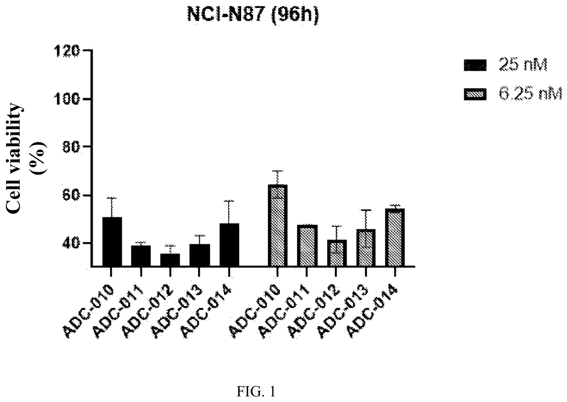

FIG. 1: a graph showing the inhibitory activity of different concentrations of ADCs against the *in vitro* proliferation of N87 tumor cells.

### DETAILED DESCRIPTION

The present disclosure is further illustrated by the following specific examples, which, however, are not intended to limit the present disclosure. Based on the teachings of the present disclosure, those skilled in the art can make various modifications or improvements without departing from the basic spirit and scope of the present disclosure.

Reagents or instruments without specified manufacturers used herein are commercially available conventional products.

The abbreviations in the present disclosure have the following meanings:

| | | | |
|---|---|---|---|
| DCC | N,N'-Dicyclohexylcarbodiimide | DMAP | 4-Dimethylaminopyridine |
| DMF | N,N-Dimethylformamide | EDCI | 1-(3-Dimethylaminopropyl)-3-ethylcarbodiimide hydrochloride |
| FA | Formic acid | HATU | O-(1H-Benzotriazol-1-yl)-N,N,N',N'-tetramethyluronium hexafluorophosphate |
| HOBt | 1-Hydroxybenzotriazole | THF | Tetrahydrofuran |
| TCEP | Tris(2-carboxyethyl)phosphine | DTT | Dithiothreitol |
| FBS | Fetal bovine serum | PB/ PBS | Phosphate buffer |

### Preparation Schemes

### Example 1. Synthesis of Toxin-Linkers (Linker-Payloads)

### Example 1.1: Synthesis of DL-2

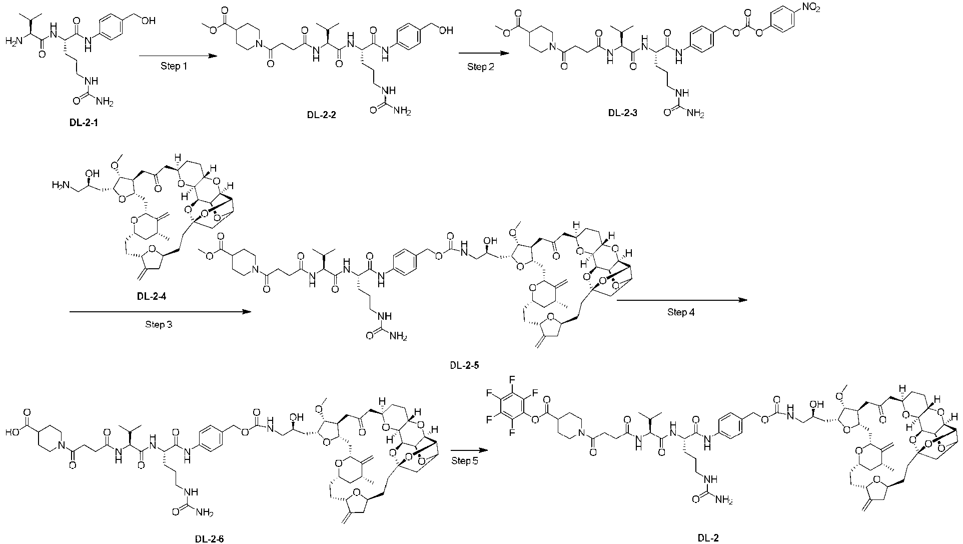

### Step 1:

Compound DL-2-1 (200.0 mg, 0.55 mmol), 4-(4-(methoxycarbonyl)piperidin-1-yl)-4-oxobutyric acid (133.79 g, 0.55 mmol) and HATU (250.95 mg, 0.66 mmol) were dissolved in DMF (2 mL), and N,N-diisopropylethylamine (213.25 mg, 1.65 mmol) was then added. The mixture was stirred at room temperature for 2 h in a nitrogen atmosphere. The reaction mixture was purified by reversed-phase column chromatography (acetonitrile:H₂O containing 0.1% FA = 5%-50%) to obtain the target compound DL-2-2 (310.0 mg).

LCMS (ESI) [M+H]⁺ =605.37.

¹H NMR (400 MHz, DMSO-*d*₆) δ 9.25 (d, *J =* 24.0 Hz, 1H), 8.22 (dd, *J* = 16.0, 8.0 Hz, 1H), 7.92-7.87 (m, 1H), 7.66-7.63 (m, 2H), 7.25-7.19 (m, 2H), 6.01-5.98 (m, 1H), 5.39 (s, 2H), 5.10-5.06 (m, 1H), 4.45-4.41 (m, 2H), 4.32 (m, 1H), 4.09-4.04 (m, 2H), 3.71-3.51 (m, 4H), 3.02-2.92 (m, 3H), 2.78 -2.55 (m, 4H), 2.49-2.32 (m, 2H), 2.14-2.08 (m, 1H), 1.90-1.85 (m, 1H), 1.70-1.66 (m, 3H), 1.51-1.29 (m, 3H), 1.15-0.99 (m, 1H), 0.95-0.90 (m, 6H).

### Step 2:

Compound DL-2-2 (200 mg, 0.33 mmol) and p-nitrophenyl carbonate (200.78 mg, 0.66 mmol) were dissolved in DMF (3 mL), and N,N-diisopropylethylamine (127.95 mg, 0.99 mmol) was then added. The mixture was stirred at room temperature for 2 h. The reaction mixture was purified by reversed-phase column chromatography (acetonitrile:H₂O containing 0.1% FA = 5%-60%) to obtain the target compound DL-2-3 (190.0 mg).

LCMS (ESI) [M+H]⁺ = 770.55.

### Step 3:

Compounds DL-2-4 (80.0 mg, 0.11 mmol) and DL-2-3 (101.61 mg, 0.13 mmol) were dissolved in DMF (1 mL), and N,N-diisopropylethylamine (42.65 mg, 0.33 mmol) was then added. The mixture was stirred at room temperature for 1 h in a nitrogen atmosphere. The reaction mixture was purified by reversed-phase column chromatography (acetonitrile:H₂O containing 0.1% FA = 5%-60%) to obtain the target compound DL-2-5 (130.0 mg).

LCMS (ESI) [M/2+H]⁺ =681.55.

¹H NMR (400 MHz, DMSO-*d*₆) δ 9.30 (d, *J =* 20.0 Hz, 1H), 8.25-8.20 (m, 1H), 7.92-7.87 (m, 1H), 7.70-7.67 (m, 2H), 7.29-7.24 (m, 2H), 7.07-7.04 (m, 1H), 6.00-5.97 (m, 1H), 5.38 (s, 2H), 5.07-5.03 (m, 1H), 5.01-4.93 (m, 3H), 4.85-4.82 (m, 1H), 4.78-4.75 (m, 1H), 4.66-4.62 (m, 1H), 4.64-4.54(m, 2H), 4.35 - 4.22 (m, 2H), 4.22 - 4.01 (m, 7H), 3.83-3.74 (m, 2H), 3.71 - 3.47 (m, 9H), 3.27-3.23 (m, 4H), 3.04-2.89 (m, 5H), 2.87-2.56 (m, 8H), 2.37-2.10 (m, 8H), 2.04-1.86 (m, 7H), 1.76-1.62 (m, 8H), 1.55-1.43 (m, 4H), 1.40-1.16 (m, 6H), 1.05-1.03 (m, 4H), 0.97-0.88 (m, 7H).

### Step 4:

Compound DL-2-5 (130.0 mg, 0.096 mmol) and lithium hydroxide monohydrate (20.14 mg, 0.48 mmol) were dissolved in THF (1 mL) and water (1 mL), and the mixture was stirred at room temperature for 0.5 h. After the reaction mixture was concentrated, the crude product was purified by reversed-phase column chromatography (acetonitrile:H₂O containing 0.1% FA = 10%-40%) to obtain the target compound DL-2-6 (95.0 mg).

LCMS (ESI) [M/2+H]⁺ = 674.52.

### Step 5:

Compound DL-2-6 (90.0 mg, 0.067 mmol) and pentafluorophenol (61.66 mg, 0.34 mmol) were dissolved in DMF (1 mL), and DMAP (24.56 mg, 0.20 mmol) and EDCI (64.22 mg, 0.34 mmol) were then added. The mixture was stirred at room temperature for 2 h in a nitrogen atmosphere. The reaction mixture was purified by preparative high performance liquid chromatography (acetonitrile:H₂O containing 0.1% FA = 10%-60%) to obtain the target compound DL-2 (30.5 mg).

LCMS (ESI) [M/2+H]⁺=757.33.

¹H NMR (400 MHz, DMSO-*d*₆) δ 9.40-9.20 (m, 1H), 8.30-8.16 (m, 1H), 7.97-7.87 (m, 1H), 7.73-7.69 (m, 2H), 7.31 (d, *J =* 8.0 Hz, 1H), 7.23 (d, *J =* 8.0 Hz, 1H), 7.08-7.04 (m, 1H), 5.98-5.97 (m, 1H), 5.39 (s, 2H), 5.08-5.06 (m, 1H), 5.02-5.00 (m, 1H), 4.97-4.95 (m, 1H), 4.88-4.85 (m, 2H), 4.78-4.76 (m, 1H), 4.68-4.65 (m, 1H), 4.58-4.54 (m, 2H), 4.40-4.23 (m, 2H), 4.21-4.02 (m, 7H), 3.86-3.66 (m, 4H), 3.55-3.50 (m, 3H), 3.26 (s, 3H), 3.14-3.00 (m, 5H), 2.88-2.62 (m, 8H), 2.40-2.12 (m, 9H), 1.5-1.90 (m, 8H), 1.81-1.61 (m, 8H), 1.55-1.48 (m, 4H), 1.38-1.24 (m, 7H), 1.06-1.05 (m, 3H), 1.00-0.90 (m, 7H).

### Example 1.2: Synthesis of 1-(4-(((S)-1-(((S)-1-((4-((SS,8S,11S,12R)-11-((S)-sec-Butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-oxa-4,7,10-triazatetradecyl)phenyl)amino)-1-oxo-5-ureidopentan-2-yl)amino)-3-methyl-1-oxobutan-2-yl)amino)-4-oxobutanoyl)piperidine-4-perfluorophenol carboxylate (DL-3)

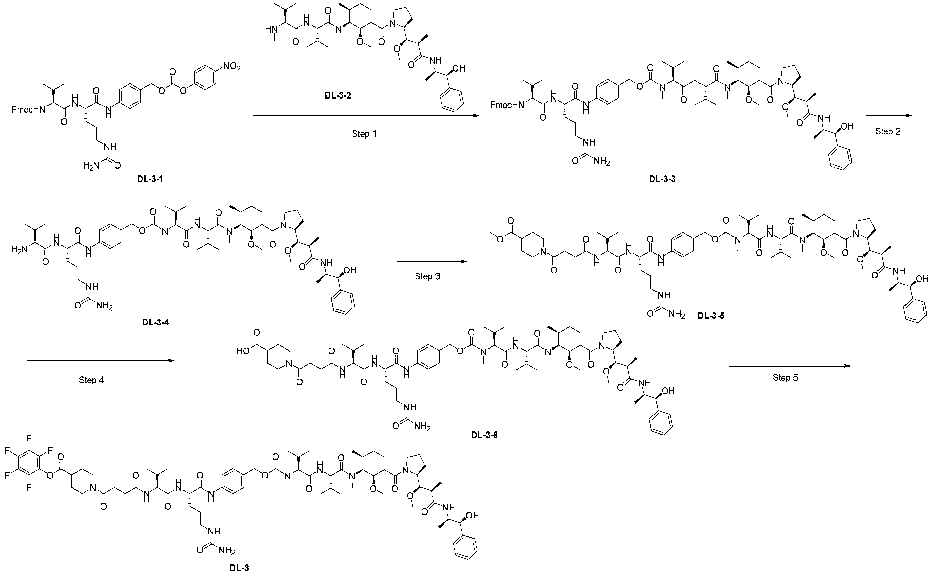

### Step 1:

DL-3-1 (1 g, 1.3 mmol) and DL-3-2 (936 mg, 1.3 mmol) were dissolved in DMF (4 mL), and pyridine (2.875 g, 36.4 mmol) and HOBt (176 mg, 1.3 mmol) were added. The mixture was stirred at room temperature for 5 h in an argon atmosphere. The reaction mixture was directly purified by reversed-phase column chromatography (acetonitrile:H₂O containing 0.1% FA = 5%-50%) to obtain the target compound DL-3-3 (1.4 g).

LCMS (ESI) [M+Na]⁺ = 1367.9.

### Step 2:

DL-3-3 (1.4 g, 1.04 mmol) was dissolved in DMF (10 mL), and diethylamine (380 mg, 5.2 mmol) was added. The mixture was stirred at room temperature for 4 h. The reaction mixture was directly purified by reversed-phase column chromatography (acetonitrile:H₂O containing 0.1% FA = 5%-40%) to obtain the target compound DL-3-4 (1.13 g).

LCMS (ESI) [M+H]⁺ = 1123.8.

### Step 3:

DL-3-4 (1.13 g, 1 mmol) was dissolved in DMF (6 mL), and 4-(4-(methoxycarbonyl)piperidin-1-yl)-4-oxobutyric acid (243 mg, 1 mmol), HATU (494 mg, 1.3 mmol), and N,N-diisopropylethylamine (387 mg, 3 mmol) were added. The mixture was stirred at room temperature for 2 h in an argon atmosphere. The reaction mixture was directly purified by reversed-phase column chromatography (acetonitrile:H₂O containing 0.1% FA = 5%-60%) to obtain the target compound DL-3-5 (1.1 g).

LCMS (ESI) [M+H]⁺ = 1349.0.

### Step 4:

DL-3-5 (1100 mg, 0.816 mmol) was dissolved in tetrahydrofuran (10 mL) and water (2 mL), and lithium hydroxide monohydrate (103 mg, 2.448 mmol) was then added. The mixture was stirred at room temperature for 1 h. 1 N hydrochloric acid was added to the reaction mixture to adjust the pH to 2, and the reaction mixture was directly purified by reversed-phase column chromatography (acetonitrile:H₂O containing 0.1% FA = 5%-60%) to obtain the target compound DL-3-6 (840 mg).

LCMS (ESI) [M+H]⁺ = 1335.0.

### Step 5:

DL-3-6 (100 mg, 0.0747 mmol) was dissolved in tetrahydrofuran (2 mL), and pentafluorophenol (28 mg, 0.149 mmol) and DCC (31 mg, 0.149 mmol) were then added. The mixture was stirred at room temperature for 2 h in an argon atmosphere. The reaction mixture was directly purified by preparative high performance liquid chromatography (acetonitrile:H₂O containing 0.1% FA = 5%-70%) to obtain the target compound DL-3 (60 mg). LCMS (ESI) [M+H]⁺ = 1501.0.

¹H NMR (400 MHz, DMSO-*d₆*) δ 9.44-9.18 (m, 1H), 8.38-8.01 (m, 2H), 7.99-7.79 (m, 2H), 7.78-7.42 (m, 3H), 7.41-7.03 (m, 8H), 6.02-5.90 (m, 1H), 5.42-5.33 (m, 3H), 5.17-4.91 (m, 2H), 4.85-4.59 (m, 1H), 4.55-4.39 (m, 2H), 4.36-4.22 (m, 2H), 4.14-3.93 (m, 4H), 3.82-3.69 (m, 1H), 3.65-3.54 (m, 1H), 3.26-3.17 (m, 8H), 3.14-3.09 (m, 2H), 3.02-2.93 (m, 4H), 2.89-2.82 (m, 3H), 2.70-2.55 (m, 4H), 2.45-2.23 (m, 5H), 2.16-2.07 (m, 3H), 1.94-1.61 (m, 10H), 1.55-1.42 (m, 4H), 1.36-1.23 (m, 3H), 1.05-0.96 (m, 7H), 0.94-0.88 (m, 7H), 0.83-0.74 (m, 13H).

### Example 1.3: Synthesis of 1-(4-(((S)-1-(((S)-1-((4-((5S,8S,11S,12R)-11-((S)-sec-Butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenyl)amino)-6-(dimethylamino)-1-oxobutan-2-yl)amino)-3-methyl-1-oxobutan-2-yl)amino)-4-oxobutyl)piperidine-4-perfluorophenol carboxylate (DL-4)

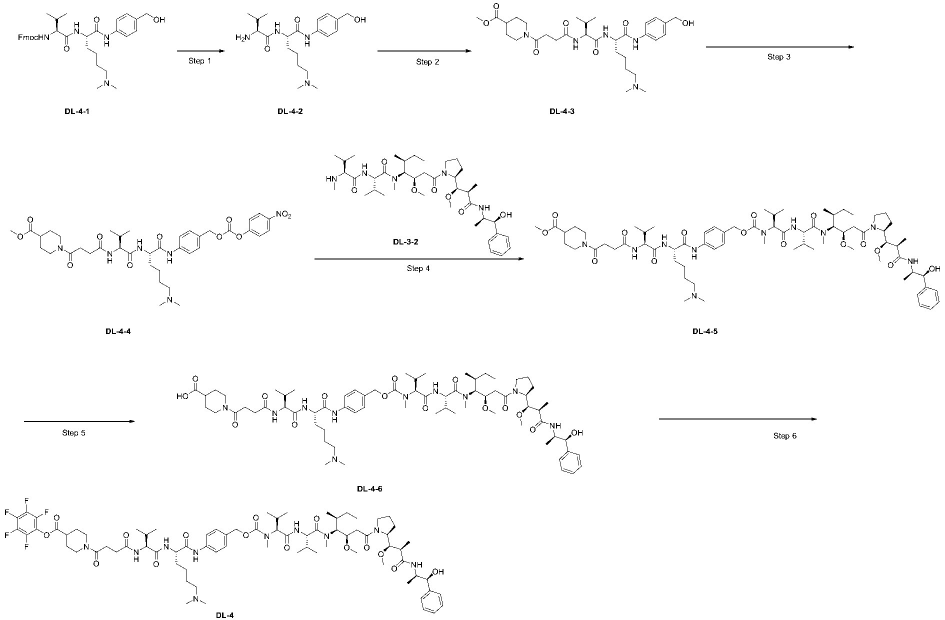

### Step 1:

DL-4-1 (2.5 g, 4.167 mmol) was dissolved in DMF (10 mL), and diethylamine (912 mg, 12.5 mmol) was added. The mixture was stirred at room temperature for 2 h. A solution of the target compound DL-4-2 (1.58 g) in DMF was obtained and directly used in the next step.

LCMS (ESI) [M+H]⁺ = 379.4.

### Step 2:

4-(4-(Methoxycarbonyl)piperidin-1-yl)-4-oxobutyric acid (1.518 g, 6.25 mmol), HATU (2.375 g, 6.25 mmol), and N,N-diisopropylethylamine (1.612 g, 12.5 mmol) were added to a solution of DL-4-2 (1.58 g, 4.18 mmol) in DMF, and the mixture was stirred at room temperature for 2 h in an argon atmosphere. The reaction mixture was directly purified by preparative high performance liquid chromatography (acetonitrile:H₂O containing 0.1% FA = 5%-20%) to obtain the target compound DL-4-3 (1.4 g).

LCMS (ESI) [M+H]⁺ = 604.5.

### Step 3:

DL-4-3 (200 mg, 0.331 mmol) was dissolved in DMF (3 mL), and p-nitrophenyl carbonate (201 mg, 0.663 mmol) and N,N-diisopropylethylamine (86 mg, 0.663 mmol) were added. The mixture was stirred at room temperature for 30 min in an argon atmosphere. A solution of the target compound DL-4-4 (255 mg) in DMF was obtained and directly used in the next step.

LCMS (ESI) [M+H]⁺ = 769.5.

### Step 4:

DL-3-2 (200 mg, 0.279 mmol), pyridine (88 mg, 1.116 mmol), and HOBt (75 mg, 0.558 mmol) were sequentially added to a solution of DL-4-4 (255 mg, 0.33 mmol) in DMF, and the mixture was stirred at room temperature for 48 h in an argon atmosphere. The reaction mixture was directly purified by preparative high performance liquid chromatography (acetonitrile:H₂O containing 0.1% FA = 5%-30%) to obtain the target compound DL-4-5 (170 mg).

LCMS (ESI) [M+H]⁺ = 1347.5.

### Step 5:

DL-4-5 (370 mg, 0.274 mmol) was dissolved in THF (2 mL) and water (2 mL), and lithium hydroxide monohydrate (35 mg, 0.824 mmol) was then added. The mixture was stirred at room temperature for 30 min. The reaction mixture was directly purified by preparative high performance liquid chromatography (acetonitrile:H₂O containing 0.1% FA = 5%-30%) to obtain the target compound DL-4-6 (260 mg).

LCMS (ESI) [M+H]⁺ = 1333.8.

### Step 6:

DL-4-6 (110 mg, 0.0825 mmol) was dissolved in THF (5 mL), and pentafluorophenol (31 mg, 0.165 mmol) and DCC (34 mg, 0.165 mmol) were then added. The mixture was stirred at room temperature for 16 h in an argon atmosphere. The reaction mixture was purified by preparative high performance liquid chromatography (acetonitrile:H₂O containing 0.1% FA = 5%-60%) to obtain the target compound DL-4 (51 mg).

LCMS (ESI) [M+H]⁺ = 1500.0.

¹H NMR (400 MHz, DMSO-*d₆*) δ 9.37-9.20 (m, 1H), 8.44-8.11 (m, 2H), 8.11-7.95 (m, 1H), 7.93-7.78 (m, 2H), 7.77-7.59 (m, 3H), 7.36-7.22 (m, 8H), 7.22-7.09 (m, 2H), 5.23-4.82 (m, 3H), 4.77-4.60 (m, 1H), 4.57-4.37 (m, 3H), 4.36-4.18 (m, 3H), 4.13-3.84 (m, 6H), 3.09-2.75 (m, 12H), 2.68-2.60 (m, 3H), 2.31-2.23 (m, 9H), 2.16-2.05 (m, 4H), 1.85-1.67 (m, 7H), 1.52-1.46 (m, 3H), 1.39-1.24 (m, 5H), 1.07-0.79 (m, 33H).

### Example 1.4: Synthesis of 4-((S)-2-((S)-3-Methyl-2-(2-((5-(2-(methylsulfonyl)pyrimidin-5-yl)pent-4-yn-1-yl)oxy)acetamido)butanamido)-ureidopentanamido)benzyl (eribulinyl)carbamate (DL-6)

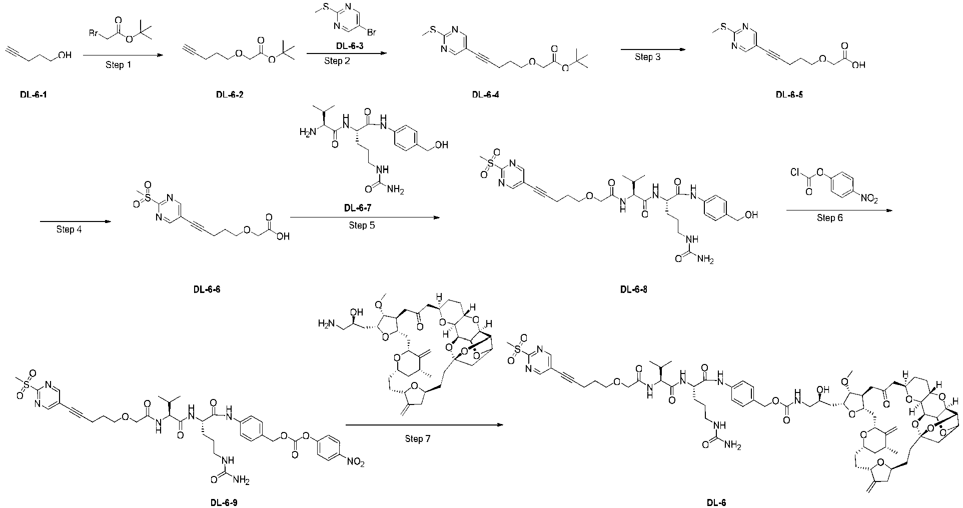

### Step 1:

Compound DL-6-1 (5.0 g, 50.9 mmol) was dissolved in toluene (170 mL), and tetrabutylammonium bromide (6.3 g, 19.6 mmol) was added. The mixture was cooled to 0 °C, sodium hydroxide (61.2 g, 535.5 mmol) was added, and tert-butyl bromoacetate (34.78 g, 178.32 mmol) was then added. The mixture was left to react overnight at room temperature. Water (80 mL) was added to the reaction mixture, and extraction was then performed three times with ethyl acetate (80 mL × 3). The organic phases were combined, dried over anhydrous sodium sulfate, filtered, and concentrated to dryness by rotary evaporation to obtain compound DL-6-2 (10.2 g). ¹H NMR (400 MHz, DMSO-*d₆*) δ 8.67 (s, 2H), 3.98 (s, 2H), 3.57 (t, *J =* 6.2 Hz, 2H), 2.57 - 2.54 (m, 2H), 2.52 (s, 3H), 1.83 - 1.76 (m, 2H), 1.42 (s, 9H).

### Step 2:

Compound DL-6-2 (10 g, 50.5 mmol) was dissolved in tetrahydrofuran (100 mL), and DL-6-3 (10.35 g, 50.5 mmol), bis(triphenylphosphine)palladium(II) dichloride (3.5 g, 5.05 mmol), cuprous iodide (1.92 g, 10.1 mmol), and triethylamine (15.3 g, 151.5 mmol) were added. The mixture was stirred overnight at 70 °C in a nitrogen atmosphere. The reaction mixture was cooled and filtered, and the filtrate was concentrated under reduced pressure and then purified by column chromatography (petroleum ether:ethyl acetate = 5:1) to obtain compound DL-6-4 (5.2 g).

LCMS (ESI) [M+H]⁺ = 323.0.

¹H NMR (400 MHz, DMSO-*d₆*) δ 8.67 (s, 2H), 3.98 (s, 2H), 3.57 (t, *J =* 6.2 Hz, 2H), 2.57 - 2.54 (m, 2H), 2.52 (s, 3H), 1.83 - 1.76 (m, 2H), 1.42 (s, 9H).

### Step 3:

Compound DL-6-4 (1.0 g, 3.1 mmol) was dissolved in dichloromethane (50 mL), and trifluoroacetic acid (3.54 g, 31 mmol) was added. The mixture was stirred overnight at room temperature. The reaction mixture was directly concentrated under reduced pressure to obtain compound DL-6-5 (1.5 g, crude).

LCMS (ESI) [M+H]⁺ = 267.0.

¹H NMR (400 MHz, DMSO-*d₆*) δ 8.67 (s, 2H), 4.03 (s, 2H), 3.59 (t, *J =* 6.2 Hz, 2H), 2.58 - 2.53 (m, 2H), 2.53 (s, 3H), 1.84 - 1.77 (m, 2H).

### Step 4:

Compound DL-6-5 (1.5 g, 5.6 mmol) was dissolved in tetrahydrofuran (20 mL) and water (20 mL), and potassium peroxymonosulfonate (10.3 g, 16.8 mmol) was added. The mixture was stirred at room temperature for 2 h. The reaction mixture was concentrated under reduced pressure to remove tetrahydrofuran, and the aqueous phase was extracted three times with ethyl acetate (50 mL). The organic phases were combined, dried over anhydrous sodium sulfate, concentrated under reduced pressure, and then purified by reversed-phase chromatography (acetonitrile:H₂O containing 0.05% HCl = 5%-50%) to obtain compound DL-6-6 (0.4 g).

LCMS (ESI) [M+H]⁺ = 299.1.

¹H NMR (400 MHz, DMSO-*d₆*) δ 12.60 (s, 1H), 9.11 (s, 2H), 4.03 (s, 2H), 3.60 (t, *J =* 6.2 Hz, 2H), 3.41 (s, 3H), 2.63 (t, *J =* 7.1 Hz, 2H), 1.87 - 1.80 (m, 2H).

### Step 5:

Compound DL-6-7 (200.0 mg, 0.53 mmol), DL-6-6 (158.10 g, 0.53 mmol), and DMTMM (161.33 mg, 0.58 mmol) were dissolved in DMF (2 mL), and the mixture was stirred at 25 °C for 2 h in a nitrogen atmosphere. Dichloromethane (10 mL) was added to the reaction mixture, and the organic phase was washed twice with a saturated aqueous sodium bicarbonate solution (5 mL × 2), dried, and concentrated to obtain the target compound DL-6-8 (310 mg).

LCMS (ESI) [M+H]⁺ = 660.45.

### Step 6:

DIPEA (182.23 mg, 1.41 mmol) was added to a solution of compound DL-6-8 (310 mg, 0.47 mmol) and p-nitrophenyl chloroformate (189.47 mg, 0.94 mmol) in DMF (3 mL), and the mixture was stirred at room temperature for 2 h. The reaction mixture was purified by reversed-phase column chromatography (ACN:H₂O containing 0.1% FA = 10%-50%) to obtain the target compound DL-6-9 (120.0 mg).

LCMS (ESI) [M+H]⁺ = 825.46.

### Step 7:

Compound DL-6-9 (80.0 mg, 0.11 mmol) and eribulin (99.81 mg, 0.12 mmol) were dissolved in DMF (1 mL), and DIPEA (42.65 mg, 0.33 mmol) was then added at 0 °C. The mixture was stirred at 0 °C for 2 h in a nitrogen atmosphere. The reaction mixture was concentrated and then purified by preparative high performance liquid chromatography (ACN:H₂O containing 0.1% TFA = 10%-60%) to obtain the target compound DL-6 (53.0 mg). LCMS (ESI) [M/2+H]⁺ =709.03.

¹H NMR (400 MHz, DMSO-*d*₆) δ 10.03 (s, 1H), 9.10 (s, 2H), 8.33 (d, J = 8.0 Hz, 1H), 7.57 (d, J *=* 8.0 Hz, 2H), 7.48 (d, J = 12.0 Hz, 1H), 7.27 (d, J = 8.0 Hz, 2H), 7.08-7.06 (m, 1H), 5.05 - 4.93 (m, 4H), 4.83-4.75 (m, 2H), 4.63-4.54 (m, 2H), 4.40 - 4.25 (m, 3H), 4.20 - 3.96 (m, 8H), 3.83 - 3.73 (m, 6H), 3.50-3.46 (m, 4H), 3.40 (s, 3H), 3.26-3.22 (m, 4H), 3.04-2.97 (m, 4H), 2.85 - 2.62 (m, 6H), 2.34 - 2.12 (m, 6H), 2.07 - 1.86 (m, 9H), 1.75 - 1.16 (m, 18H), 1.04-0.96 (m, 4H), 0.87-0.79 (m, 6H).

### Example 2. Synthesis of Antibody-Drug Conjugates (ADCs)

### Example 2.1: Preparation of ADC-001

3.0 mL of A166 (Her2-ADC, 16.1 mg/mL) was taken and diluted with 0.03 mL of a 20 mM PB + 100 mM disodium edetate solution (pH 7.6), and the pH was adjusted to 7.44 with a 0.5 M Na₂HPO₄ solution. 1.0 mL of the above solution was taken, and a 20 mM TCEP (0.0276 mL, 0.552 µmol) solution was added and well mixed. The mixture was left to stand at room temperature for 90 min. Then a solution of DL-1 in dimethyl sulfoxide was added and well mixed, and the mixture was left to stand at room temperature. After that, buffer exchange was performed using a centrifugal ultrafiltration tube (Merck, Amicon Ultra-15). The conjugate product ADC-001 of DL-1 and A166 was obtained.

The structure of A166 is shown below (prepared by referring to a known synthesis method described in WO2019214492):

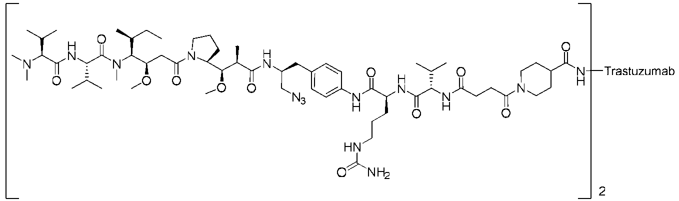

The structure of DL-1 is shown below (prepared by referring to a known synthesis method described in WO2015115091):

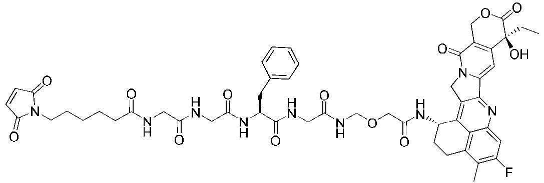

The structure of ADC-001 is shown below:

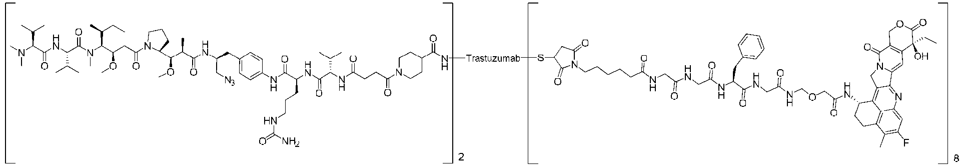

### Example 2.2: Preparation of ADC-002

1.0 mL of trastuzumab (24.7 mg/mL) was taken and diluted with 0.10 mL of a 20 mM PB + 100 mM disodium edetate solution (pH 7.6), and the pH was adjusted to 7.98-8.47 with a 0.5 M Na₂HPO₄ solution. A solution of DL-2 (2.086 mg, 8 times the amount of substance (moles) of the antibody) in dimethyl sulfoxide (0.138 mL) was added to the above solution and well mixed, and the mixture was left to stand at 37 °C for 4 h. After that, the buffer was exchanged for a 20 mM His-HCl buffer solution (pH 5.9) using a centrifugal ultrafiltration tube (Merck, Amicon Ultra-15). After the buffer exchange, the solution was diluted with 0.10 mL of a 20 mM PB + 100 mM disodium edetate solution (pH 7.6), and the pH was adjusted to 7.40 with a 0.5 M Na₂HPO₄ solution. Then a 20 mM TCEP (0.0446 mL, 0.892 µmol) solution was added and well mixed, and the mixture was left to stand at room temperature for 90 min. Then a solution of DL-5 (2.15 mg, 10 times the amount of substance (moles) of the antibody) in dimethyl sulfoxide (0.1958 mL) was added and well mixed, and the mixture was left to stand at room temperature for 2 h. After that, the buffer was exchanged for a 20 mM His-HCl buffer solution (pH 5.9) using a centrifugal ultrafiltration tube (Merck, Amicon Ultra-15). ADC-002 was obtained.

The structure of DL-5 is shown below, and see WO2022170971 for the synthesis method.

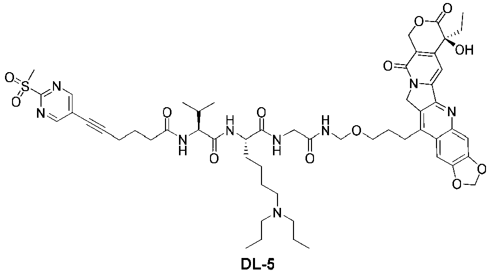

The DAR value was determined as follows:

### Sample treatment

A 50 µg ADC sample was taken and diluted to 0.5 mg/mL in ultrapure water, and 1 µL of 1 M DTT was then added and well mixed. The mixture was then centrifuged, and the supernatant was collected and injected for analysis.

### Instrument information

| Name | Manufacturer | Model |
|---|---|---|
| UPLC | AB SCIEX | ExionLC |
| High resolution mass spectrometer | AB SCIEX | X500B |

### Liquid phase parameters

| | | |
|---|---|---|
| Chromatographic column | Xbridge Protein BEH SEC (2.5 µm,4.6* 150 mm) | |
| Flow rate | 0.3 mL/min | |
| Column temperature | 30°C | |
| Injection volume | 1 µL | |
| Sample chamber temperature | 8°C | |
| Elution mode | Isocratic elution | |
| Mobile phase | Mobile phase A: 0.1% FA in H₂O | |
| | Mobile phase B: 0.1% FA in ACN | |
| Time/min | 0 | 10 |
| A% | 65 | 65 |
| B% | 35 | 35 |

### Mass spectrometry parameters

| Mass spectrometry parameters | | |
|---|---|---|
| Convention | Run time: | 10 min |
| | Total scan time: | 1.028 s |
| | Estimated number of cycles: | 584 |
| | Complete protein mode: | On |
| | Large protein (>70 kDa): | No |
| | Decrease detector voltage: | Yes |
| | Scan type: | Primary parent ion full scan |
| Source gas parameters | Source gas 1: | 45 psi |
| | Source gas 2: | 45 psi |
| | Air curtain gas: | 30 psi |
| | Collision gas: | 7 |
| | Temperature of heated gas (°C): | 450 |
| Primary mass spectrum | Ionization polarity: | Positive |
| | Lower limit of mass number scan range (Da): | 600 |
| | Upper limit of mass number scan range (Da): | 4000 |
| | Cumulative time: | 1 s |
| | Electrospray voltage: | 5000 V |
| | Declustering voltage: | 120 V |
| | Declustering voltage variation step | 0 V |
| | Collision energy: | 12 V |
| | Collision energy variation step: | 0 V |
| | Total number of ions counted per unit time: | 80 |

According to mass spectrometry analysis, in ADC-002, the DAR value of DL-2 was 1.4, and the DAR value of DL-5 was 8.0.

The structure of ADC-002 is shown below:

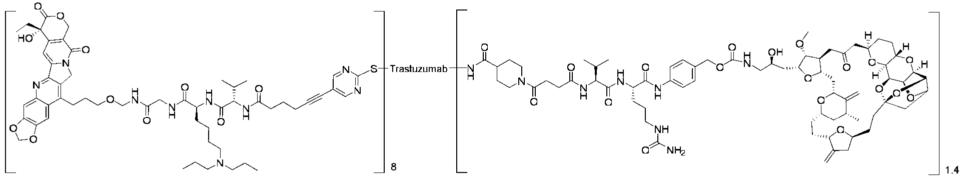

### Example 2.3: Preparation of ADC-003

Reference was made to the synthesis method of Example 2.2. The feeding amount of DL-2 was increased to 12 times the amount of substance (moles) of the antibody (1.565 mg), and ADC-003 was obtained. According to mass spectrometry analysis, in ADC-003, the DAR value of DL-5 was 8.0, and the DAR value of DL-2 was 2.3. The structure of ADC-003 is shown below:

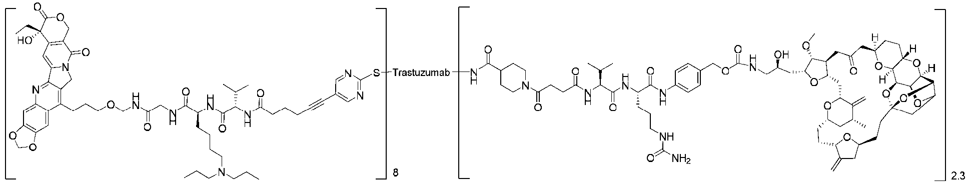

### Example 2.4: Preparation of ADC-004

Reference was made to the synthesis method of Example 2.2. The feeding amount of DL-2 was increased to 20 times the amount of substance (moles) of the antibody (2.609 mg), and ADC-004 was obtained. According to mass spectrometry analysis, in ADC-004, the DAR value of DL-5 was 8.0, and the DAR value of DL-2 was 3.3. The structure of ADC-004 is shown below:

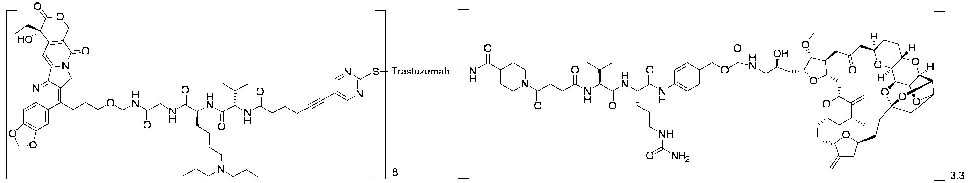

### Example 2.5: Preparation of ADC-005

0.8 mL of trastuzumab (24.7 mg/mL) was taken and diluted with 0.08 mL of a 20 mM PB + 100 mM disodium edetate solution (pH 7.6), and the pH was adjusted to 8.0 with a 0.5 M Na₂HPO₄ solution. A solution of DL-3 (0.888 mg, 4 times the amount of substance (moles) of the antibody) in dimethyl sulfoxide (0.0592 mL) was added to the above solution and well mixed, and the mixture was left to stand at 37 °C for 4 h. After that, the buffer was exchanged for a 20 mM PB buffer solution (pH 7.6) using a centrifugal ultrafiltration tube (Merck, Amicon Ultra-15). After the buffer exchange, the solution was diluted with 0.015 mL of a 20 mM PB + 100 mM disodium edetate solution (pH 7.6), and the pH was adjusted to 7.40. Then a 20 mM TCEP (0.0335 mL, 0.670 µmol) solution was added and well mixed, and the mixture was left to stand at 25 °C for 90 min. Then a solution of DL-5 (1.613 mg, 10 times the amount of substance (moles) of the antibody) in dimethyl sulfoxide (0.1469 mL) was added and well mixed, and the mixture was left to stand at 25 °C for 2 h. After that, the buffer was exchanged for a 20 mM His-HCl buffer solution (pH 5.9) using a centrifugal ultrafiltration tube (Merck, Amicon Ultra-15). ADC-005 was obtained. According to mass spectrometry analysis, the DAR value of DL-5 was 8.0, and the DAR value of DL-3 was 1.1.

The structure of ADC-005 is shown below:

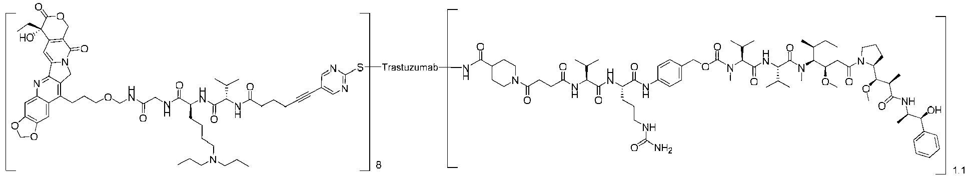

### Example 2.6: Preparation of ADC-006

Reference was made to the synthesis method of Example 2.5. The feeding amount of DL-3 was increased to 12 times the amount of substance (moles) of the antibody (1.333 mg), and ADC-006 was obtained. According to mass spectrometry analysis, in ADC-006, the DAR value of DL-5 was 8.0, and the DAR value of DL-3 was 2.4. The structure of ADC-006 is shown below:

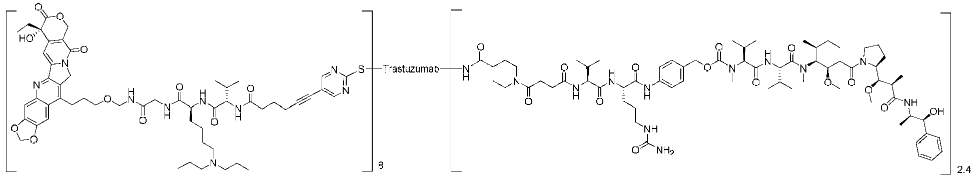

### Example 2.7: Preparation of ADC-007

Reference was made to the synthesis method of Example 2.5. The feeding amount of DL-3 was increased to 20 times the amount of substance (moles) of the antibody (2.221 mg), and ADC-007 was obtained. According to mass spectrometry analysis, in ADC-007, the DAR value of DL-5 was 8.0, and the DAR value of DL-3 was 3.7. The structure of ADC-007 is shown below:

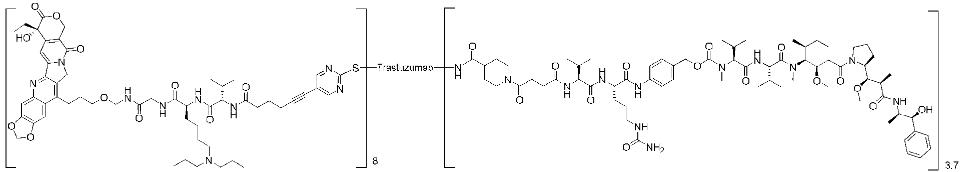

### Example 2.8: Preparation of ADC-008

0.4 mL of trastuzumab (24.7 mg/mL) was taken and diluted with 0.04 mL of a 20 mM PB + 100 mM disodium edetate solution (pH 7.6), and the pH was adjusted to 8.0 with a 0.5 M Na₂HPO₄ solution. A solution of DL-4 (3.337 mg, 20 times the amount of substance (moles) of the antibody) in dimethyl sulfoxide (0.2225 mL) was added to the above solution and well mixed, and the mixture was left to stand at 37 °C for 4 h. After that, the buffer was exchanged for a 20 mM PB buffer solution (pH 7.6) using a centrifugal ultrafiltration tube (Merck, Amicon Ultra-15). After the buffer exchange, the solution was diluted with 0.015 mL of a 20 mM PB + 100 mM disodium edetate solution (pH 7.6), and the pH was adjusted to 7.40. Then a 20 mM TCEP (0.0167 mL, 0.334 µmol) solution was added and well mixed, and the mixture was left to stand at 25 °C for 90 min. Then a solution of DL-5 (0.806 mg, 10 times the amount of substance (moles) of the antibody) in dimethyl sulfoxide (0.0734 mL) was added and well mixed, and the mixture was left to stand at 25 °C for 2 h. After that, the buffer was exchanged for a 20 mM His-HCl buffer solution (pH 5.9) using a centrifugal ultrafiltration tube (Merck, Amicon Ultra-15). ADC-008 was obtained. According to mass spectrometry analysis, in ADC-008, the DAR value of DL-5 was 8.0, and the DAR value of DL-4 was 1.9.

The structure of ADC-008 is shown below:

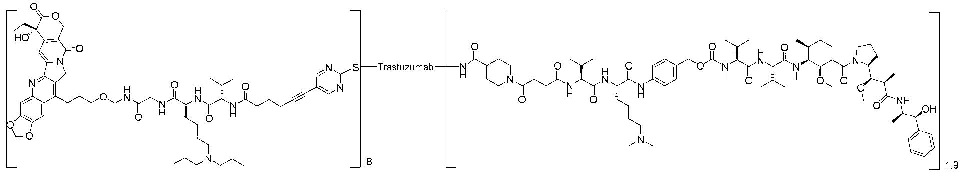

### Example 2.9: Preparation of ADC-009

Trastuzumab (0.4 mL, 9.88 mg) was added to a sodium edetate solution with a final concentration of 1 mM and well mixed, and the pH of the sample was adjusted to 7.45 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. 5.0 molar equivalents (relative to the antibody) of a 10 mmol/L solution of DL-6 (0.055 mg) in dimethyl sulfoxide was added. After 2 h of reaction at 25 °C, cysteine was added in excess. The buffer was exchanged for a 20 mM His-Hel buffer solution (pH 5.9) using a centrifugal ultrafiltration tube (Merck, Amicon Ultra-15), and an ADC-009 sample was obtained. According to analysis, the DAR value was 1.8.

The structure of ADC-009 is shown below:

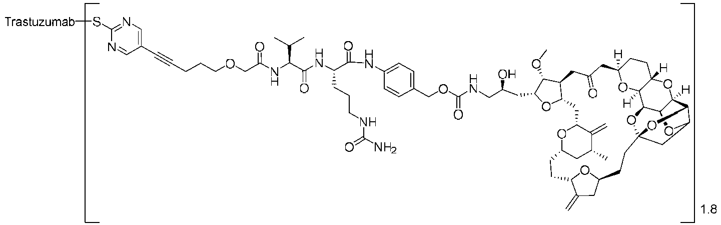

### Example 2.10: Preparation of ADC-010

5.0 mL of trastuzumab (16.1 mg/mL) was taken and diluted with 0.10 mL of a 20 mM PB + 100 mM disodium edetate solution (pH 7.6), and the pH was adjusted to 7.46 with a 0.5 M Na₂HPO₄ solution. 1.0 mL of the above solution was taken, and a 20 mM TCEP (tris(2-carboxyethyl)phosphine, 0.0278 mL, 0.556 µmol) solution was added and well mixed. The mixture was left to stand at room temperature for 90 min. Then a solution of DL-5 (1.332 mg, 10 times the amount of substance (moles) of the antibody) in dimethyl sulfoxide (0.1213 mL) was added and well mixed, and the mixture was left to stand at room temperature for 2 h. After that, the buffer was exchanged for a 20 mM His-HCl buffer solution (pH 5.9) using a centrifugal ultrafiltration tube (Merck, Amicon Ultra-15). An ADC-010 sample was obtained. According to analysis, the DAR value was 8.0.

The structure of ADC-010 is shown below:

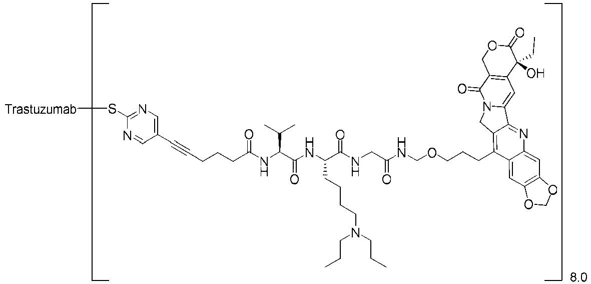

### Example 2.11: Preparation of ADC-011

5.76 mg of the ADC-010 sample was taken, and a solution of DL-7 (0.242 mg, 4.0 times the amount of substance (moles) of the antibody) in dimethyl sulfoxide (0.0159 mL) was added and well mixed, and the mixture was left to stand at 25 °C for 4 h. After that, the buffer was exchanged for a 20 mM His-HCl buffer solution (pH 5.9) using a centrifugal ultrafiltration tube (Merck, Amicon Ultra-15). ADC-011 was obtained. According to mass spectrometry analysis, in ADC-011, the DAR value of DL-5 was 8.0, and the DAR value of DL-7 was 2.0.

The structure of DL-7 (purchased from Levena (Suzhou) Biopharma Co., Ltd., Lot No. 20190201-A) is shown below:

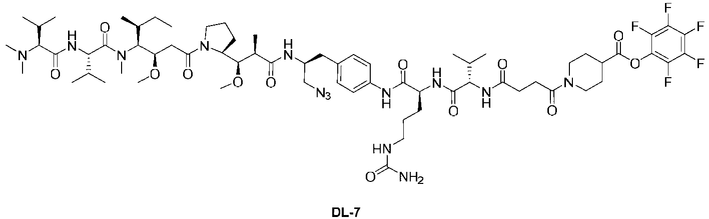

The structure of ADC-011 is shown below:

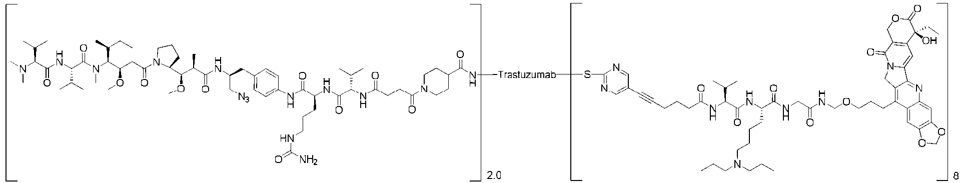

### Example 2.12: Preparation of ADC-012

Reference was made to the synthesis method of Example 2.11. The feeding amount of DL-7 was reduced to 3.6 times the amount of substance (moles) of the antibody (0.217 mg), and ADC-012 was obtained. According to mass spectrometry analysis, in ADC-012, the DAR value of DL-5 was 8.0, and the DAR value of DL-7 was 1.9. The structure of ADC-012 is shown below:

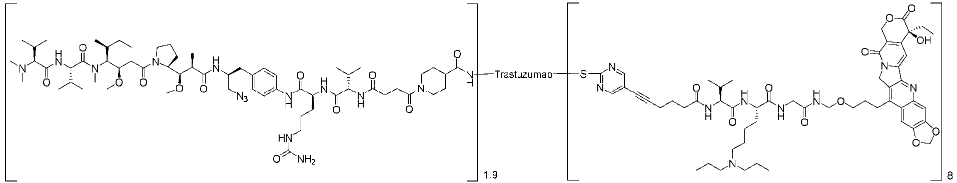

### Example 2.13: Preparation of ADC-013

Reference was made to the synthesis method of Example 2.11. The feeding amount of DL-7 was increased to 5 times the amount of substance (moles) of the antibody (0.301 mg), and ADC-013 was obtained. According to mass spectrometry analysis, in ADC-013, the DAR value of DL-5 was 8.0, and the DAR value of DL-7 was 2.4. The structure of ADC-013 is shown below:

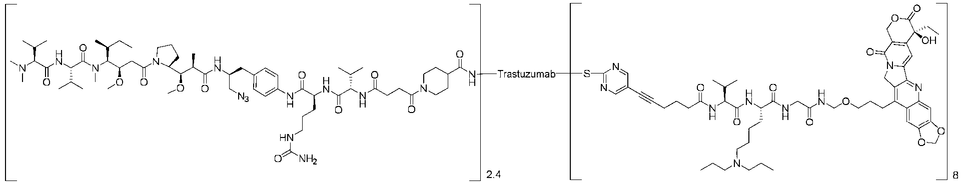

### Example 2.14: Preparation of ADC-014

0.5 mL of trastuzumab (24.7 mg/mL) was taken and diluted in 0.10 mL of a 20 mM PB + 100 mM disodium edetate solution (pH 7.6), and the pH was adjusted to 7.5 with a 0.5 M Na₂HPO₄ solution. A solution of DL-7 (0.453 mg, 3.5 times the amount of substance (moles) of the antibody) in dimethyl sulfoxide (0.0298 mL) was added to the above solution and well mixed, and the mixture was left to stand at 25 °C for 4 h. After that, the buffer was exchanged for a 20 mM His-HCl buffer solution (pH 5.9) using a centrifugal ultrafiltration tube (Merck, Amicon Ultra-15), and an ADC-014 sample was obtained. According to analysis, the DAR value was 2.0.

The structure of ADC-014 is shown below:

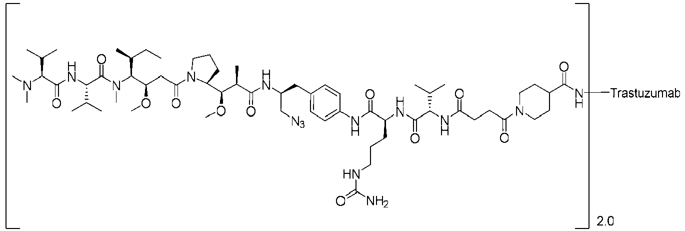

### Example 3. Bioactivity Assays of Antibody-Drug Conjugates (ADCs)

### Example 3.1: Assay for Inhibitory Activity of ADCs Against In Vitro Cell (N87) Activity

### 1. Method:

N87 (RPMI1640 + 10% FBS) cells were thawed and cultured, plated onto a 96-well flat-bottom plate at a density of 5000 cells/well and 100 µL/well, and cultured overnight in an incubator at 37 °C. The next day, the test ADC molecules were diluted in a complete culture medium to a maximum concentration of 100 nM, and the solutions were diluted 4-fold to obtain 10 concentration points. The diluted ADC samples (100 µL) were added to cell culture wells. After 4 days of culture at 37 °C, 50 µL of Cell titer glo substrate was added to each well. After 5 min of incubation, the fluorescence values were measured. The key material sources are shown in Table 1 below:

**Table 1. Material sources**

| Name | Manufacturer or source | Catalog No. |
|---|---|---|
| N87 cells | Procell | CL-0211 |
| 1640 culture medium | ADamas life | C8016 |
| Fetal bovine serum | Cornning | 35-081-CV |
| Cell titer glo substrate | ADamas life | RA-GL11-A |

### 2. Results:

As shown in Table 2, the double-toxin ADC molecules of the present disclosure all exhibited significant inhibitory activity against the proliferation of the N87 tumor cell line.

**Table 2. The inhibitory activity of ADCs against the in vitro proliferation of N87 tumor cells**

| ADC No. | IC50 value (nM) |
|---|---|
| ADC-002 | 0.04 |
| ADC-003 | 0.03 |
| ADC-004 | 0.04 |
| ADC-005 | 0.24 |
| ADC-006 | 0.11 |
| ADC-007 | 0.09 |
| ADC-008 | 0.11 |
| ADC-009 | 0.06 |
| ADC-010 | 0.54 |

### Example 3.2: Assay for Inhibitory Activity of ADCs Against In Vitro Cell (NCI-N87) Activity

### 1. Method:

NCI-N87 (RPMI 1640 + 10% FBS + 1% P/S) cells were thawed and cultured, plated onto a 96-well flat-bottom plate at a density of 5000 cells/well and 190 µL/well, and cultured overnight in an incubator at 37 °C. The next day, the test ADC molecules were diluted in a serum-free culture medium to a maximum concentration of 100 nM, and the solutions were diluted 4-fold to obtain 10 concentration points. The diluted ADC samples (10 µL) (20*) were added to cell culture wells. After 4 days of culture at 37 °C, 100 µL of Cell titer glo substrate was added to each well. After 10 min of incubation, the fluorescence values were measured. The key material sources are shown in Table 3 below:

**Table 3. Material sources**

| Name | Manufacturer or source | Catalog No. |
|---|---|---|
| NCI-N87 cells | Procell | CL-0211 |
| RPMI 1640 culture medium | ADamas life | C8016 |
| Fetal bovine serum | Cornning | 35-081-CV |
| Cell Titer Turbo substrate | ADamas life | RA-GL11-A |

### 2. Results:

The double-toxin ADC molecules of the present disclosure all exhibited significant inhibitory activity against the proliferation of the NCI-N87 tumor cell line. The results are shown in Table 4 and FIG. 1.

**Table 4. The inhibitory activity of ADCs against the in vitro proliferation of N87 tumor cells**

| ADC No. | IC50 value (nM) |
|---|---|
| ADC-010 | 2.09 |
| ADC-011 | 1.64 |
| ADC-012 | 1.51 |
| ADC-013 | 1.14 |
| ADC-014 | 1.47 |

### Example 3.3: Assay for Inhibitory Activity of ADCs Against In Vitro Proliferation of N87 Cells Overexpressing ABCB1/ABCG2 Transporters

### 1. Method

The inhibitory activity of a double-toxin ADC and single-toxin ADCs against proliferation was assessed using N87 cells overexpressing human ABCB1 or human ABCG2 (ATCC, provided by WuXi AppTec). N87 tumor cells overexpressing human ABCB1 and human ABCG2 were cultured in a specified cell culture medium (RPMI1640 + 10% FBS) in a 5% CO₂ incubator at 37 °C according to a conventional method. The tumor cells were digested with trypsin by a conventional method, collected, and counted. The cells were resuspended in corresponding complete culture media and added to a 96-well plate at 3000 cells/well and 100 µL/well. Preparation of ADC sample concentration gradients: The test drugs were diluted in a complete culture medium. For N87 cells overexpressing human ABCB1, the ADCs and antibody drugs were 5-fold diluted from a concentration of 2000 nM to obtain 10 concentration points. For N87 cells overexpressing human ABCG2, ADC-010 was 5-fold diluted from 2000 nM to obtain 10 concentration points, and the other ADC drugs were diluted from 200 nM to obtain 10 concentration points. A total of 100 µL of the diluted ADCs was added to a 96-well plate containing 100 µL of culture medium and cells, and the plate was incubated in a 5% CO₂ incubator at 37 °C for 6 days. After the incubation was completed, 75 µL of cell titer glow substrate was added to each cell well. After 10 min of incubation at room temperature, the full-wavelength fluorescence values were measured. GraphPad software was used to plot dose-dependent cell proliferation inhibition and analyze their half maximal inhibitory concentrations (IC₅₀, nM).

### 2. Results

As shown in Table 5, eribulin ADC-009 lost activity in N87 tumor cells overexpressing human ABCB1, and camptothecin ADC-010 lost activity in N87 cells overexpressing human ABCG2, while the double-toxin molecule ADC-003 maintained good activity in N87 cells overexpressing the two transporters described above. Therefore, the double-toxin molecule is expected to resolve the drug resistance problem associated with existing drugs caused by the overexpression of the two transporters.

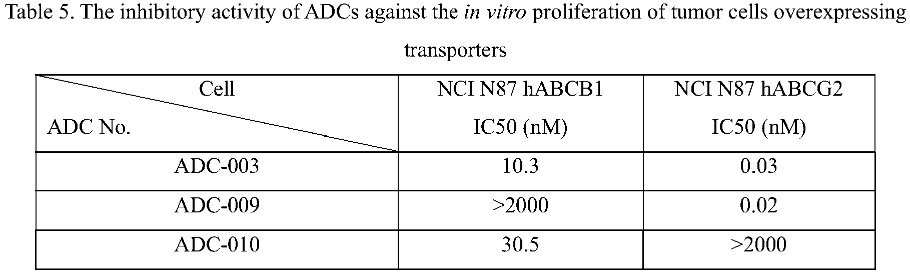

### Example 3.4: Assay for Inhibitory Activity of ADCs Against Tumor Proliferation in In Vivo Drug Resistance Model

### 1. Method:

NCI N87 cells with Enhertu-induced drug resistance were inoculated into immunodeficient Balb/c nude mice. When the tumors grew to a size of 100-200 (about 150) mm³, the mice were randomly grouped. The injection of the test ADC drugs was started, and the body weight and tumor size of the mice were measured twice a week.

### 2. Main observation indexes:

Relative tumor proliferation rate, T/C (%), refers to the percentage value of the relative tumor volume or tumor weight of the treatment group and the control group at a certain time point.

Relative tumor growth inhibition rate, TGI (%), refers to the relative tumor volume at a particular time point.

### 3. Results:

Compared to single-toxin ADCs, the double-toxin ADCs of the present disclosure exhibited good inhibitory effects on tumor proliferation in the drug-resistant model, indicating that they can overcome the drug resistance problem associated with single-toxin ADCs.

The above description is only for the purpose of illustrating the preferred examples of the present invention and is not intended to limit the scope of the present invention. Any modifications, equivalents, improvements, etc. made without departing from the spirit and principle of the present invention shall fall within the protection scope of the present invention. In addition, the technical solutions of the examples may be combined with each other, provided that the combination can be realized by those of ordinary skill in the art. If a combination of technical solutions results in contradictions or cannot be realized, the combination shall be deemed non-existent and falls outside the protection scope claimed by the present invention.

## Claims

1. An antibody-drug conjugate represented by formula (I), 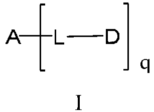
or a stereoisomer of the antibody-drug conjugate, a prodrug thereof, a pharmaceutically acceptable salt thereof, or a pharmaceutically acceptable solvate thereof,
wherein:
A is a known ADC;
D is a bioactive molecular fragment that targets a different target compared to a bioactive molecule in A;
L is a linker that covalently binds to A and D;
q is selected from any integer between 1 and 20.

2. An antibody-drug conjugate represented by formula (II), 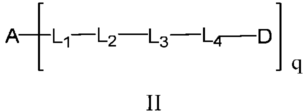
or a stereoisomer of the antibody-drug conjugate, a prodrug thereof, a pharmaceutically acceptable salt thereof, or a pharmaceutically acceptable solvate thereof,
wherein:
A is a known ADC;
D is a bioactive molecular fragment that targets a different target compared to a bioactive molecule in A;
L₁ is a linker unit that covalently binds to A and L₂;
L₂ is a connector unit that covalently binds to L₁ and L₃;
L₃ is an amino acid residue or a short peptide consisting of 2-10 amino acid residues; the amino acid residues are selected from natural amino acid residues, non-natural amino acid residues, and amino acid residues represented by AA¹ or stereoisomers thereof;
the amino acid residues represented by AA¹ have a structure as shown below: 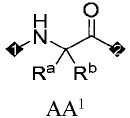
wherein:
R^{a} and R^{b} are each independently selected from hydrogen and 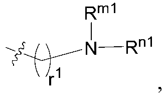 and R^{a} and R^{b} are not simultaneously hydrogen;
or R^{a} and R^{b}, together with the carbon atom to which they are attached, form a 4-10 membered heterocyclic ring or 4-10 membered carbocyclic ring, wherein the 4-10 membered heterocyclic ring or 4-10 membered carbocyclic ring is optionally substituted with one or more R⁰;
r¹ is selected from any integer between 0 and 20;
R^{m1} and Rⁿ¹ are each independently selected from hydrogen, C1-6 alkyl, and C3-6 cycloalkyl;
or R^{m1} and Rⁿ¹, together with the nitrogen atom to which they are attached, form a 4-10 membered heterocyclic ring, wherein the 4-10 membered heterocyclic ring is optionally substituted with one or more R^{0'};
R⁰ and R^{0'} are each independently selected from C1-6 alkyl, C3-6 cycloalkyl, -NR^{m2}Rⁿ², and 4-10 membered heterocyclyl, wherein the 4-10 membered heterocyclyl is optionally substituted with C1-6 alkyl;
R^{m2} and Rⁿ² are each independently selected from hydrogen and C1-6 alkyl;
L₄ is a direct bond or spacer unit that covalently binds to L₃ and D;
q is selected from any integer between 1 and 20.

3. The antibody-drug conjugate according to claim 1 or 2, wherein one or more of the following are met:
(1) A is selected from an ADC that targets a free antigen in a tumor tissue and/or a tumor cell surface antigen; preferably, A is selected from an ADC that targets one or more of the following targets: B7H3, CD20, CD19, CD30, GPNMB, Her2, Trop-2, EGFR, GD-2, CD79b, FRα, NAPI2B, and BCMA;
preferably, A comprises an antibody or an antigen-binding fragment thereof, Tb;
more preferably, Tb targets a target selected from: B7H3, CD20, CD19, CD30, GPNMB, Her2, Trop-2, EGFR, GD-2, CD79b, FRα, NAPI2B, and BCMA; further preferably, Tb is an anti-B7H3 antibody or an antigen-binding fragment thereof, an anti-Trop-2 antibody or an antigen-binding fragment thereof, an anti-Her 2 antibody or an antigen-binding fragment thereof, an anti-FRα antibody or an antigen-binding fragment thereof, or an anti-NAPI2B antibody or an antigen-binding fragment thereof;
(2) A is selected from a known ADC with a tubulin inhibitor as a bioactive molecule;
preferably, A is selected from a known ADC with an auristatin compound as a bioactive molecule, a known ADC with a maytansinoid compound as a bioactive molecule, a known ADC with a tubulysin compound as a bioactive molecule, and a known ADC with an eribulin compound as a bioactive molecule;
preferably, A is selected from ARX788, ARX517, FS-1502, AGS62P1, A166, ADC2122, mirvetuximab soravtansine, and trastuzumab emtansine;
D is a bioactive molecular fragment that targets a different target compared to a bioactive molecule in A, wherein the bioactive molecule is selected from, but is not limited to, glycopeptide antibiotics; glucocorticoid drugs; calcineurin inhibitors; DNA topoisomerase inhibitors, such as topoisomerase I inhibitors (e.g., camptothecin bioactive molecules) and topoisomerase II inhibitors; drugs that interfere with DNA synthesis; drugs that act on structural proteins; tumor signaling pathway inhibitors, such as kinase inhibitors; drugs that act on RNA; proteasome inhibitors; protease inhibitors; inhibitors of epigenetically related targets; tumor angiogenesis inhibitors; cyclin inhibitors; protein degradation agents, such as PROTACs and molecular glues; nucleic acid drugs; immunomodulators; steroidal saponins; and other active substances that inhibit tumor cell growth and promote tumor cell apoptosis or necrosis;
preferably, D is a camptothecin bioactive molecular fragment;
preferably, D is a camptothecin bioactive molecular fragment represented by formula (III) that is attached to L₄ via an oxygen atom, a sulfur atom, or a nitrogen atom contained therein, 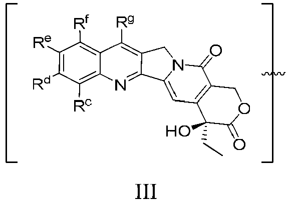
wherein:
R^{c}, R^{d}, R^{e}, R^{f}, and R^{g} are each independently selected from hydrogen, halogen, -OH, -NH₂, optionally substituted C1-6 alkyl, optionally substituted C1-6 alkoxy, and 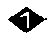 -X-W-H, wherein position 1 is attached to a parent ring;
or R^{d} and R^{e}, together with the carbon atoms to which they are attached, form a 5-7 membered carbocyclic ring or 5-7 membered heterocyclic ring, wherein the heterocyclic ring comprises one or more O, S, N, carbonyl,
sulfoxide groups, or sulfonyl, or any combination thereof;
or R^{e} and R^{f}, together with the carbon atoms to which they are attached, form a 5-7 membered carbocyclic ring or 5-7 membered heterocyclic ring, wherein the heterocyclic ring comprises one or more O, S, N, carbonyl, sulfoxide groups, or sulfonyl, or any combination thereof;
or R^{f} and R^{g}, together with the carbon atoms to which they are attached, form a 5-7 membered carbocyclic ring or 5-7 membered heterocyclic ring, wherein the heterocyclic ring comprises one or more O, S, N, carbonyl, sulfoxide groups, or sulfonyl, or any combination thereof;
the carbocyclic ring and heterocyclic ring may be substituted with one or more deuterium, halogens, C1-6 alkyl, or 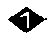 -X-W-H, wherein position 1 is attached to the carbocyclic ring or heterocyclic ring;
X is selected from a direct bond, optionally substituted -(CH₂)ₙ₁-, -O-(CH₂)ₙ₁-, -N(R₁)-(CH₂)ₙ₁-, -S-(CH₂)ₙ₁-, - C(O)-(CH₂)ₙ₁, -SO₂-(CH₂)ₙ₁-, 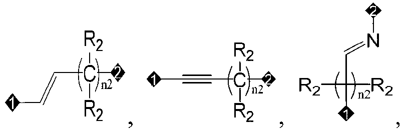 C3-6 cycloalkyl, C6-10 aryl, 5-10 membered heteroaryl, and 4-10 membered heterocyclyl, wherein position 1 is attached to a parent ring, a carbocyclic ring, or a heterocyclic ring, and position 2 is attached to W or H; the substituent is selected from one or more C1-4 alkyl and C3-6 cycloalkyl, or more than one C1-4 alkyl, together with the carbon atom to which they are simultaneously attached, forms C3-6 cycloalkyl;
R₁ and R₂, at each occurrence, are each independently selected from hydrogen, optionally substituted C1-4 alkyl, optionally substituted C1-4 alkoxy, and optionally substituted C3-6 cycloalkyl;
n1 and n2 are each independently selected from any integer between 0 and 6;
W is absent or present, and when W is present, W is selected from -O-, -S-, -NR₃-, 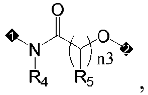 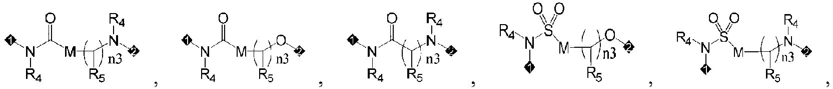 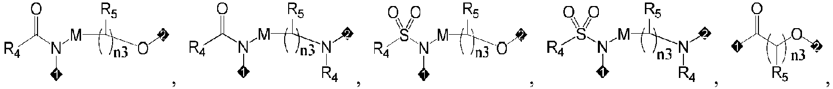 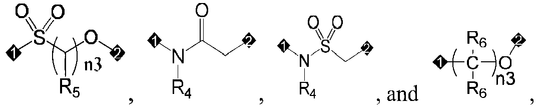 wherein position 1 is attached to X, and position 2 is attached to H;
each M is independently selected from a direct bond, -CR₇R₈-, a 3-7 membered carbocyclic ring, and a 3-7 membered heterocyclic ring;
R₃, R₄, R₅, R₆, R₇, and R₈, at each occurrence, are each independently selected from hydrogen, optionally substituted C1-4 alkyl, optionally substituted C1-4 alkenyl, optionally substituted C1-4 alkynyl, optionally substituted C1-4 alkoxy, and optionally substituted C3-6 cycloalkyl;
n3 is selected from any integer between 0 and 6;
preferably, D is the following structure: 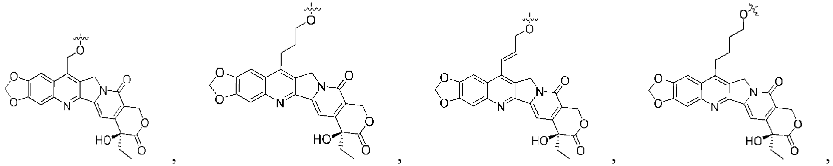 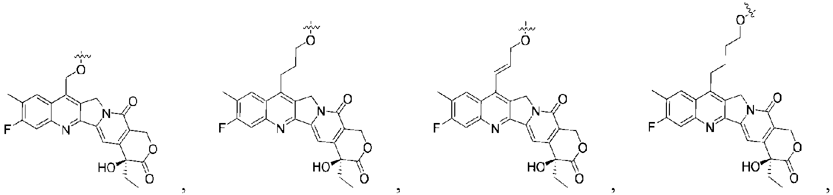 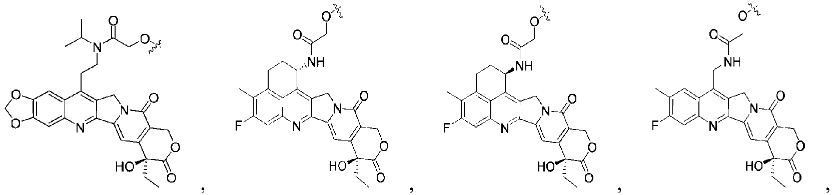 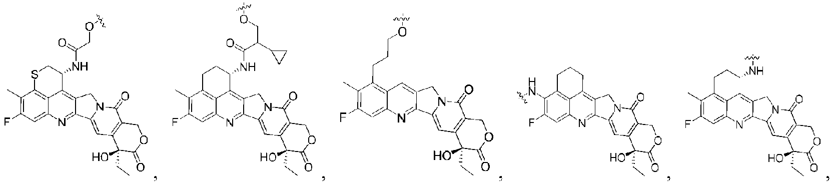 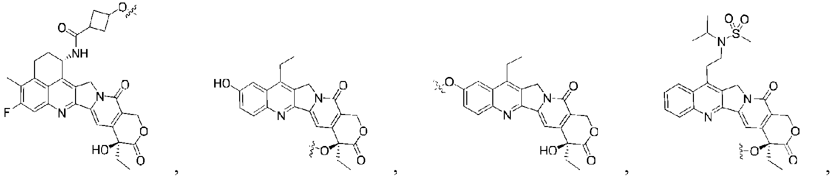 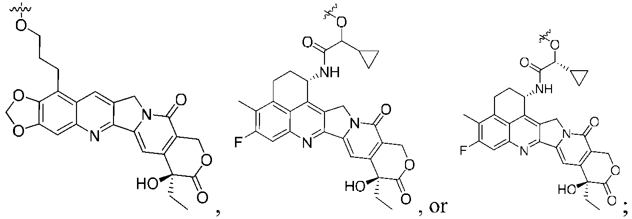
(3) A is selected from a known ADC with camptothecin as a bioactive molecule; preferably, A is selected from trastuzumab deruxtecan, SHR-A1811,
and 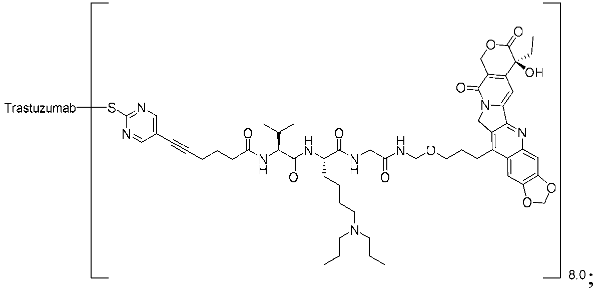
D is selected from a tubulin inhibitor bioactive molecular fragment; for example, D is MMAE and a derivative thereof, MMAF and a derivative thereof, or an eribulin bioactive molecular fragment;
preferably, D is the following structure:

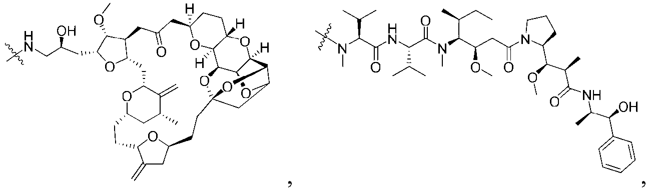

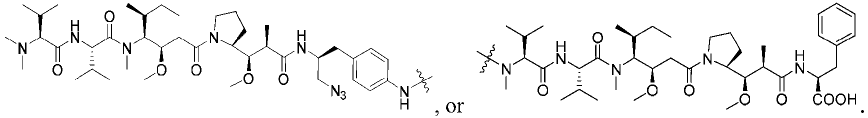

4. The antibody-drug conjugate according to any one of claims 1-3, wherein one or more of the following are met:
(1) q is selected from any integer between 1 and 12; preferably, q is selected from 1, 2, 3, 4, 5, 6, 7, 8, 9, and 10;
(2) L₁ is selected from 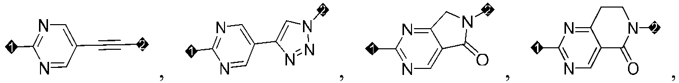 and 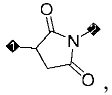 wherein position 1 is attached to A via a sulfur (S) atom, and position 2 is attached to L₂; or L₁ is selected from 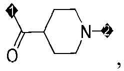 wherein position 1 is attached to A via a nitrogen (N) atom, and position 2 is attached to L₂;
(3) L₂ is selected from 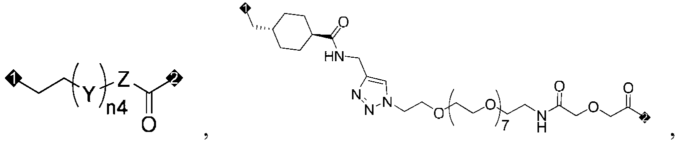 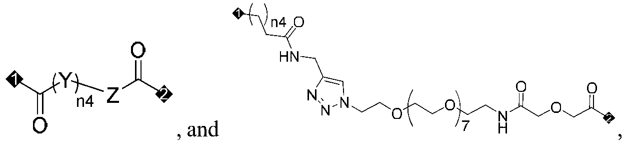 wherein position 1 is attached to L₁, and position 2 is attached to L₃;
Y is selected from -O-, -CH₂-, -OCH₂CH₂-, 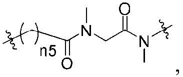 and any combination of more than one of the above groups;
n4 and n5 are each independently selected from any integer between 0 and 30;
Z is selected from -CR^{m}Rⁿ- and -NR^{m}-;
R^{m} and Rⁿ are each independently selected from hydrogen, C1-6 alkyl, C2-6 alkenyl, C2-6 alkynyl, C3-10 cycloalkyl, and 4-10 membered heterocyclyl; or R^{m} and Rⁿ, together with the carbon atom to which they are attached, form a 3-6 membered carbocyclic ring or 3-6 membered heterocyclic ring;
(4) L₃ is selected from an amino acid residue and a dipeptide, tripeptide, or tetrapeptide consisting of 2-4 amino acid residues, wherein the amino acid residues are selected from natural amino acid residues, non-natural amino acid residues, and amino acid residues represented by AA¹ or stereoisomers thereof;
preferably, L₃ is selected from a dipeptide, tripeptide, or tetrapeptide consisting of 2-4 amino acid residues, wherein the amino acid residues are selected from amino acid residues represented by AA¹ or stereoisomers thereof;
(5) L₄ is selected from a direct bond, 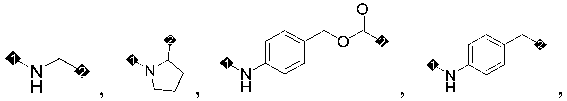 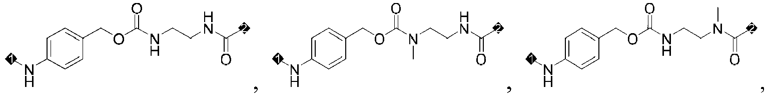 and 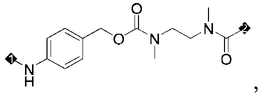 wherein position 1 is attached to L₃, and position 2 is attached to D;
(6) the 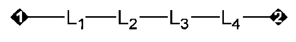 is selected from the following: 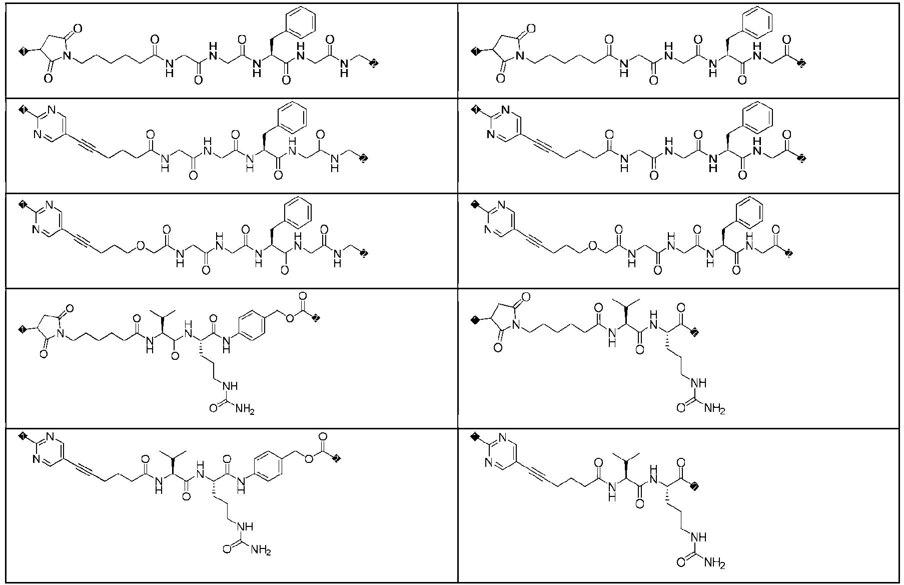 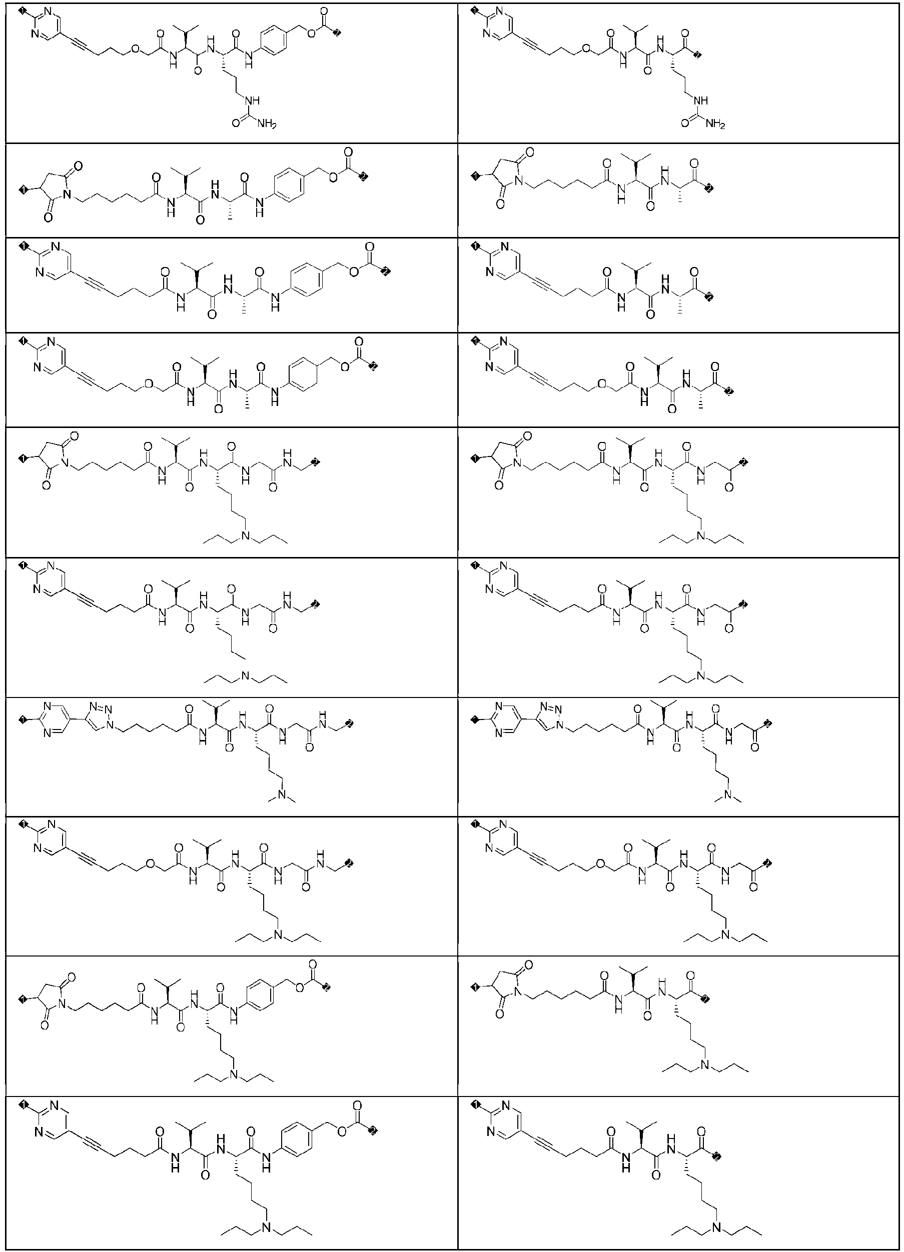 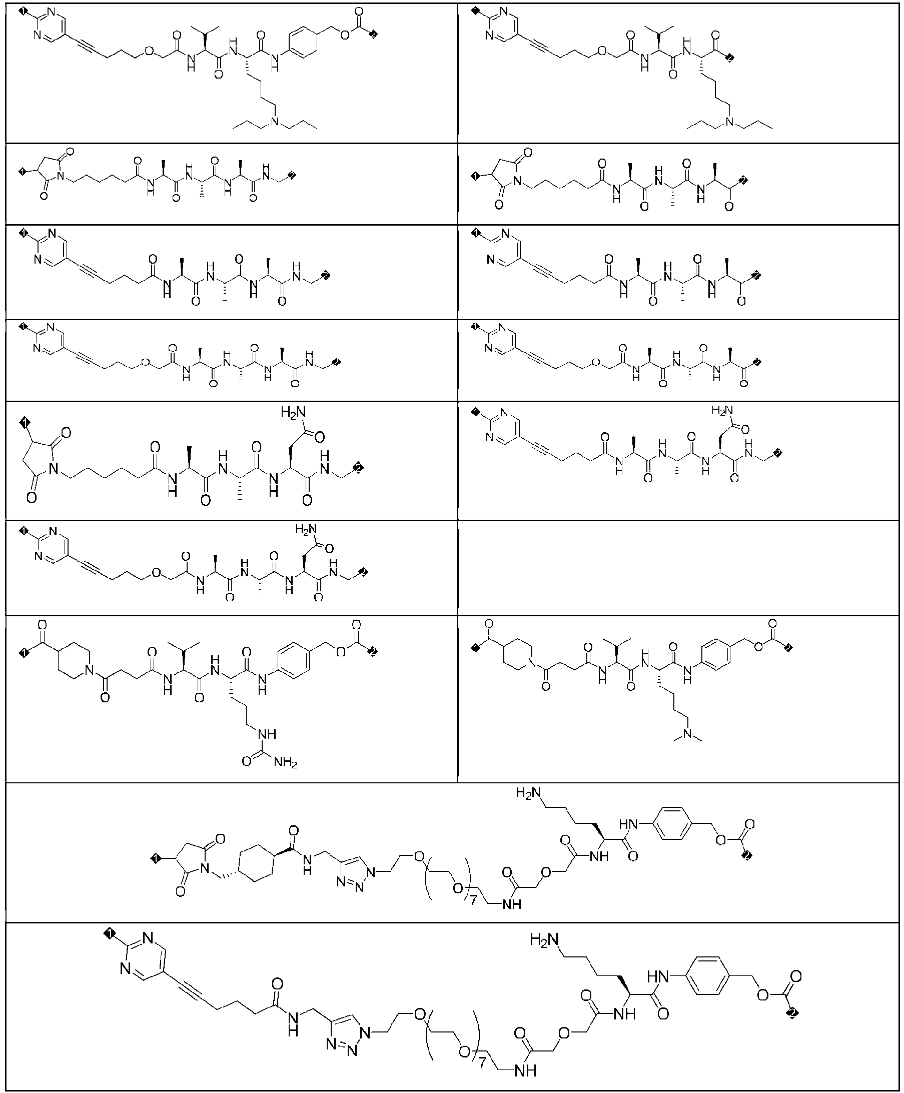 wherein position 1 is attached to A, and position 2 is attached to D.

5. The antibody-drug conjugate according to any one of claims 1-4, wherein the antibody-drug conjugate is selected from: 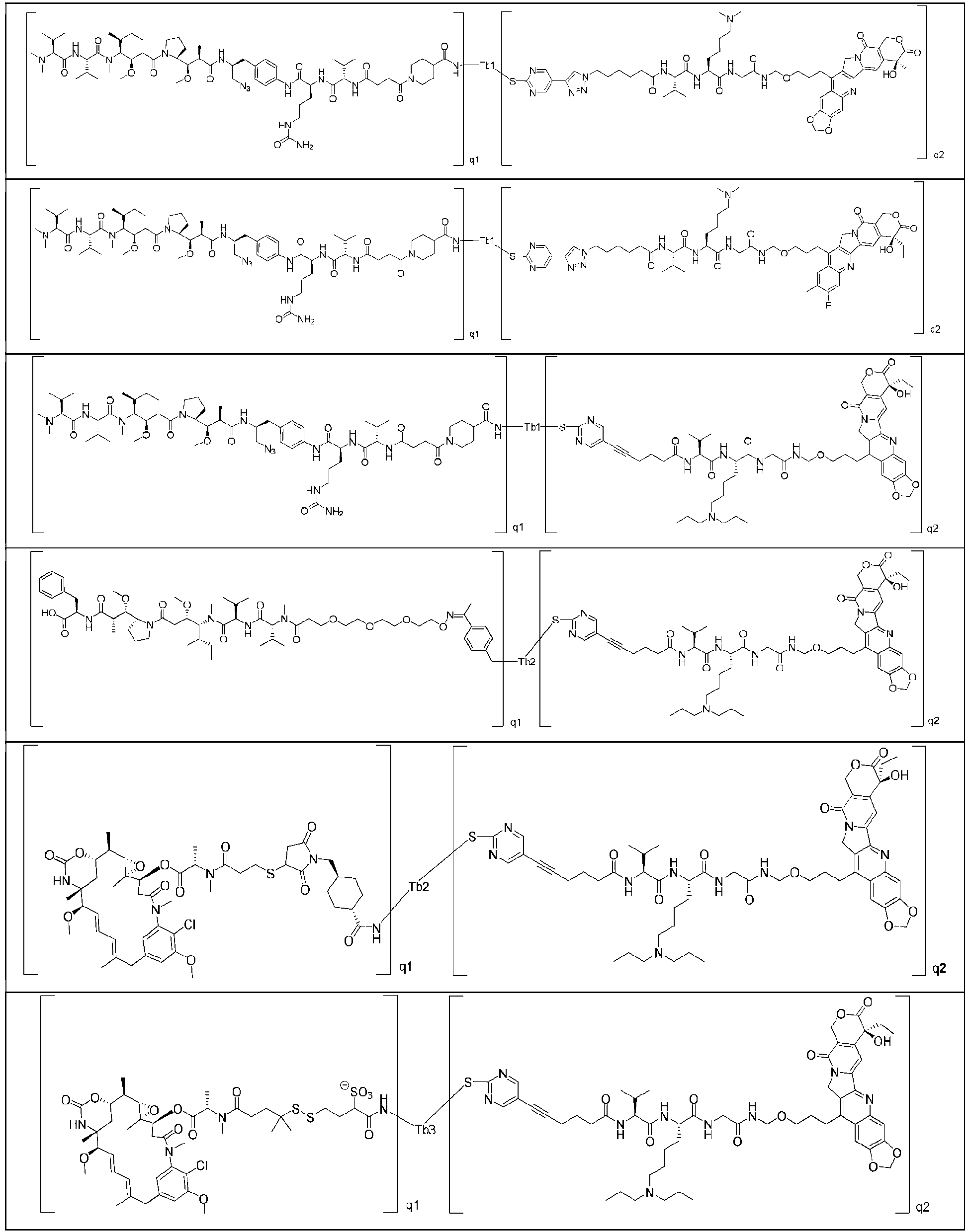 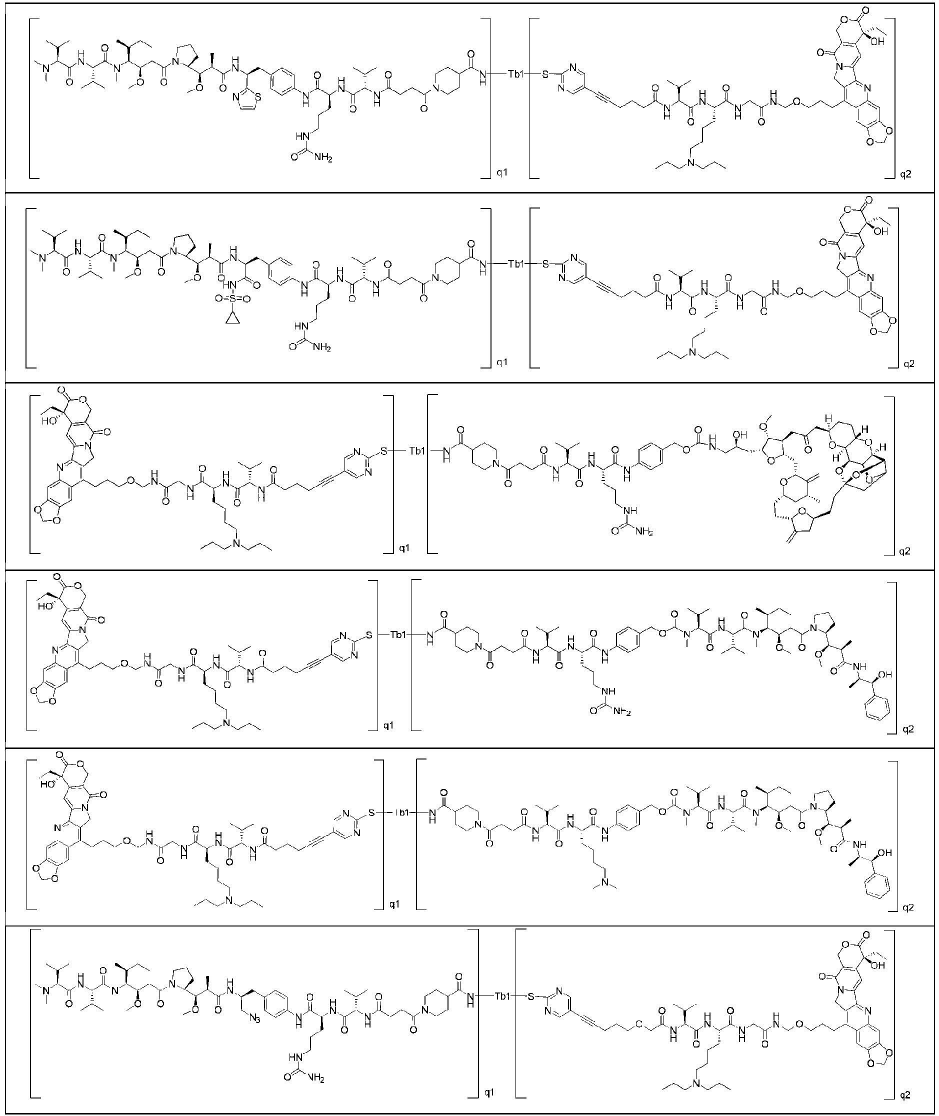
wherein q1 and q2 are each independently selected from any numerical value between 1 and 10;
Tb1, Tb2, and Tb3 are each independently an antibody or an antigen-binding fragment thereof;
Tb1, Tb2, and Tb3 are each independently selected from the antibody or the antigen-binding fragment thereof described above for Tb;
preferably, Tb1, Tb2, and Tb3 are each independently selected from an antibody or an antigen-binding fragment thereof that targets one or more of the following targets: B7H3, CD20, CD19, CD30, GPNMB, Her2, Trop-2, EGFR, GD-2, CD79b, FRα, NAPI2B, and BCMA;
more preferably, Tb1, Tb2, and Tb3 are each independently selected from an anti-B7H3 antibody or an antigen-binding fragment thereof, an anti-Trop-2 antibody or an antigen-binding fragment thereof, an anti-Her 2 antibody or an antigen-binding fragment thereof, an anti-FRα antibody or an antigen-binding fragment thereof, and an anti-NAP12B antibody or an antigen-binding fragment thereof;
further preferably, the antibody-drug conjugate is selected from the following: 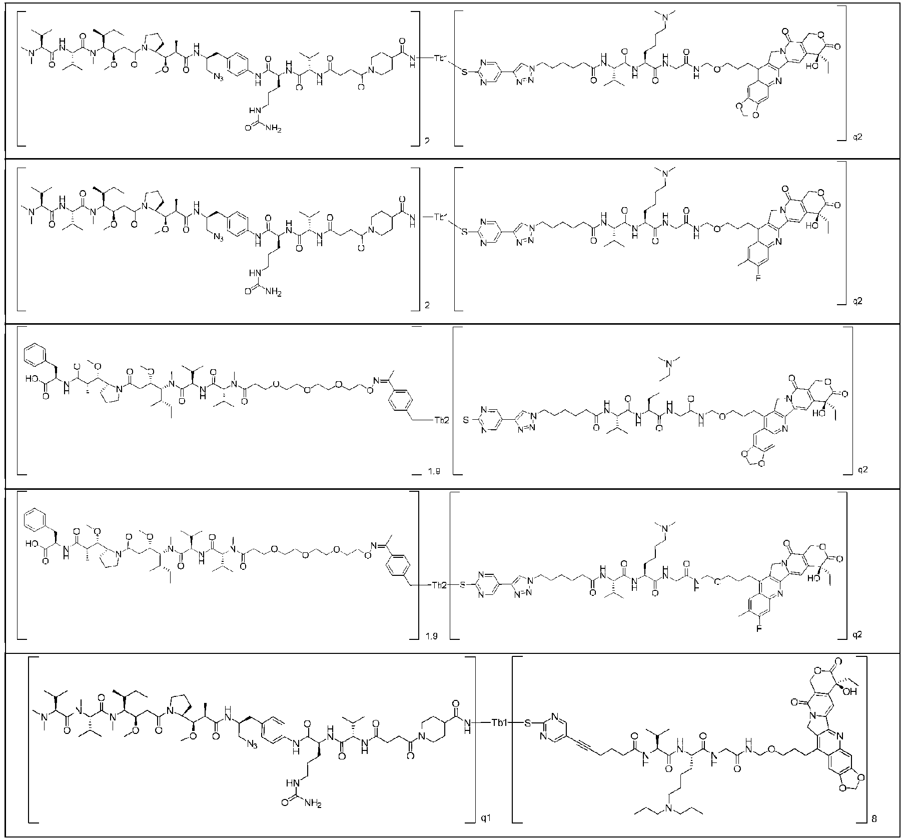 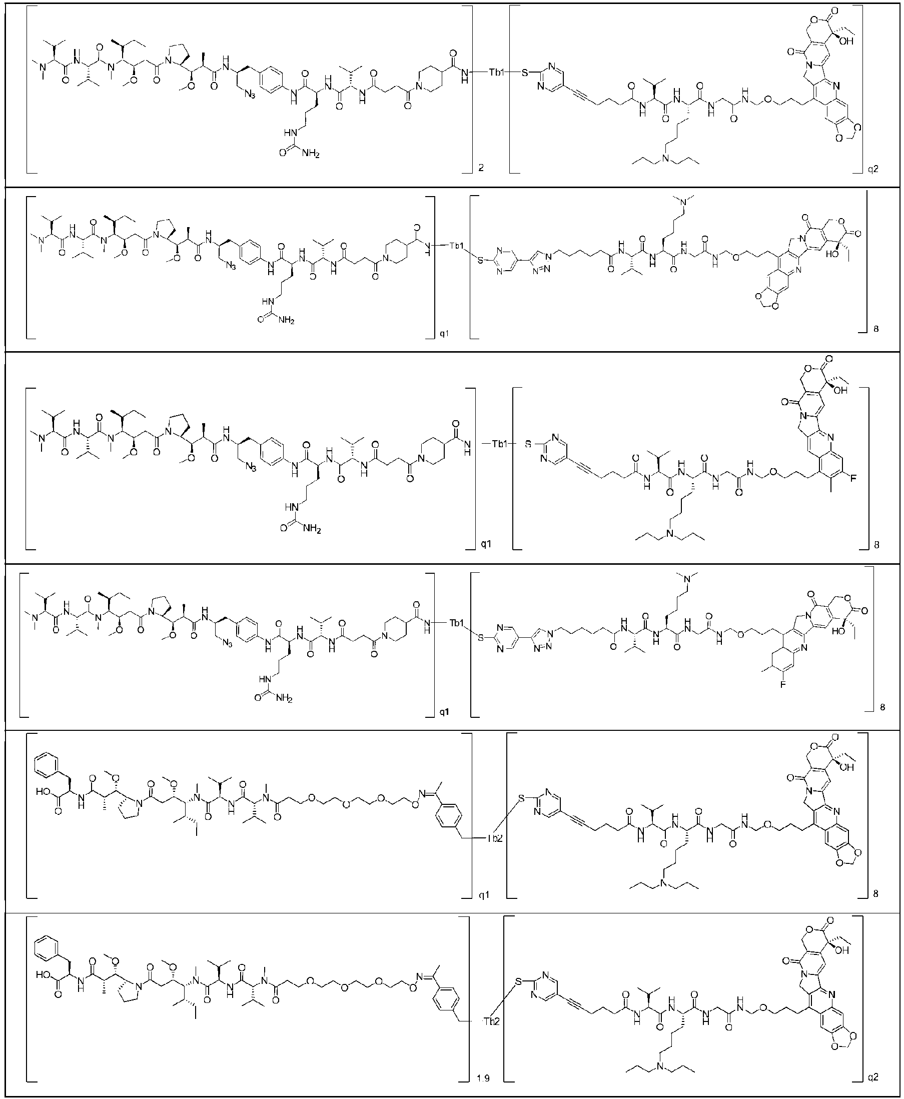 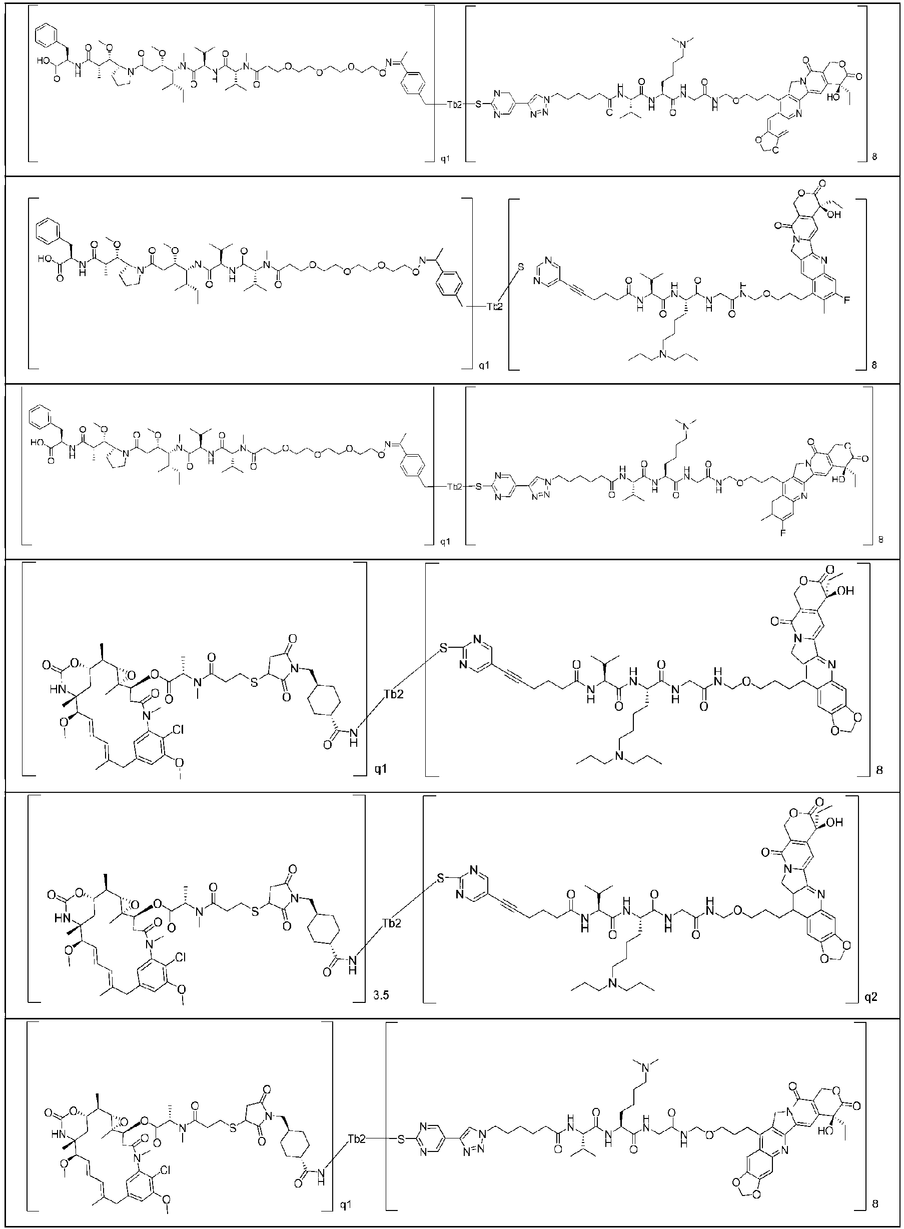 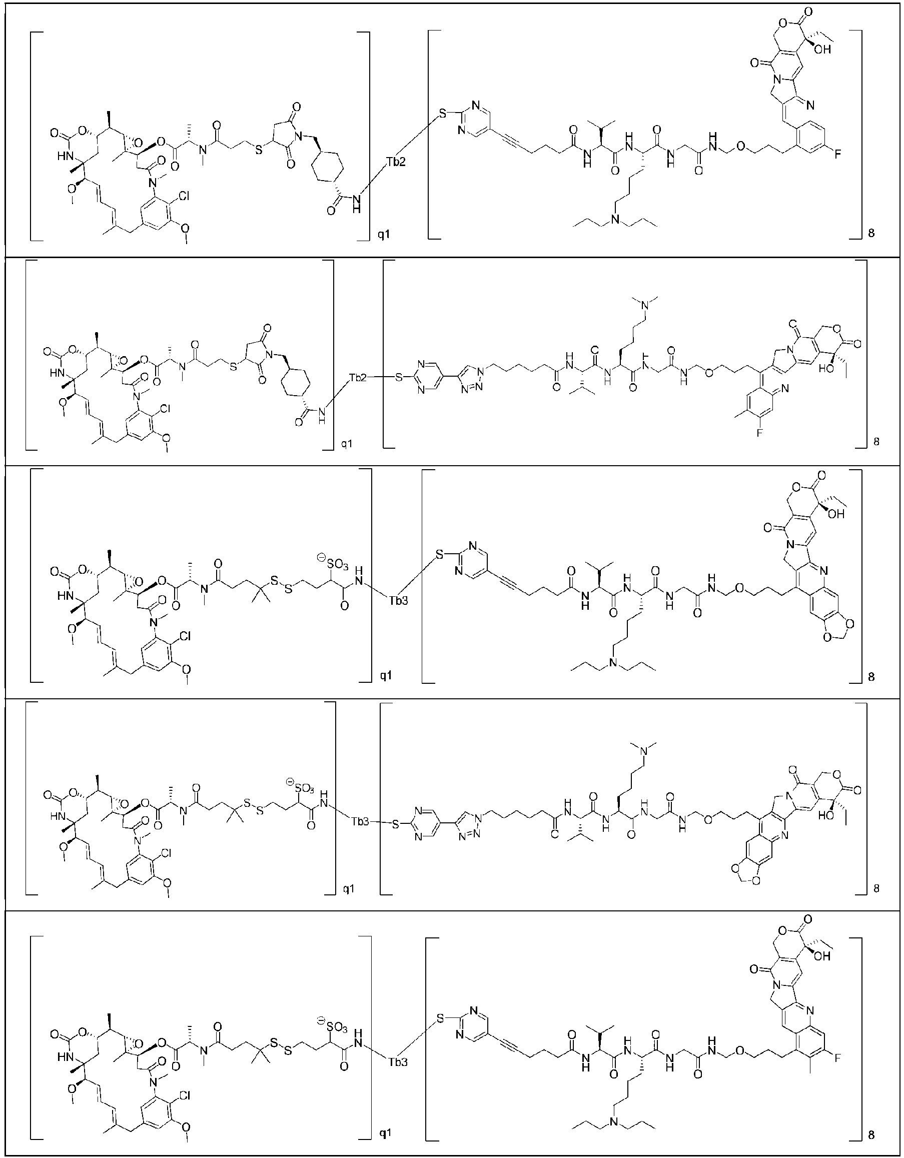 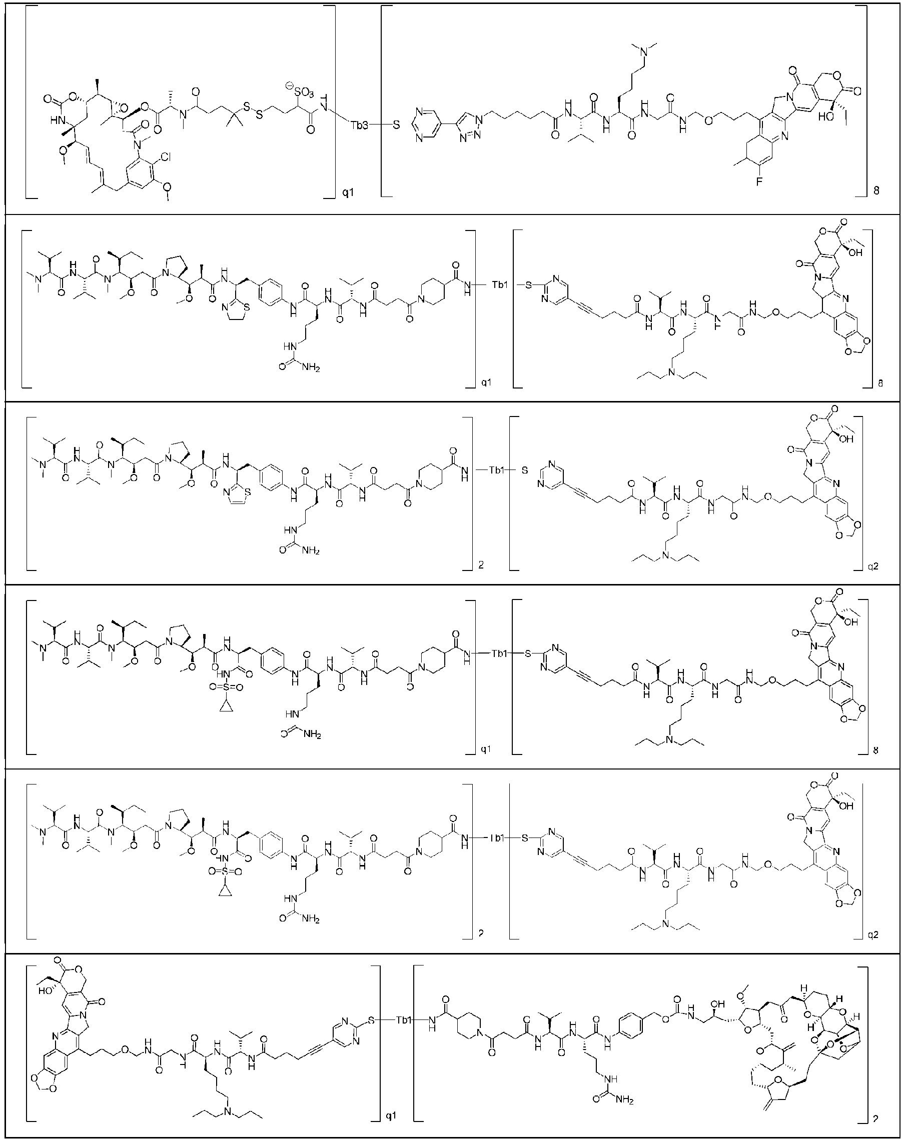 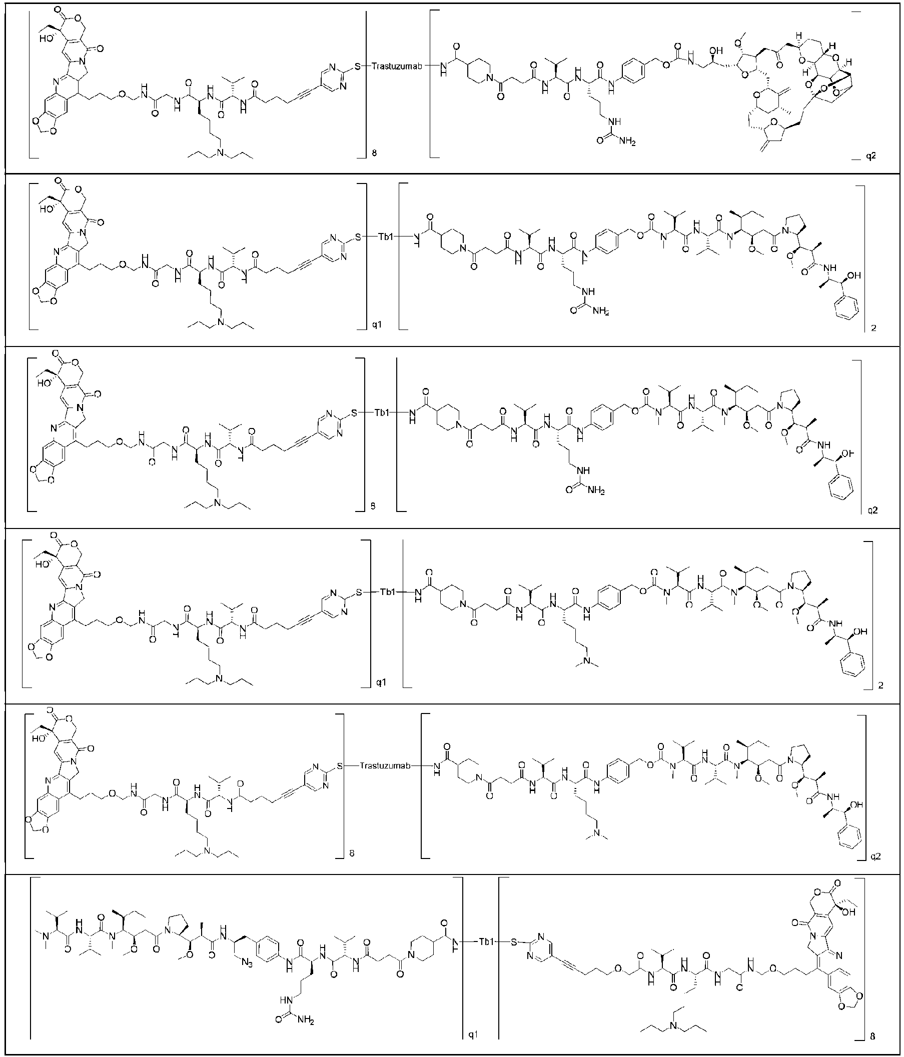 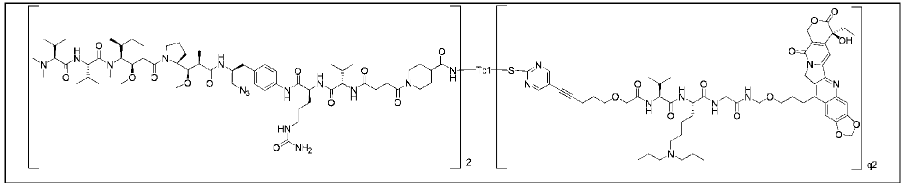
wherein q1 is selected from 1.0-6.0, 1.2-5.5, 1.4-5, 1.5-4.5, 1.6-4.0, 1.7-3.5, 1.8-3.0, 1.8-2.5, 1.8-2.4, 1.8-2.3, 1.9-2.2, and 2.0;
q2 is selected from 4.0-10, 4.0-9.5, 5.0-9.0, 6.0-8.8, 7.0-8.6, 7.5-8.5, 7.6-8.4, 7.7-8.3, 7.8-8.2, 7.9-8.1, and 8.0; preferably, Tb1 is an anti-Her 2 antibody or an antigen-binding fragment thereof; more preferably, Tb1 is trastuzumab, pertuzumab, or an antigen-binding fragment thereof;
preferably, Tb2 is an anti-Her 2 antibody or an antigen-binding fragment thereof; more preferably, Tb2 is trastuzumab or an antigen-binding fragment thereof;
preferably, Tb3 is an anti-FRα antibody or an antigen-binding fragment thereof; more preferably, Tb3 is anti-mirvetuximab or an antigen-binding fragment thereof.

6. The antibody-drug conjugate according to any one of claims 1-5, wherein the antibody-drug conjugate is selected from:

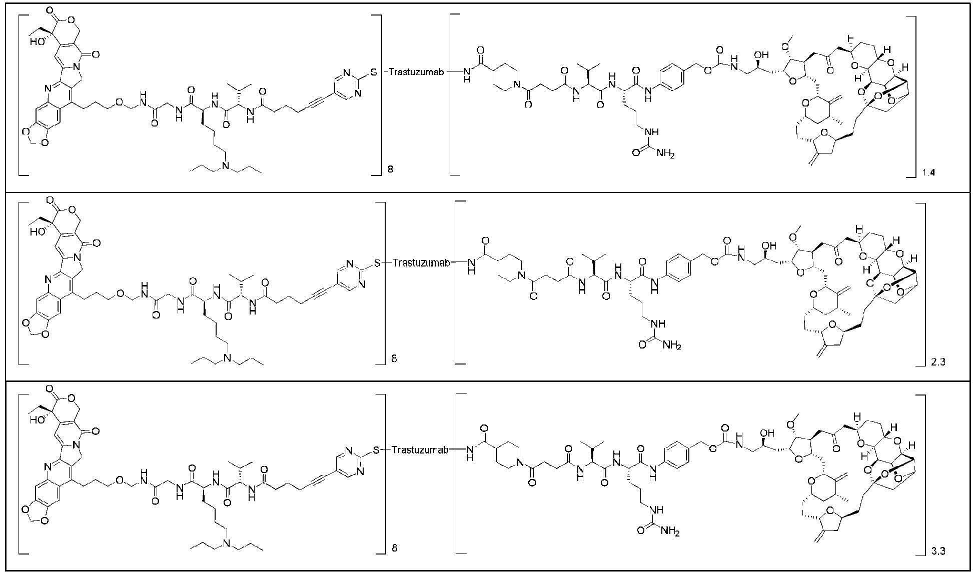

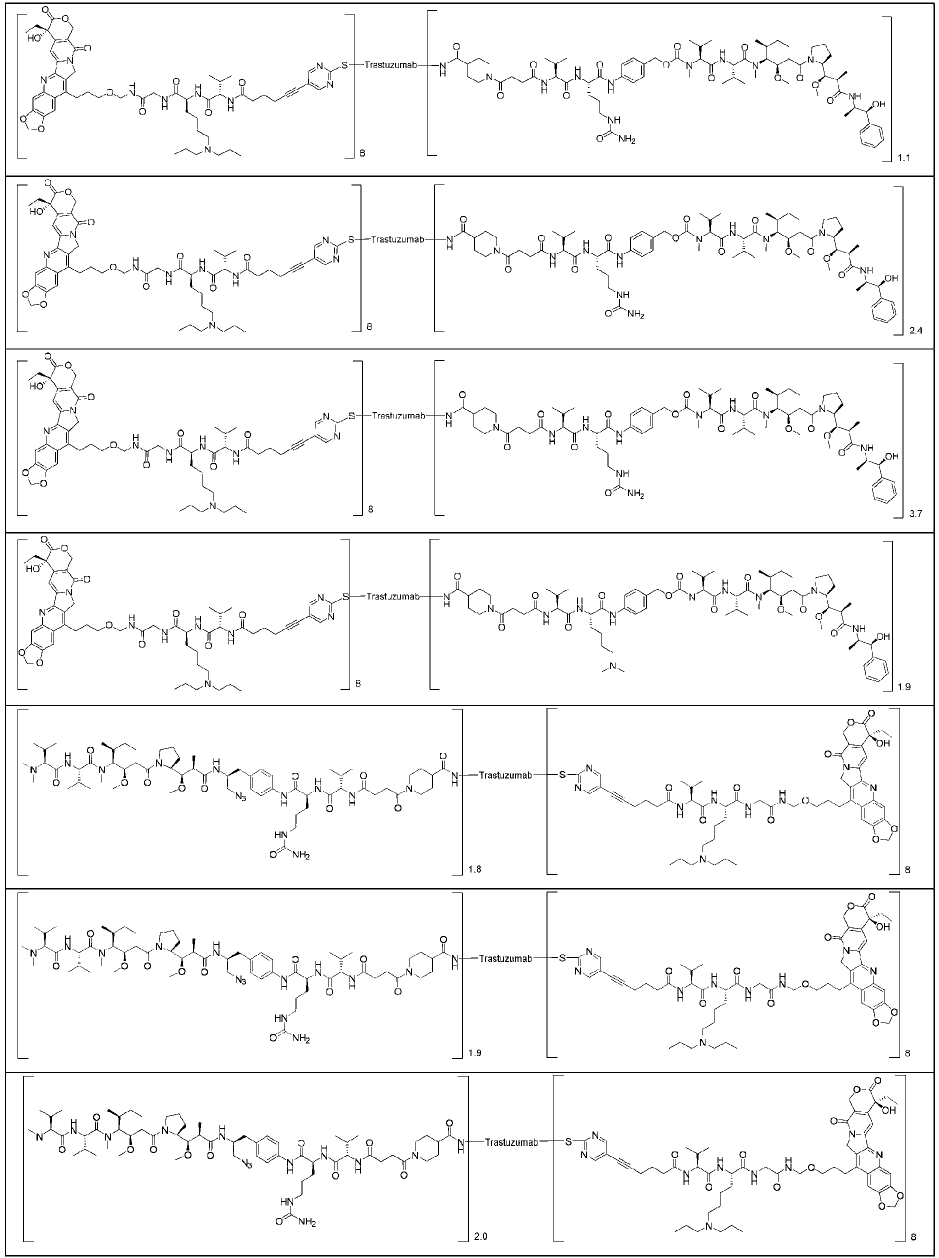

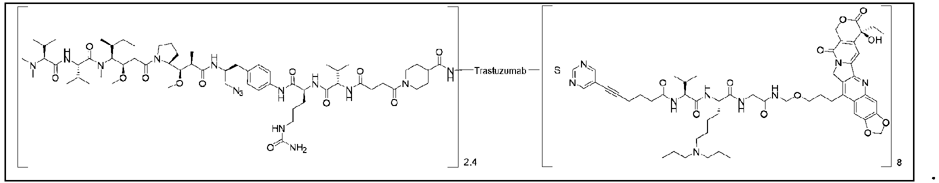

7. A method for preparing an antibody-drug conjugate represented by formula (1), comprising subjecting A to a conjugation reaction with a toxin-linker represented by formula (IV) L'-D,
wherein L' is a linker precursor, and D is as defined in any one of claims 1-3;
a molar ratio of A to the toxin-linker represented by formula (IV) L'-D is 1:(1-20), such as 1:2-16, 1:4-16, 1:6-16, 1:8-16, or 1:10-16;
the conjugation reaction is preferably performed in water and/or an organic solvent; the organic solvent is preferably one or more of N,N-dimethylformamide, N,N-dimethylacetamide, dimethyl sulfoxide, and N-methylpyrrolidone.

8. A population of antibody-drug conjugates, comprising or consisting of the antibody-drug conjugate or the stereoisomer of the antibody-drug conjugate, the prodrug thereof, the pharmaceutically acceptable salt thereof, or the pharmaceutically acceptable solvate thereof according to any one of claims 1-6, wherein the antibody-drug conjugates have one, two, or more q values; preferably, an average DAR of the population of antibody-drug conjugates is selected from integers and decimals of 1-16, e.g., from 1-1.5, 1.5-2.5, 2.5-3.5, 3.5-4.5, 4.5-5.5, 5.5-6.5, 6.5-7.5, and 7.5-8.5.

9. A pharmaceutical composition, comprising the antibody-drug conjugate or the stereoisomer of the antibody-drug conjugate, the prodrug thereof, the pharmaceutically acceptable salt thereof, or the pharmaceutically acceptable solvate thereof according to any one of claims 1-6 or the population of antibody-drug conjugates according to claim 8 and optionally one or more pharmaceutical adjuvants.

10. Use of the antibody-drug conjugate or the stereoisomer of the antibody-drug conjugate, the prodrug thereof, the pharmaceutically acceptable salt thereof, or the pharmaceutically acceptable solvate thereof according to any one of claims 1-6, or the population of antibody-drug conjugates according to claim 8, or the pharmaceutical composition according to claim 9 in the manufacture of a medicament for treating and/or preventing a disease associated with abnormal cell activity (e.g., a cancer disease).
